(19) Europäisches Patentamt

European Patent Office

Office européen des brevets

(11) **EP 4 219 676 A1**

(12) **EUROPEAN PATENT APPLICATION**
published in accordance with Art. 153(4) EPC

(43) Date of publication:
**02.08.2023 Bulletin 2023/31**

(21) Application number: **21872589.3**

(22) Date of filing: **24.09.2021**

(51) International Patent Classification (IPC):
**C12M 1/04** *(2006.01)*     **C12N 1/00** *(2006.01)*
**C12N 1/02** *(2006.01)*

(52) Cooperative Patent Classification (CPC):
**C12M 1/04; C12N 1/00; C12N 1/02**

(86) International application number:
**PCT/JP2021/035209**

(87) International publication number:
**WO 2022/065460 (31.03.2022 Gazette 2022/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.09.2020 JP 2020160241**

(71) Applicant: **NIKON CORPORATION
Minato-ku
Tokyo 108-6290 (JP)**

(72) Inventors:
• **MORIYAMA Masaki
Tokyo 108-6290 (JP)**

• **ISHIZAWA Naoya
Tokyo 108-6290 (JP)**
• **KOBAYASHI Ryo
Tokyo 108-6290 (JP)**
• **NAKAMURA Taichi
Tokyo 108-6290 (JP)**
• **TANAKA Shuhei
Tokyo 108-6290 (JP)**
• **HAYASHI Seri
Tokyo 108-6290 (JP)**
• **TAKUBO Makiko
Tokyo 108-6290 (JP)**

(74) Representative: **Hoffmann Eitle
Patent- und Rechtsanwälte PartmbB
Arabellastraße 30
81925 München (DE)**

(54) **OPERATION METHOD AND OPERATION DEVICE FOR LIVING BODY**

(57)     In a first aspect of the present invention, a manipulation method of an organism is provided which includes: forming a gas-liquid interface, in which a restoring force acts against minute interface movement, in a flow channel or at an end part of the flow channel, with the end part immersed in a liquid in which an organism is immersed; and manipulating the organism by using the gas-liquid interface.

EP 4 219 676 A1

**(Cont. next page)**

START

RECEIVE SAMPLE — S100

CAPTURE IMAGE — S120

RECEIVE MANIPULATION CONDITION — S140

IS THERE LIQUID REPLACEMENT/ADDITION PROCESSING?

Yes → LIQUID REPLACEMENT/ADDITION PROCESSING — S600

No

S160

INSTALL NOZZLE — S180

MOVE RELATIVE POSITION BETWEEN NOZZLE AND CELL — S200

FORM AIR BUBBLE — S300

EXECUTE MANIPULATION — S400

REMOVE AIR BUBBLE — S500

IS THERE RELEASE OF MANIPULATION TARGET?

Yes → RELEASE TO DESIGNATED POSITION — S645

S640

No

IS THERE ANOTHER MANIPULATION TARGET?

Yes → EXCHANGE NOZZLE?

S650

No → REMOVE NOZZLE — S680

No

Yes → REMOVE NOZZLE — S670

S660

END

*FIG.6*

**Description**

BACKGROUND

1. TECHNICAL FIELD

The present invention relates to a manipulation method of an organism and a manipulation device of the organism.

2. RELATED ART

In cell biology research and the like, specific cells are sucked from many cells in a culture vessel. Patent Document 1 discloses a cell suction support system which sucks a target cell by using a chip.
Patent Document 1: Japanese Patent Application Publication No. 2016-000007

GENERAL DISCLOSURE

In a first aspect of the present invention, a manipulation method of an organism is provided. The manipulation method of the organism may include forming a gas-liquid interface, in which a restoring force acts against minute interface movement, in a flow channel or at an end part of the flow channel, with the end part immersed in a liquid in which an organism is immersed. In addition, the manipulation method of the organism may include manipulating the organism by using the gas-liquid interface.
In a second aspect of the present invention, in the manipulation method of the organism, the forming the gas-liquid interface may include releasing or sucking a gas from the end part.
In a third aspect of the present invention, in the manipulation method of the organism, the forming the gas-liquid interface may include releasing a gas from the end part and maintaining the gas-liquid interface, in which the restoring force acts against the minute volume change, at the end part.
In a fourth aspect of the present invention, in the manipulation method of the organism, in the forming the gas-liquid interface, a gas volume V (m3) occupied by the gas continuous from the gas-liquid interface to an inside of the flow channel may satisfy a following formula with respect to a radius rh (m) of the end part of the flow channel.

$$V \leq a \times r_h^4 + b \times r_h^3$$

where $a = 2.65 \times 10^8$ and $b = 2.59 \times 10^2$ are satisfied.
In a fifth aspect of the present invention, the manipulation method of the organism may further include decreasing a gas volume occupied by the gas continuous from the end part to an inside of the flow channel before the forming the gas-liquid interface.
In a sixth aspect of the present invention, in the manipulation method of the organism, the decreasing the gas volume may include taking the liquid into the flow channel and partitioning the gas inside the flow channel with the taken liquid.
In a seventh aspect of the present invention, in the manipulation method of the organism, the decreasing the gas volume may include filling the flow channel with a filler which blocks a partial inner space of the flow channel.
In an eighth aspect of the present invention, in the manipulation method of the organism, the flow channel may include a nozzle and a space formed inside a pump connected to the nozzle. The decreasing the gas volume may include reducing a capacity of the pump to 10% or less of a maximum capacity.
In a ninth aspect of the present invention, in the manipulation method of the organism, the flow channel may be provided with a dividing member which divides an inner space of the flow channel.
In a tenth aspect of the present invention, in the manipulation method of the organism, a cross-sectional shape of the end part of the flow channel may be a shape having a protruding portion inward.
In an eleventh aspect of the present invention, in the manipulation method of the organism, at the end part of the flow channel, a corner may be formed by an inner side surface of the flow channel and a surface on an end part side of a flow channel member forming the flow channel, and an angle formed by the corner may be in a range of $90 \pm 5$ degrees.
In a twelfth aspect of the present invention, in the manipulation method of the organism, a contact angle between a flow channel member forming the flow channel and the liquid by a droplet method may be 90 degrees or less.
In a thirteenth aspect of the present invention, in the manipulation method of the organism, the flow channel may have an edge portion provided on an end part side. The flow channel may have an inner portion which is connected to the edge portion and has a flow channel diameter different from a flow channel diameter of the edge portion. The flow channel may form a stepped portion at a connection portion between the edge portion and the inner portion.

In a fourteenth aspect of the present invention, in the manipulation method of the organism, the flow channel may include a portion having a large flow channel diameter and a portion having a small flow channel diameter. A flow channel diameter of the portion having a small flow channel diameter may be 1/10 or less of a flow channel diameter of the portion having a large flow channel diameter.

In a fifteenth aspect of the present invention, the manipulation method of the organism may further include decreasing, before the forming the gas-liquid interface, a surface tension of the liquid with the gas before the forming the gas-liquid interface.

In a sixteenth aspect of the present invention, the manipulation method of the organism may further include detecting a first pressure applied to the gas-liquid interface. In the detecting, a change in the first pressure when a gas is continuously released into the flow channel may be detected, and a second pressure which allows formation of the gas-liquid interface may be decided on the basis of a value of the first pressure that has been changed.

In a seventeenth aspect of the present invention, the manipulation method of the organism may further include detecting a first pressure applied to the gas-liquid interface. In the manipulation method of the organism, on the basis of a change in the first pressure, the end part of the flow channel having come into contact with the liquid may be detected, or the gas-liquid interface formed at the end part of the flow channel having come into contact with a bottom portion of a vessel for culturing the organism may be detected.

In an eighteenth aspect of the present invention, the manipulation method of the organism may further include moving, before the forming the gas-liquid interface, relative position of the flow channel and the organism closer to each other.

In addition, in a nineteenth aspect of the present invention, an organism manipulation device for manipulating an organism is provided. The organism manipulation device may include a flow channel having an end part immersed in a liquid in which the organism is immersed. The organism manipulation device may include a gas-liquid interface manipulation unit which forms a gas-liquid interface in which a restoring force acts against minute interface movement in the flow channel or at the end part and manipulates the organism by the gas-liquid interface.

In a twentieth aspect of the present invention, the gas-liquid interface manipulation unit of the organism manipulation device may release a gas into the liquid from the end part. The gas-liquid interface, in which a restoring force acts against a minute volume change, may be maintained at the end part.

In a twenty-first aspect of the present invention, in the organism manipulation device, a gas volume V (m3) occupied by the gas continuous from the gas-liquid interface to an inside of the flow channel may satisfy a following formula with respect to a radius rh (m) of the end part of the flow channel.

$$V \leq a \times r_h^4 + b \times r_h^3,$$

where $a = 2.65 \times 10^8$ and $b = 2.59 \times 10^2$ are satisfied.

In addition, in a twenty-second aspect of the present invention, an organism manipulation device for manipulating an organism is provided. The organism manipulation device may include a pump which introduces a gas into the flow channel to form a gas-liquid interface at the end part. The organism manipulation device may include a pressure detector which detects a first pressure applied to the gas-liquid interface. The organism manipulation device may include a storing unit which stores a second pressure which allows maintenance of the gas-liquid interface. The pump may change an introduction amount of the gas on the basis of a change in the first pressure, and control the gas introduced into the flow channel to maintain a state where the first pressure is equal to or lower than the second pressure.

The summary clause does not necessarily describe all necessary features of the embodiments of the present invention. The invention may also include a sub-combination of the features described above.

BRIEF DESCRIPTION OF THE DRAWINGS

Fig. 1A illustrates an example of a device configuration of an organism manipulation device 100 in the present embodiment.

Fig. 1B illustrates an example of the device configuration of the organism manipulation device 100 in the present embodiment.

Fig. 2A illustrates an example of a schematic view illustrating a structure of a nozzle 49 in the present embodiment.

Fig. 2B illustrates an example of the schematic view illustrating the structure of the nozzle 49 in the present embodiment.

Fig. 3A illustrates an example of the schematic view illustrating the structure of the nozzle 49 in the present embodiment.

Fig. 3B illustrates an example of the schematic view illustrating the structure of the nozzle 49 in the present embodiment.

Fig. 4 illustrates an example of a schematic view illustrating an example of a method of recovering an organism in the present embodiment.

Fig. 5 illustrates an example of a specific configuration of an information processing device 170 in the present embodiment.

Fig. 6 illustrates an example of a flow of a method of manipulating the organism in the present embodiment.

Fig. 7A illustrates an example of a GUI image displayed on an output unit 160 in the present embodiment.

Fig. 7B illustrates an example of the GUI image displayed on the output unit 160 in the present embodiment.

Fig. 7C illustrates an example of the GUI image displayed on the output unit 160 in the present embodiment.

Fig. 7D illustrates an example of the GUI image displayed on the output unit 160 in the present embodiment.

Fig. 7E illustrates an example of the GUI image displayed on the output unit 160 in the present embodiment.

Fig. 8 illustrates an example of a flow of replacing or adding the liquid in S600 in the present embodiment.

Fig. 9A illustrates an example of a flow of moving a relative position between the nozzle 49 and a cell in S200 in the present embodiment.

Fig. 9B illustrates an example of a flow of moving the relative position between the nozzle 49 and the cell in S200 in the present embodiment.

Fig. 9C illustrates an example of the flow of moving the relative position between the nozzle 49 and the cell in S200 in the present embodiment.

Fig. 10A illustrates an example of a flow of forming an air bubble in S300 in the present embodiment.

Fig. 10B illustrates an example of the flow of forming the air bubble in S300 in the present embodiment.

Fig. 11A illustrates an example of the flow for executing a manipulation of S400 in the present embodiment.

Fig. 11B illustrates an example of recovering cytoplasm and a cell membrane from the cell in the present embodiment.

Fig. 11C illustrates an example of separating the cell by attaching the cell to the air bubble in the present embodiment.

Fig. 11D illustrates an example of a schematic view illustrating a method of recovering the cell in the present embodiment.

Fig. 11E illustrates an example of subcultured cells in the present embodiment.

Fig. 11F illustrates an example of the subcultured cells in the present embodiment.

Fig. 11G illustrates an example of analyzing recovered cells in the present embodiment.

Fig. 11H illustrates an example of a schematic view illustrating retained cells in the present embodiment.

Fig. 11I illustrates an example of a compressed cell in the present embodiment.

Fig. 12A is an example of a flow of removing the air bubble in S500 in the present embodiment.

Fig. 12B is an example of the flow of removing the air bubble in S500 in the present embodiment.

Fig. 13A is a diagram for explaining a stabilization formula of a gas-liquid interface.

Fig. 13B is a diagram illustrating a relationship between an angle $\theta_m$ formed by a meniscus at the gas-liquid interface and a nozzle surface and a right side of the stabilization formula.

Fig. 13C is a diagram for explaining a method for stabilizing an air bubble.

Fig. 13D illustrates an example of a method of decreasing an internal volume of a flow channel of a nozzle.

Fig. 13E illustrates an example of the method of decreasing the internal volume of the flow channel of the nozzle.

Fig. 13F is a diagram illustrating a relationship between a dimensionless volume and a dimensionless curvature when a shape of an end part of the nozzle is changed.

Fig. 13G illustrates a relationship between a Laplace pressure change rate and an air bubble volume when a chamfering angle at the end part of the nozzle is changed.

Fig. 13H is a diagram illustrating the relationship between the dimensionless volume and the dimensionless curvature when a contact angle of the nozzle surface is changed.

Fig. 13I is a diagram for explaining provision of a stepped portion on the nozzle.

Fig. 13J illustrates an example of the nozzle provided with the stepped portion.

Fig. 13K is a diagram illustrating a behavior in which an air bubble is stably formed by a minute volume of gas.

Fig. 13L is a diagram illustrating a relationship between a radius of the end part of the flow channel and a maximum stable volume of the air bubble.

Fig. 14 illustrates an example of a hardware configuration of a computer.

DESCRIPTION OF EXEMPLARY EMBODIMENTS

Hereinafter, the invention will be described through embodiments of the invention, but the following embodiments do not limit the invention according to claims. In addition, not all combination of the features described in the embodiments are necessary for the solution of the invention. Note that in the drawings, same or similar parts are assigned with same reference signs, and duplicated descriptions may be omitted.

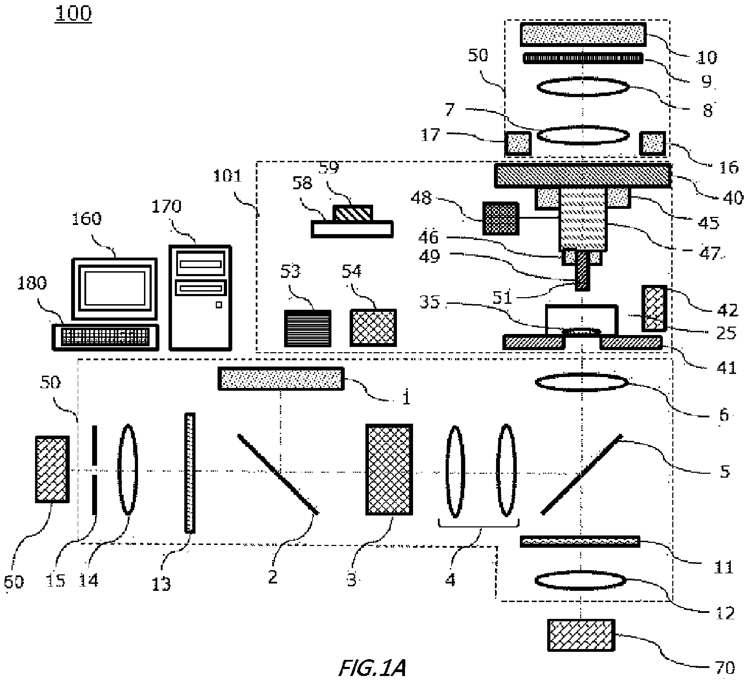

Fig. 1A illustrates an example of a device configuration of an organism manipulation device 100 in the present embodiment. The organism manipulation device 100 according to the present invention manipulates an organism such as a cell by using an interface between a gas and a liquid. For example, the organism manipulation device 100 can perform, on an organism, various manipulations such as attaching the organism to the interface and then separating the organism adhered on a solid phase. The organism manipulation device 100 includes a microscope unit 50, a camera 60, a camera 70, a gas-liquid interface manipulation unit 101, an output unit 160, an information processing device 170, and an input unit 180.

The microscope unit 50 is a device for observing or displaying a manipulation target 35 in an enlarged manner by using a microscope. The manipulation target 35 is an organism. The organism may be an organic creature. For example, the organism may be a cell. As an example, the cell may be an animal cell or a plant cell. As an example, the cell may be a living cell or a dead cell. In addition, for example, the organism may be an organism other than a cell. As an example, the organism may be a microorganism, a fungus, an alga, a biological tissue, a spheroid, or the like. In addition, the organism may contain an intracellular organelle.

The microscope unit 50 includes a fluorescence image observation light source 1, a dichroic mirror 2, an optical deflector 3, a relay lens 4, a dichroic mirror 5, an objective lens 6, a condenser lens 7, a condensing lens 8, a band pass filter 9, a transmission image observation light source 10, a barrier filter 11, a projection lens 12, a barrier filter 13, a projection lens 14, a pinhole 15, a light source 16, and a light source 17.

The fluorescence image observation light source 1 is a light source used when a fluorescence image of the manipulation target 35 is observed. The manipulation target 35 may be labeled with one type or two or more types of fluorescent substances, or may not be fluorescently labeled. The fluorescence image observation light source 1 emits, to the manipulation target 35, light to be excited or reflected.

The transmission image observation light source 10 is a light source used when a transmission image of the manipulation target 35 is observed. The transmission image observation light source 10 emits light to be transmitted through the manipulation target 35. The light to be transmitted through the manipulation target 35 may pass through the outside of the nozzle or may pass through the inside of the nozzle.

The configuration of the microscope unit 50 other than that described above will be described below. Note that the present invention is not limited to the example of the above description, and the microscope unit 50 may have a known configuration. For example, the configuration of the microscope unit 50 may have the configuration described in Japanese Patent Application Publication No. 7-13083 or Japanese Patent No. 3814869.

The camera 60 captures a fluorescence image of the manipulation target 35 to generate an image. The image data generated by the camera 60 may be recorded in the information processing device 170 (for example, a recording unit 190 to be described below) and/or output to the output unit 160. For example, the camera 60 may be a camera that captures a fluorescence image, but is not limited thereto. In the following description, the camera 60 is a camera that captures a fluorescence image.

The camera 70 captures a transmission image of the manipulation target 35 to generate an image. The image data generated by the camera 70 may be recorded in the information processing device 170 (for example, the recording unit 190 to be described below) and/or output to the output unit 160. For example, the camera 70 may be a camera that captures a transmission image, but is not limited thereto. In the following description, the camera 70 is a camera that captures a transmission image.

The camera 60 and the camera 70 include an imaging sensor (not illustrated). The camera 60 and the camera 70 may be cooling cameras. The cooling camera is a camera that can cool the imaging sensor to suppress the noise generated by heat. The imaging sensor may be a CMOS imaging sensor (Complementary Metal Oxide Semiconductor) or a CCD imaging sensor (Charge Coupled Device). The camera 60 and the camera 70 may be housed in a housing different from the microscope unit 50.

The gas-liquid interface manipulation unit 101 manipulates the manipulation target 35 by using a gas-liquid interface between a gas and a liquid. For example, the gas-liquid interface manipulation unit 101 forms an air bubble in the liquid to manipulate an organism (for example, a cell) in the liquid. The gas-liquid interface manipulation unit 101 includes all or at least some of a nozzle actuator 40, a sample actuator 41, a flow channel imaging camera 42, a light source 45, a light source 46, a pressure generating unit 47, a sensor unit 48, a nozzle 49, a flow channel 51, a flow channel exchange part 53, a liquid storage part 54, a sample lid 58, and a sample lid housing part 59. The gas-liquid interface manipulation unit 101 may form a gas-liquid interface 255, in which a restoring force acts against minute interface movement, inside or at an end part of the flow channel 51. For example, the gas-liquid interface manipulation unit 101 may release a gas from the end part 254 of the nozzle 49 to a liquid and maintain the gas-liquid interface 255 where a restoring force acts against minute interface movement at the end part 254. When the restoring force acts against the minute interface movement, the formed air bubble is stabilized, and a mechanism thereof will be described below.

The nozzle actuator 40 mounts the nozzle 49 via the pressure generating unit 47 and moves the nozzle 49. As described below, the flow channel 51 is formed inside the nozzle 49, and a gas-liquid interface 255 of the air bubble is formed at an edge part of the flow channel 51. The nozzle actuator 40 may be operable in any of a longitudinal direction, a lateral direction, and an up-and-down direction. The nozzle actuator 40 may be operable only in the up-and-down direction. In this case, the longitudinal and lateral movements of the nozzle actuator 40 may be operated by the stage of the microscope unit 50. The nozzle actuator 40 may be operable only in the longitudinal and lateral directions. In this case, the movement of the nozzle actuator 40 in the up-and-down direction may be operated by the stage of the microscope unit 50. The nozzle actuator 40 may be fixed without operation. In this case, the movement of the nozzle actuator 40 in the longitudinal and lateral directions and the up-and-down direction may be operated by the stage of the microscope unit 50. The operation of the nozzle actuator 40 is controlled by a nozzle position control part (not illustrated) of an air bubble forming unit in the information processing device 170.

The sample actuator 41 moves a stage (not illustrated) on which a vessel 25 is mounted. The sample actuator 41 may be operable in any direction of the longitudinal direction, the lateral direction, and the up-and-down direction. The transparent vessel 25 that houses the manipulation target 35 may be mounted on the stage. The vessel 25 may be a liquid-filled culture vessel. The sample actuator 41 may be mounted with one or more vessels and/or tubes, but is not limited thereto. The operation of the sample actuator 41 is controlled by a stage position control part (not illustrated) of the air bubble forming unit in the information processing device 170. Note that the stage may be provided in the gas-liquid interface manipulation unit 101 or may be provided in the microscope unit 50.

The flow channel imaging camera 42 images the edge part of the nozzle 49. The flow channel imaging camera 42 may image an air bubble formed at the edge part of the nozzle 49. The captured image may be sent to an image processing unit in the information processing device 170. On the basis of the captured image, an air bubble forming unit 200 may instruct the nozzle actuator 40 and/or the sample actuator 41 to move a relative position between the nozzle 49 and the manipulation target 35. Note that instead of the flow channel imaging camera 42, the camera 60, the camera 70, or the like may image the edge part of the nozzle 49. The present invention is not limited thereto, and in the following description, the flow channel imaging camera 42 may be a microscope-attached camera provided in the microscope unit 50. In the camera provided in the microscope unit 50, the fluorescence image observation light source 1, the transmission image observation light source 10, the light source 16, the light source 17, the light source 45, and the light source 46 may be used as illumination. The light source 16 and the light source 17 may be ring illumination, but are not limited thereto.

The light source 45 and the light source 46 illuminate the nozzle 49 and/or the manipulation target 35. The light source 45 and the light source 46 may be ring illumination, but are not limited thereto.

The pressure generating unit 47 generates a pressure to be loaded on the flow channel 51. The pressure generating unit 47 is connected to one end of the flow channel 51 which is not in contact with the liquid, and supplies a preset gas to the one end. For example, the pressure generating unit 47 may include an actuator that reciprocates a syringe pump and a plunger of the syringe pump. The actuator may supply the gas to the flow channel 51 by pushing the plunger of the syringe pump toward the flow channel 51, and the actuator may take in the gas from the flow channel 51 by drawing the plunger of the syringe pump from the flow channel 51. The pressure generating unit 47 is controlled by the air bubble forming unit in the information processing device 170.

The liquid in which the manipulation target 35 is immersed may be a complete medium, a basic medium, or a buffer solution, but is not limited thereto. The complete medium is a medium containing maintenance and proliferation factors necessary for maintenance and proliferation of cells. The basic medium is a medium containing only a small portion of proteins, amino acids or salts. The buffer solution is a liquid that maintain a pH and an osmotic pressure suitable for cell survival. As the liquid, the complete medium, the basic medium, and the buffer solution, known one can be used.

The gas may be air. The gas may contain moisture.

The sensor unit 48 includes one or more sensors, and detects the states of the nozzle 49 and the liquid and gas of the nozzle 49. For example, the sensor unit 48 may detect the position, speed, and acceleration of the nozzle 49. The sensor unit 48 may detect the position of the nozzle actuator 40, the pressure generated in the pressure generating unit 47, the position of the plunger of the syringe pump in the pressure generating unit 47, and the like. The sensor unit 48 may detect an environmental temperature and the temperature of the liquid in the vessel 25. The sensor unit 48 may detect the humidity of the environment. In addition, the sensor unit 48 may detect the pH of the liquid in the vessel 25. The sensor unit 48 may detect the temperature and humidity of the gas in the nozzle 49. The sensor unit 48 sends these pieces of information to the information processing device 170 (for example, the air bubble forming unit 200 to be described below). As the sensor used in the sensor unit 48, a known sensor can be used. Note that the sensor unit 48 may be a housing different from the pressure generating unit 47, or may be stored inside the pressure generating unit 47.

The nozzle 49 is an instrument including the flow channel 51 to be described below. The nozzle 49 may have a rod shape or a flat plate shape.

The liquid and gas to be sucked (gas intake) or discharged (gas supply) pass through the flow channel 51. The flow channel 51 is provided inside the nozzle 49 to penetrate the nozzle 49 in a longer direction. The end part 254 of the flow channel 51 may be immersed in the liquid in which the manipulation target 35 is immersed. The pressure generating unit 47 is connected to the other end side of the flow channel 51.

The flow channel exchange part 53 is an instrument that houses and discards the nozzle 49. When exchanging the nozzle 49, the flow channel exchange part 53 may remove the nozzle 49 installed in the nozzle actuator 40 and discard the nozzle in a nozzle discarding part (not illustrated) of the flow channel exchange part 53, and instead install, to the nozzle actuator 40, the nozzle 49 housed in a nozzle housing part (not illustrated) of the flow channel exchange part 53. The flow channel exchange part 53 may be omitted, and in that case, the nozzle 49 may be exchanged by the hand of a manipulator.

The liquid storage part 54 is an instrument that houses a liquid to be supplied to the vessel 25, recovers the liquid from the vessel 25, and discards the liquid. When exchanging the liquid, the liquid storage part 54 may recover the liquid accommodated in the vessel 25 from the vessel 25, discard the liquid in a liquid discarding part (not illustrated) of the liquid storage part 54, and replenish the vessel 25 with the liquid housed in a liquid housing part (not illustrated) of the liquid storage part 54. In the exchange of liquids, the same type of liquids may be exchanged with each other. In the exchange of liquids, different types of liquids may be exchanged with each other. The liquid storage part 54 may be omitted, and in that case, the liquid may be exchanged by the hand of the manipulator.

The sample lid 58 is a lid attached to the vessel 25. The sample lid 58 may be attached to the vessel 25 or may be stored in the sample lid housing part 59. As necessary, by a sample lid actuator (not illustrated), the sample lid 58 may be taken out from the sample lid housing part 59 and attached to the vessel 25, and may be removed from the vessel 25 and stored in the sample lid housing part 59. In this case, the operation of the sample lid actuator may be controlled by a sample lid control part (not illustrated) of the air bubble forming unit 200 in the information processing device 170. The sample lid 58 and the sample lid housing part 59 may be omitted, and in this case, the sample lid 58 may be attached to the vessel 25 and removed from the vessel 25 by the hand of the manipulator.

The output unit 160 outputs a processing result of the information processing device 170. For example, the output unit 160 outputs an image which has been subject to image processing by the inside (for example, an image processing unit 300 to be described below) of the information processing device 170. For example, the output unit 160 is a monitor connected to the information processing device 170.

The information processing device 170 interchanges commands and data with the microscope unit 50, the camera 60, the camera 70, the gas-liquid interface manipulation unit 101, the output unit 160, and the input unit 180. For example, the information processing device 170 is connected to the microscope unit 50 and the gas-liquid interface manipulation unit 101, and controls the microscope unit 50 and the gas-liquid interface manipulation unit 101.

Specifically, the information processing device 170 switches the combination of the type of the objective lens 6 and/or the type of a filter cube of a fluorescence filter arranged in the optical path of the microscope unit 50. For example, the transmission image observation and the fluorescence image observation are different in both the type of the filter cube and the type of the objective lens 6 arranged in the optical path. In addition, the two types of fluorescence image observation are different only in the type of the filter cube arranged in the optical path. In addition, the transmission image observation and the fluorescence image observation use different light sources (the transmission image observation light source 10 and the fluorescence image observation light source 1, respectively). Therefore, in the inside (for example, an imaging control unit 171 to be described below) of the information processing device 170, one or more of a filter block, the objective lens 6, and the light source may be switched according to whether to perform at least any one or more types of observation among the transmission image observation and one type or two or more types of fluorescence image observation.

When performing the fluorescence image observation, the information processing device 170 turns on the fluorescence image observation light source 1 and turns off the transmission image observation light source 10 in order to enable the optical path of the fluorescence image observation light source 1. When the fluorescence image observation is performed, the light emitted from the fluorescence image observation light source 1 illuminates the manipulation target 35 via the dichroic mirror 2, the optical deflector 3, the relay lens 4, the dichroic mirror 5, and the objective lens 6.

When the manipulation target 35 is fluorescently labeled, the fluorescent substance of the manipulation target 35 is excited and emits fluorescence. The fluorescence emitted from the manipulation target 35 reaches the light receiving surface of the camera 60 via the objective lens 6, the dichroic mirror 5, the relay lens 4, the optical deflector 3, the dichroic mirror 2, the barrier filter 13, the projection lens 14, and the pinhole 15 (when the microscope unit 50 is a confocal microscope). At this time, a fluorescence image of the manipulation target 35 is formed in the camera 60. Note that even when the manipulation target 35 is not fluorescently labeled, the manipulation target 35 can be observed by using the light obtained when the light emitted from the fluorescence image observation light source 1 hits the manipulation target 35 and is reflected from the manipulation target 35.

When performing the transmission image observation, the information processing device 170 turns on the transmission image observation light source 10 and turns off the fluorescence image observation light source 1 in order to enable the optical path of the transmission image observation light source 10. When performing the transmission image observation, the light emitted from the transmission image observation light source 10 illuminates the manipulation target 35 via the band pass filter 9, the condensing lens 8, and the condenser lens 7. The light transmitted through the manipulation target 35 reaches the light receiving surface of the camera 70 via the objective lens 6, the dichroic mirror 5, the barrier filter 11, and the projection lens 12. At this time, a transmission image of the manipulation target 35 is formed in the camera 70. Note that when it is difficult to see the end part of the nozzle 49 by fluorescence observation, the transmission image observation may be performed together.

In addition, the information processing device 170 controls a relative position of the nozzle 49 of the gas-liquid interface manipulation unit 101 and the stage. In addition, in addition to the control of the microscope unit 50 and the gas-liquid interface manipulation unit 101, the information processing device 170 may receive the manipulation target 35 imaged by the camera 60 and/or the camera 70, and/or an image captured by the flow channel imaging camera 42 of the gas-liquid interface manipulation unit 101 and perform image processing such as generating one composite image from a plurality of images. The information processing device 170 may perform the control of other operations of the organism manipulation device 100, data processing, and the like as necessary. The configuration of the information processing device 170 will be described below.

The input unit 180 inputs, to the information processing device 170, an instruction, data, and the like from the manipulator. For example, the input unit 180 inputs an instruction regarding selection of a manipulation application for the manipulation target 35 from the manipulator. In addition, the input unit 180 inputs, to the information processing device 170, the operation amount of the nozzle actuator 40 and/or the sample actuator 41 from the manipulator. For example, the input unit 180 is a keyboard or a mouse connected to the organism manipulation device 100.

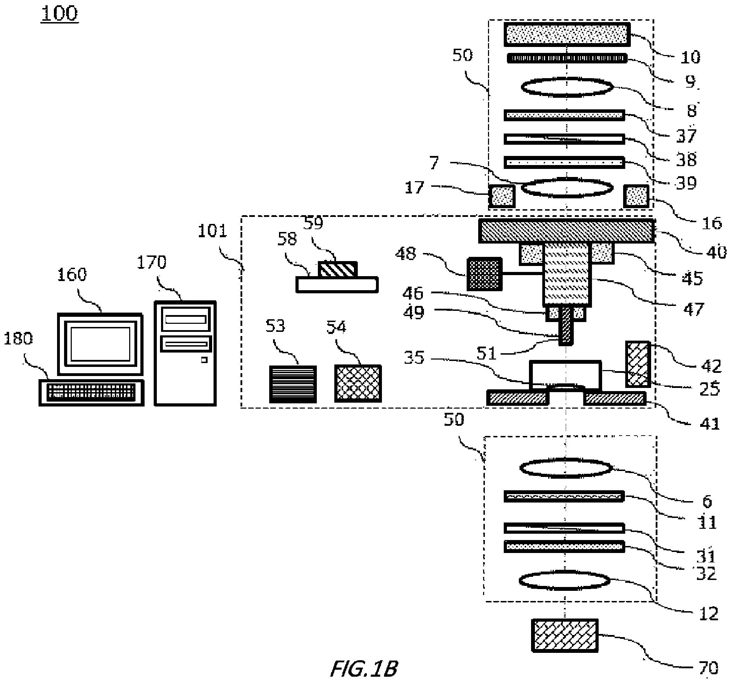

Fig. 1B illustrates another example of the device configuration of the organism manipulation device 100 in the present embodiment. Fig. 1B illustrates the organism manipulation device 100 when the microscope unit 50 is a phase contrast microscope or a differential interference microscope. When the microscope unit 50 is a phase contrast microscope, the microscope unit 50 may include the objective lens 6 (which may include a phase plate), the condenser lens 7, the condensing lens 8, the band pass filter 9, the transmission image observation light source 10, the barrier filter 11, the projection lens 12, the light source 16, the light source 17, and a ring aperture 39. When the microscope unit 50 is a differential interference microscope, the microscope unit 50 may include the objective lens 6, the condenser lens 7, the condensing lens 8, the band pass filter 9, the transmission image observation light source 10, the barrier filter 11, the projection lens 12, the light source 16, the light source 17, a Nomarski prism 31, an analyzer (polarizing plate) 32, a polarizer (polarizing plate) 37, and a Nomarski prism 38. In addition, the present invention is not limited thereto, and the microscope unit 50 may have a configuration other than those described above. For example, the phase contrast microscope may include the Nomarski prism 31 or the like, and the differential interference microscope may include the ring aperture 39 or the like. The description of Fig. 1A may be applied to the configuration of the organism manipulation device 100 other than the microscope unit 50.

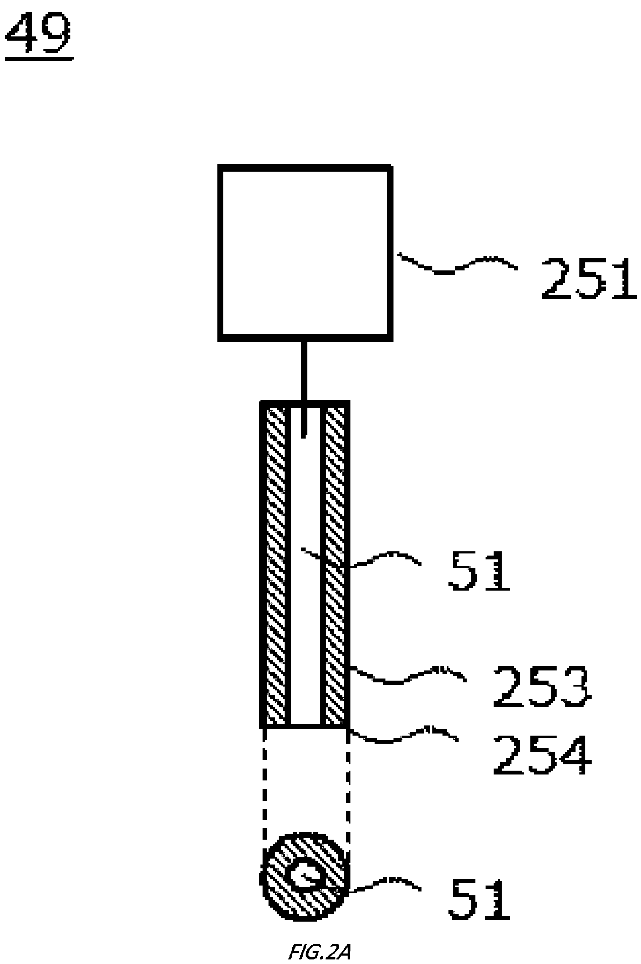

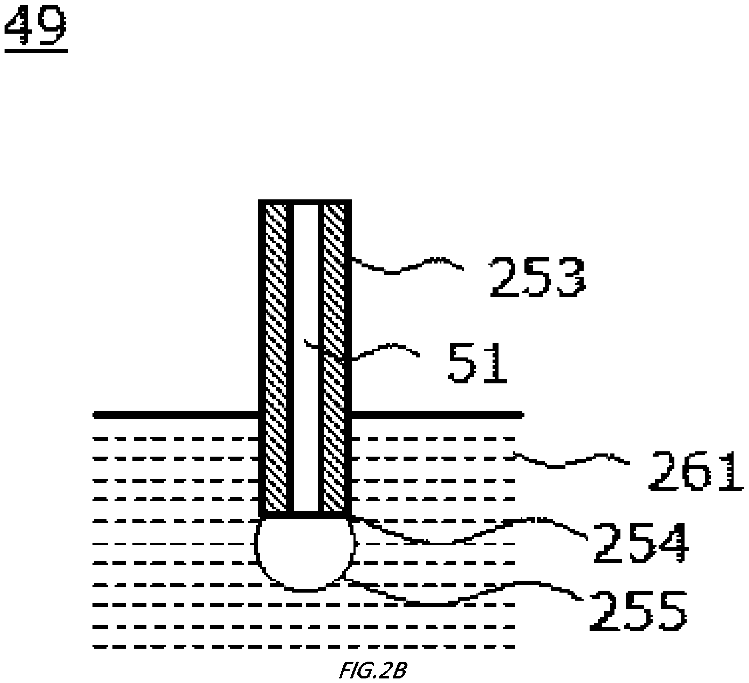

Figs. 2A and 2B are examples of schematic views illustrating a structure of the nozzle 49 in the present embodiment. In Fig. 2A, the nozzle 49 includes a cylindrical portion 253 having the flow channel 51. The cylindrical portion 253 may have a hollow cylindrical shape. In this case, the cross section of the cylindrical portion 253 orthogonal to an axial direction has a circular shape. In addition, the flow channel 51 may be connected to a pump 251 (for example, the syringe pump of the pressure generating unit 47) on one end side. When receiving an instruction from the information processing device 170 (for example, an air bubble forming unit 200 to be described below), the pump 251 adjusts the pressure and/or volume of the air bubble by adjusting the amount of the gas supplied to the flow channel 51 or taken in from the flow channel 51.

In Fig. 2B, when an end part 254 of the cylindrical portion 253 on the side not connected to the pump (not illustrated: for example, the syringe pump of the pressure generating unit 47) is arranged in the liquid 261, the pump can form an air bubble at the end part 254 by supplying gas to the flow channel 51. In this case, the gas-liquid interface 255 is formed at a boundary between the gas of the air bubble and the liquid 261. Note that the shape of the air bubble is not limited to a spherical shape, and may be deformed according to the shape of the end part 254. When gas is retained at the end part 254 of the flow channel 51, the gas-liquid interface 255 is formed at the end part 254 of the flow channel 51, but when both gas and liquid are present inside the flow channel 51, the gas-liquid interface 255 can be formed at an interface between the both inside the flow channel 51.

When the gas-liquid interface 255 comes into contact with the organism adhered on the solid phase such as the inner bottom surface of the vessel 25 in the liquid 261, the gas-liquid interface 255 is moved, so that the gas-liquid interface 225 applies a force to the organism, and the organism can be separated from the solid phase and attached to the gas-liquid interface 255. The movement of the gas-liquid interface 255 may be performed by the nozzle actuator 40 moving the nozzle 49 in which the air bubble is formed, may be performed by moving the liquid, or may be performed by changing the volume of the air bubble. The solid phase may be a surface on which adherent cells can be adhered and cultured. For example, the solid phase may be a glass; resin such as polystyrene; a metal; a surface coated with one or more types of extracellular matrix components selected from collagen, fibronectin, laminin, polylysine, or the like; a surface coated with various types of polymers (as an example, a polymer capable of controlling hydrophilicity and adsorption to cells), and the like, but is not limited thereto. Note that in the present embodiment, the gas-liquid interface 255 is formed by the interface between the gas and the liquid, but is not limited thereto, and may be changed according to a phase or a substance in contact with the interface. Details of a method of separating the organism from the solid phase will be described below.

The opening area of the flow channel 51 at the end part 254 is not particularly limited as long as the opening area has a size that allows the manipulation of the organism. For example, the opening area may be larger than the adhesion area per organism. The shape of the end part 254 is not particularly limited. In addition, the inner diameter of the flow channel 51 may be the same over the entire length of the cylindrical portion 253.

In addition, the flow channel 51 may take the gas-liquid interface 225 into the flow channel 51 by the pump 251 taking in the gas in the air bubble to which the organism is attached, and further recover the organism. Alternatively, separately from the flow channel 51, the nozzle 49 may further include another flow channel for recovering the organism.

In the embodiment of Figs. 2A and 2B, since only one flow channel 51 is formed in the nozzle 49 and only one pump 251 is connected to the flow channel 51, a considerably simple minimum configuration is obtained, and the maintenance and cost of the organism manipulation device 100 can be reduced.

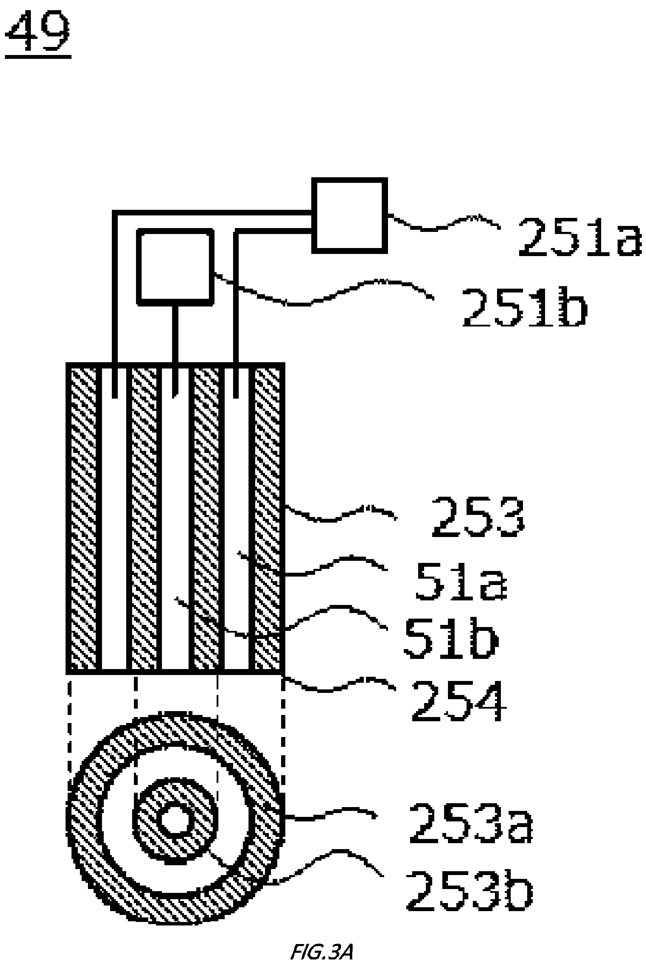

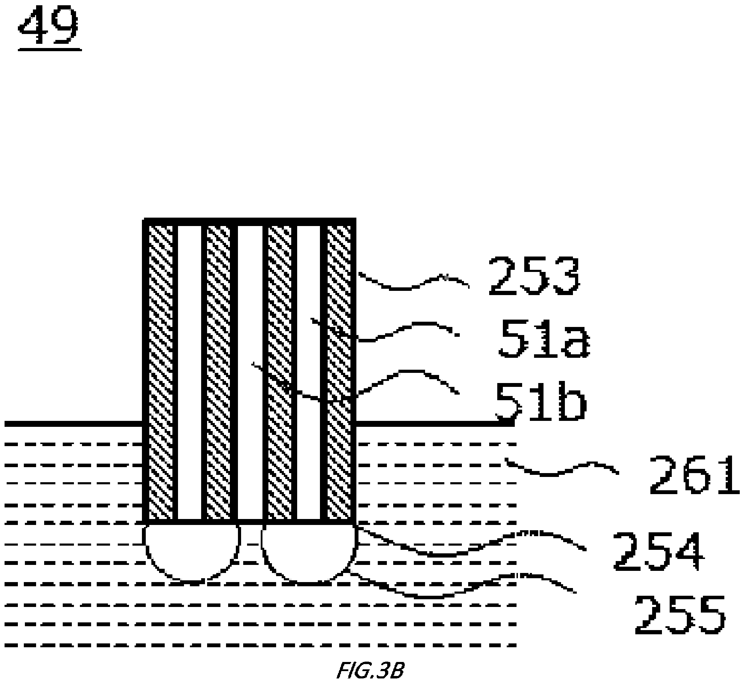

Figs. 3A and 3B are examples of schematic views illustrating the structure of the nozzle 49 in another embodiment. In the examples of Figs. 2A and 2B, a case is illustrated in which the flow channel for forming the air bubble and the flow channel for recovering the organism are the same, but in the examples of Figs. 3A and 3B, a case is illustrated in which the flow channel for forming the air bubble and the flow channel for recovering the organism are different.

In Fig. 3A, the cylindrical portion 253 of the nozzle 49 has a double structure of an outer cylinder 253a and an inner cylinder 253b. A space between the outer cylinder 253a and the inner cylinder 253b is a first flow channel 51a through which gas flows, and the inside of the inner cylinder 253b is a second flow channel 51b. For example, the first flow channel 51a may be a gas supply flow channel, and the second flow channel 51b may be a gas recovery flow channel.

In addition, the first flow channel 51a and the second flow channel 51b of the nozzle 49 may be connected to a first pump 251a and a second pump 251b, respectively, on one end side. For example, the pressure generating unit 47 may include the first pump 251a and the second pump 251b as syringe pumps, and each may be controlled by a separate actuator. Each of the first pump 251a and the second pump 251b adjusts the pressure and/or volume of the air bubble by the actuator, which has received an instruction from the air bubble forming unit 200, adjusting the amount of gas supplied to or taken into the first flow channel 51a and the second flow channel 51b. The cross section of the cylindrical portion 253 orthogonal to the axial direction has a donut shape in the first flow channel 51a and a circular shape in the second flow channel 51b.

In Fig. 3B, when the end part 254 of the cylindrical portion 253 on the side not connected to the first pump 251a and the second pump 251b is arranged in the liquid 261, an air bubble can be formed at the end part 254 by the first pump 251a supplying gas to the first flow channel 51a. In this case, the gas-liquid interface 255 is formed at a boundary between the gas of the air bubble and the liquid 261.

When the gas-liquid interface 255 comes into contact with the organism adhered on the solid phase in the liquid 261, the gas-liquid interface 255 is moved so that the organism can be separated from the solid phase, and the organism can be attached to the gas-liquid interface 255. The second pump 251b may take in the air bubble, to which the organism is attached, via the second flow channel 51b to take the gas-liquid interface 225 into the flow channel 51 and recover the organism.

In the above embodiment, the first pump 251a forms the air bubble by supplying gas to the first flow channel 51a, and the second pump 251b recovers the organism by taking in gas in the second flow channel 51b, but the second pump 251b may form the air bubble by supplying gas to the second flow channel 51b, and the first pump 251a may recover the organism by taking in gas in the first flow channel 51a. In addition, the first pump 251a and the second pump 251b may be the same syringe pump provided in the pressure generating unit 47. One of the first pump 251a and the second pump 251b may be omitted.

In the embodiment of Figs. 3A and 3B, forming the air bubble in one flow channel to attach the organism to the gas-liquid interface 255 and recovering the organism attached in another flow channel can be performed simultaneously, so that there is an effect that the time for recovering cells is shortened. Note that in Figs. 3A and 3B, the embodiment is illustrated in which the cross section of the cylindrical portion 253 orthogonal to the axial direction has the donut shape in the first flow channel 51a and the circular shape in the second flow channel 51b, but the shape of the cross section is not limited to the donut shape or the circular shape, and the attachment and recovery of the organism can be performed simultaneously as long as two flow channels are provided.

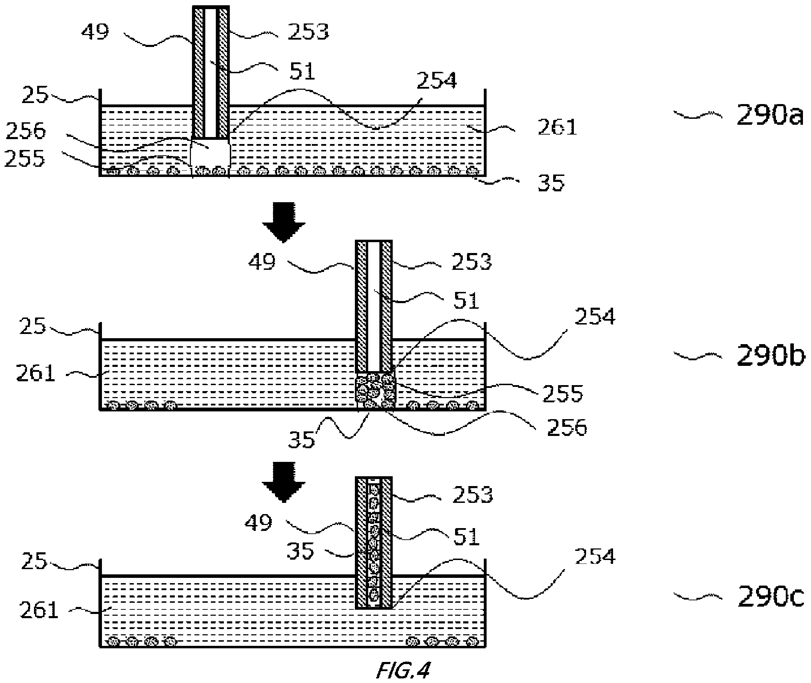

Fig. 4 is a schematic view illustrating an example of a method of recovering the manipulation target 35 in the present embodiment. In 290a, the manipulation target 35 is cultured in the solid phase on the inner bottom surface of the vessel 25. The manipulation target 35 may be cultured in the liquid 261. For example, the manipulation target 35 is an adherent cell. For example, the liquid may be a complete medium.

In 290a, the pump 251 supplies gas to the flow channel 51 of the nozzle 49 to form an air bubble 256 at the end part 254 of the nozzle 49. By bringing the air bubble 256 into contact with the manipulation target 35, the gas-liquid interface 255 between the gas and the liquid 261 comes into contact with the manipulation target 35. In this case, the pump 251 regulates the supply and intake of the gas to maintain the formed air bubble 256. In this manner, it is possible to more easily perform the manipulation of the manipulation target 35 by the air bubble 256.

Next, in 290b, the nozzle actuator 40 moves the nozzle 49 along the surface of the solid phase while keeping the air bubble 256 in contact with the manipulation target 35. In Fig. 4, a behavior is illustrated in which the nozzle actuator 40 moves the nozzle 49 from left to right, but the direction in which the nozzle 49 is moved is not limited as long as the direction is parallel to the surface of the solid phase. When the nozzle actuator 40 moves the nozzle 49, the manipulation target 35 can be separated from the solid phase. At this time, the separated manipulation target 35 is attached to the gas-liquid interface 255 of the air bubble 256. The air bubble forming unit 200 controls the pump 251 to adjust the pressure and/or volume of the gas to be supplied or taken in and change the size of the air bubble 256, so that the manipulation target 35 within a desired range can be separated. Note that instead of moving the nozzle 49 along the surface of the solid phase, the stage may be moved.

Next, in 290c, the pump 251 may take in the gas in the flow channel 51 to recover the manipulation target 35 attached to the gas-liquid interface 255. In this manner, the manipulation target 35 can be selectively separated from the solid phase and recovered by using the air bubble 256 formed in the nozzle 49.

When the manipulation target 35 is an adherent cell strongly adhered on the solid phase, the adhesion of the adherent cell may be alleviated in advance before the method of Fig. 4 is executed. The alleviation of the adhesion of the adherent cell can be performed by using a known method as described below.

In Fig. 4, an example is illustrated in which the nozzle 49 is moved to move the gas-liquid interface 255, and the cells are separated and recovered, but the movement of the gas-liquid interface 255 is not limited to the above example. For example, the volume of the air bubble 256 may be increased after the air bubble 256 is brought into contact with the manipulation target 35. In this case, a contact surface between the air bubble 256 and the solid phase expands, and the manipulation target can be selectively separated from the solid phase and recovered. For example, after the air bubble 256 is brought into contact with the manipulation target 35, the nozzle actuator 40 may move the nozzle 49 to approach the solid phase. Also in this case, when the air bubble 256 is pressed against the solid phase, the contact surface between the air bubble 256 and the solid phase expands, and the manipulation target can be selectively separated from the solid phase and recovered.

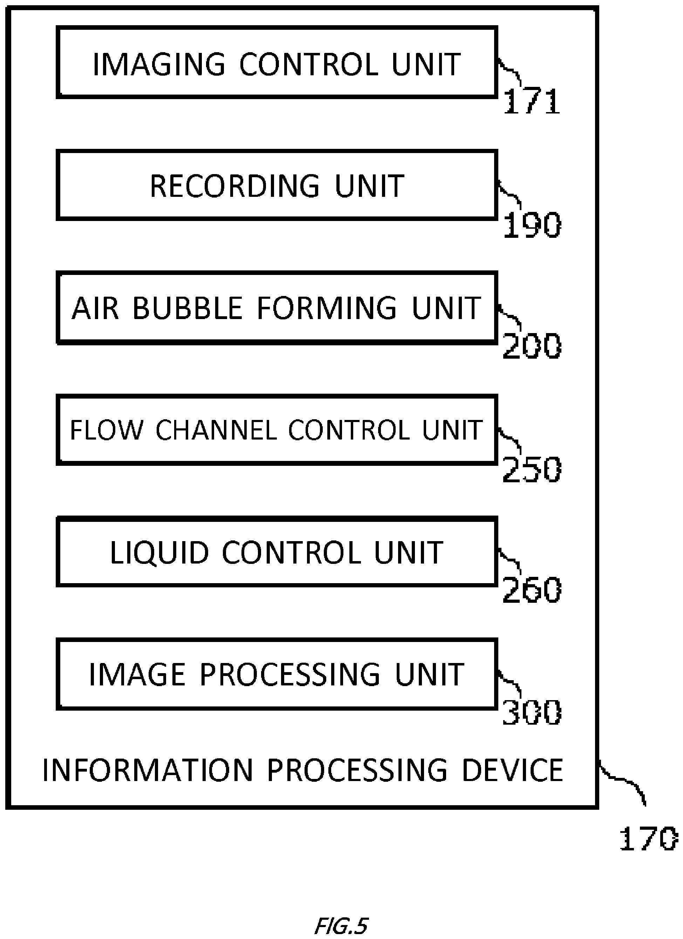

Fig. 5 illustrates an example of a specific configuration of the information processing device 170 in the present embodiment. The information processing device 170 includes the imaging control unit 171, the recording unit 190, the air bubble forming unit 200, a flow channel control unit 250, a liquid control unit 260, and an image processing unit 300.

The imaging control unit 171 controls the fluorescence image observation light source 1, the objective lens 6, the fluorescence filter, the transmission image observation light source 10, the flow channel imaging camera 42, the light source 45, the light source 46, the camera 60, the camera 70, and the like described in Figs. 1A and 1B. For example, when the imaging condition of the manipulation target 35 is input to the input unit 180, according to the input imaging condition, the imaging control unit 171 performs necessary regulation for each imaging in the switching of the camera, the switching of the type of the objective lens 6 in the microscope unit 50, the switching of the light source, the switching of the type of the fluorescence filter, the position of the stage, and the height of the objective lens 6. After the imaging control unit 171 performs the necessary regulation, one or more cameras among the flow channel imaging camera 42, the camera 60, and the camera 70 image the manipulation target 35 or the nozzle 49, and generate an image of the manipulation target 35 or the nozzle 49. The one or more cameras send data of the generated image to the image processing unit 300. In addition, the generated image data may also be recorded in the recording unit 190 and/or output to the output unit 160.

The recording unit 190 may be a memory, an internal hard disk drive, or an external recording medium, but is not limited thereto. The information processing device 170 includes a central processing unit (CPU), and the CPU executes a computer program recorded in the recording unit 190 to realize the information processing device 170 or the like.

The air bubble forming unit 200 controls the pressure and volume of the air bubble formed in the flow channel 51, the gas supply, the gas intake, the movement of the nozzle 49, and the movement of the stage. The air bubble forming unit 200 may include all or some of a nozzle position control part, a stage position control part, a volume control part, a gas supply control part, and a gas intake control part.

The nozzle position control part controls the nozzle actuator 40 to control the operation of the nozzle 49, the air flow in the air bubble accompanying the operation of the nozzle 49, and the movement of the gas-liquid interface 255 accompanying the operation of the nozzle 49. In addition, the nozzle position control part receives the position information of the nozzle 49 from the sensor unit 48 or the nozzle actuator 40.

The stage position control part controls the sample actuator 41 to control the operation of the stage on which the vessel 25 for accommodating the manipulation target 35 is mounted, the air flow in the air bubble accompanying the operation of the stage, and the movement of the gas-liquid interface 255 accompanying the operation of the stage. The stage position control part receives the position information of the stage and the manipulation target 35 from the sensor unit 48 or the sample actuator 41.

The volume control part controls the actuator of the pressure generating unit 47 to supply the gas from the syringe pump or take in the gas into the syringe pump, thereby controlling the pressure and/or volume of the air bubble formed in the flow channel 51. In addition, the volume control part receives the information on the pressure and/or volume of the air bubble from the nozzle actuator 40, the pressure generating unit 47, or the sensor unit 48.

In addition, when the nozzle 49 includes a gas supply flow channel for supplying (gas supply) gas and a gas recovery flow channel for recovering (gas intake) gas, the volume control part or the gas supply control part controls the first pump 251a connected to the gas supply flow channel, thereby controlling the volume of the gas to be supplied to the gas supply flow channel. The volume control part or the gas supply control part receives, from the nozzle actuator 40, the pressure generating unit 47, or the sensor unit 48, the information on the amount of the gas to be supplied to the gas supply flow channel.

In addition, when the nozzle 49 includes a gas supply flow channel for supplying (gas supply) gas and a gas recovery flow channel for recovering (gas intake) gas, the volume control part or the gas intake control part controls the second pump 251b connected to the gas recovery flow channel, thereby controlling the amount (volume) of the gas to be taken in from the gas recovery flow channel. The volume control part or the gas intake control part receives, from the nozzle actuator 40, the pressure generating unit 47, or the sensor unit 48, the information on the amount of the gas to be taken in from the gas recovery flow channel.

The flow channel control unit 250 controls housing, installation, and discarding of the nozzle 49. The flow channel control unit 250 receives, from the input unit 180, a manipulation instruction regarding the installation and discarding of the nozzle 49 from the manipulator. In accordance with the received instruction, the flow channel control unit 250 instructs the flow channel exchange part 53 to take out the nozzle 49 from a flow channel housing part of the flow channel exchange part 53 and install the nozzle 49 in the nozzle actuator 40 or to remove the nozzle 49 installed in the nozzle actuator 40 and discard the nozzle in the flow channel discarding part of the flow channel exchange part 53.

The liquid control unit 260 controls housing, replenishment, and discarding of the liquid. The liquid control unit 260 receives, from the input unit 180, a manipulation instruction regarding the replenishment and discarding of the liquid from the manipulator. In accordance with the received instruction, the liquid control unit 260 instructs the liquid storage part 54 to replenish the vessel 25 with the liquid housed in the liquid housing part of the liquid storage part 54 or to recover the liquid accommodated in the vessel 25 from the vessel 25 and discard the liquid in the liquid discarding part of the liquid storage part 54.

The image processing unit 300 receives the images captured by the flow channel imaging camera 42, the camera 60, and the camera 70 from these cameras. The image processing unit 300 may use a plurality of images among the received images to perform synthesis into one composite image. For example, the image processing unit 300 may synthesize the fluorescence image captured by the camera 60 and the transmission image captured by the camera 70 to generate a composite image. The image processing unit 300 may record the images received from these cameras and/or the composite image in the recording unit 190 and/or output them to the output unit 160.

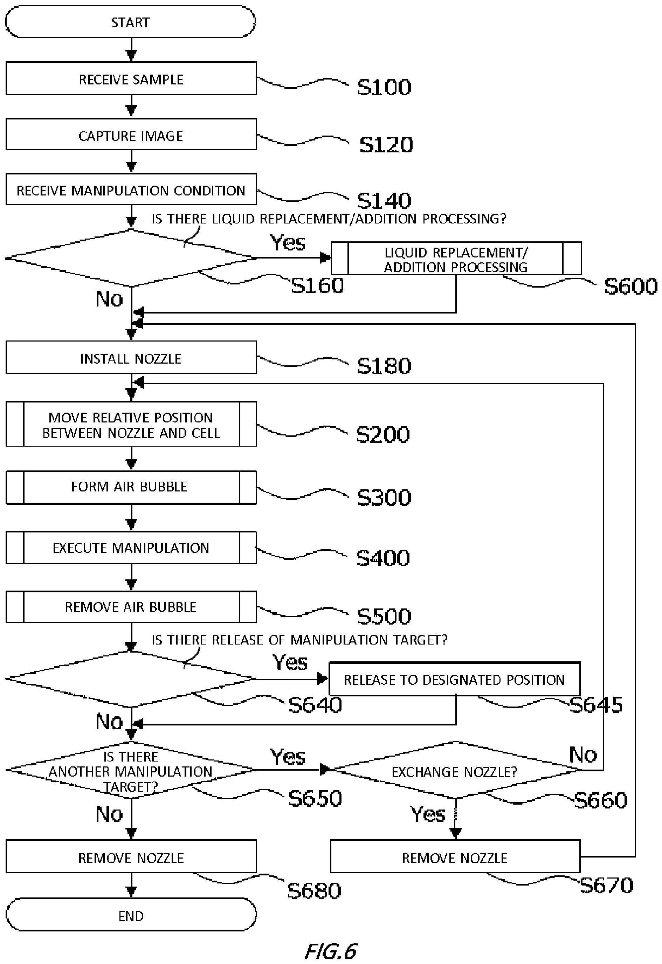

Fig. 6 is an example of a flow of a method of manipulating the organism in the present embodiment. The organism to be the manipulation target 35 of the present embodiment can be manipulated by performing processing of S100 to S680 of Fig. 6. Note that, for convenience of description, the processing of S100 to the processing of S680 will be described in order; however, at least some processing may be executed in parallel, and each step may be interchanged and executed within a range not deviating from the spirit of the present invention.

First, in S100, the sample actuator 41 receives an organism to be the manipulation target 35. For example, in S100, the sample actuator 41 mounts the vessel 25 accommodating the manipulation target 35 together with the liquid on the stage. In order to manipulate the manipulation target 35, the lid of the vessel 25 may be removed. The lid may be exchanged by an actuator for exchanging the lid, or may be exchanged by the hand of the manipulator. After the sample actuator 41 receives the organism to be the manipulation target 35, the information processing device 170 proceeds the processing to S120.

Next, in S120, the camera 60 or the camera 70 images a wide range of observation field including the manipulation target 35 to generate an image. The imaging control unit 171 sets an observation method to low magnification transmission image capturing and instructs the camera 70 to image the observation field. The imaging control unit 171 may set the observation method to fluorescence image capturing and instruct the camera 60 to capture an image of the observation field. The imaging control unit 171 may receive, via the input unit 180, the input of imaging conditions from the manipulator. The camera 60 or the camera 70 captures an image of the observation field. The image processing unit 300 may record the captured image in the recording unit 190 and/or output the captured image to the output unit 160. After the camera 60 or the camera 70 images the observation field, the imaging control unit 171 proceeds the processing to S140.

Next, in S140, the information processing device 170 receives, via the input unit 180, an input regarding the manipulation target 35 and the type of the manipulation from the manipulator. The manipulation target 35 may be a single cell, a population (colony) of cells, a cytoplasm and/or a cell membrane of a cell, or a spheroid, but is not limited thereto. The type of the manipulation may be the recovery of the manipulation target 35, the removal of the manipulation target 35, the retention of the manipulation target 35, or the compression of the manipulation target 35, but is not limited thereto.

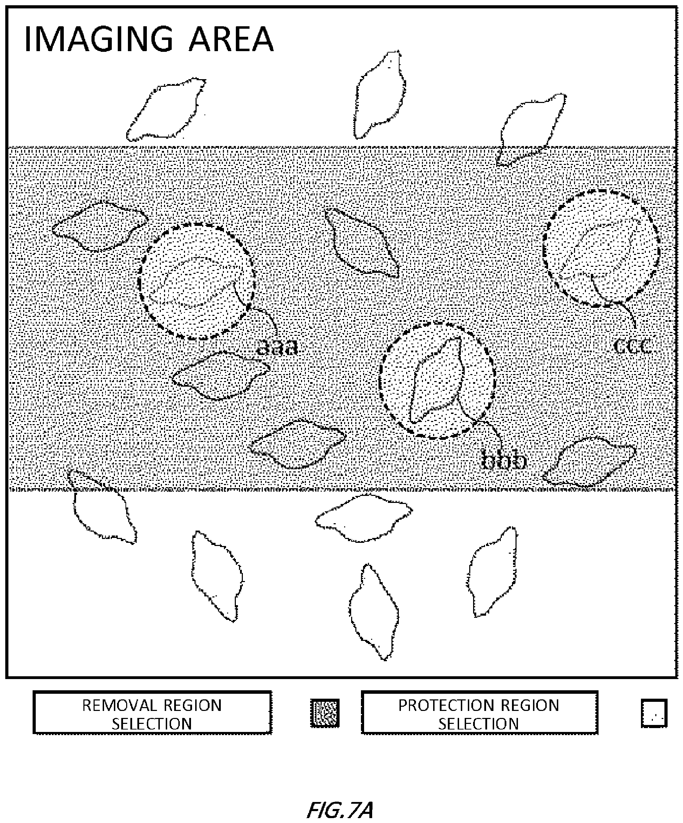

Fig. 7A is an example of a graphical user interface (GUI) image displayed on the output unit 160 and obtained by the camera 60 or the camera 70 imaging the observation field. In Fig. 7A, cells aaa, bbb, and ccc to be manipulation targets are designated as the manipulation target 35 via the input unit 180. As illustrated in Fig. 7A, the organism to be the manipulation target 35 is arbitrarily designated via the input unit 180. As illustrated in Fig. 7A, a removal region and/or a protection region may be provided in the observation field such that the removal region and/or the protection region is selected in the GUI image. By providing the removal region, it is possible to reduce a risk of recovering cells other than the cells to be recovered. In addition, by providing the protection region, it is possible to reduce a risk of erroneously removing recovered cells when removing the cells in the removal region.

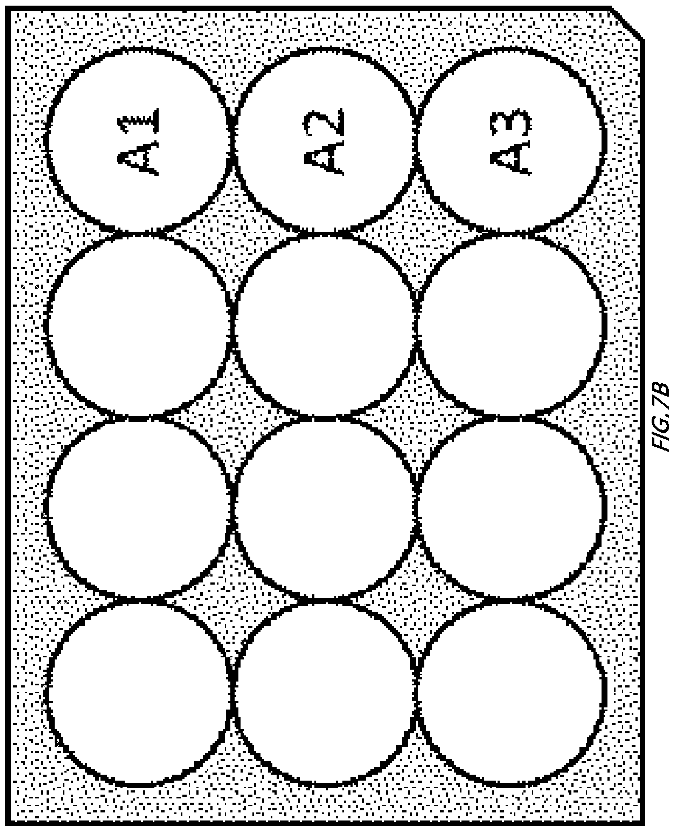

Fig. 7B is an example of a GUI image which is displayed on the output unit 160 and in which the recovery destinations and the movement destinations of the cells aaa, bbb, and ccc to be the manipulation target 35 are designated as A1, A2, and A3 of twelve hole plates, respectively. As illustrated in Fig. 7B, the movement destination is arbitrarily designated via the input unit 180. For example, the movement destination may be the same plate, a different plate, a petri dish, a micro test tube, a PCR tube, or a conical tube.

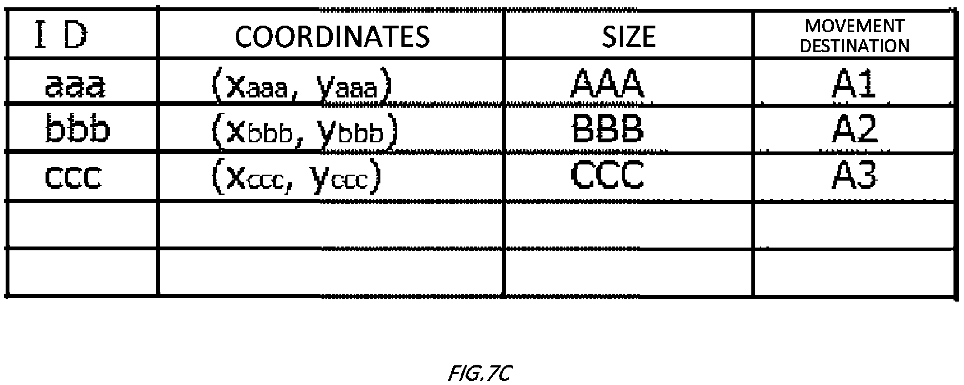

Fig. 7C is an example of a GUI image which is displayed on the output unit 160 and shows a table listing the ID number of the cell to be the manipulation target 35, the xy coordinates of the manipulation target 35 on the sample actuator 41, the size of the manipulation target 35, and the movement destination of the manipulation target 35. In the table illustrated in Fig. 7C, a case is shown in which the ID number, the xy coordinate, the size, and the movement destination are set as items, but the items to be displayed are not limited thereto. In this manner, the image processing unit 300 may output the table to the output unit 160 by designating the manipulation target 35, the movement destination, and the like via the input unit 180.

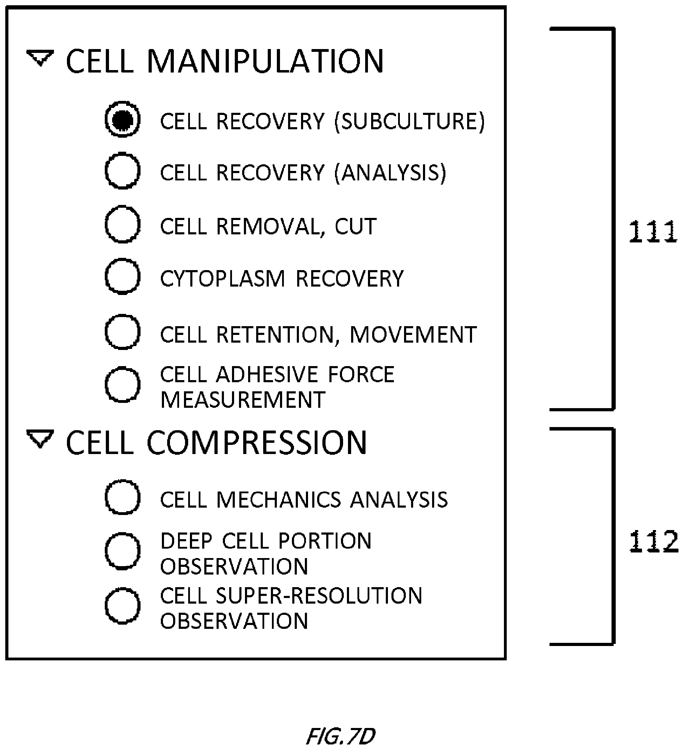

Fig. 7D is an example of a GUI screen, which is displayed on the output unit 160, for selecting the type of the manipulation of the manipulation target 35. The input unit 180 receives, from the manipulator, an instruction as to what manipulation is desired to be performed on the manipulation target 35, and inputs the instruction to the information processing device 170. For example, as shown in the display region 111, the manipulation on the cell may be subculture or retention or movement of the cell, but is not limited thereto. For example, as shown in the display region 112, the manipulation on the cell may be to compress the cell and observe the deep portion of the cell, but is not limited thereto. Fig. 7D illustrates an example in which the type of the manipulation is selected by a radio button, but a selection method is not limited to the radio button.

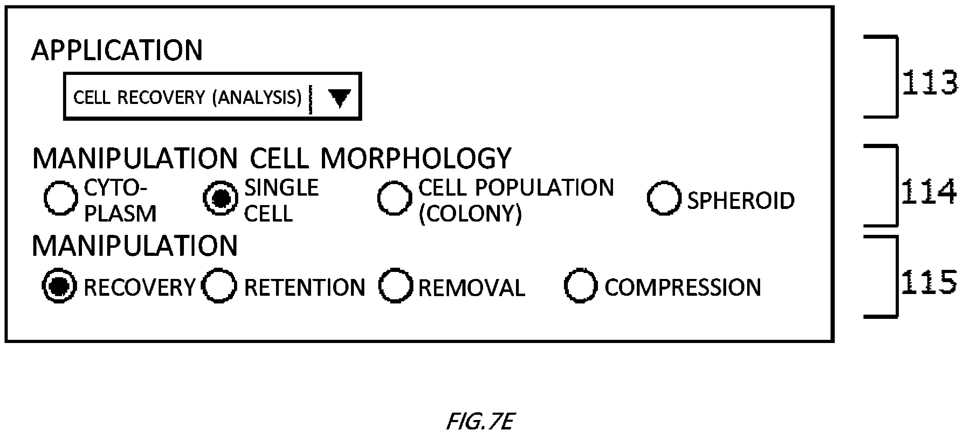

Fig. 7E is another example of the GUI screen, which is displayed on the output unit 160, for selecting the type of the manipulation of the manipulation target 35. For example, as shown in the display region 113, the type of the manipulation on the cell may be selected by a pull-down menu. For example, as shown in the display region 113, the display region 114, and the display region 115, the pull-down menu and the radio button may be used together for selection of the type of the manipulation.

The input unit 180 may send, to the information processing device 170, the instruction input from the manipulator on the basis of the screen illustrated in Figs. 7A to 7E. After the information processing device 170 receives the instruction, the imaging control unit 171 proceeds the processing to S160.

In S160, when the manipulation target 35 is an adherent cell which strongly adheres on the solid phase, a step of alleviating the adhesion of the adherent cell in advance may be additionally performed. In this case, in a step of receiving the manipulation condition of S140, the information processing device 170 may receive an input as to whether to perform processing of alleviating the adhesion.

The alleviation of the adhesion of the adherent cell can be performed by using a known method. For example, the alleviation of the adhesion of the adherent cell may be performed by removing a liquid (for example, medium), washing with a buffer solution, and then processing the adherent cell with an adhesion alleviation solution. For example, the adhesion alleviation solution may be a protease solution, a solution containing no metal ion, or a chelating agent solution. As an example, the adhesion alleviation solution is a trypsin-EDTA solution. The adhesion of the adherent cells may be alleviated by the liquid control unit 260 or may be alleviated by the hand of the manipulator. After processing the adherent cells with the adhesion alleviation solution to weaken an adhesive force, the processing may proceed to S160. Note that the step is not limited to the alleviation of the adhesion of the adherent cell, and when the step is performed by the hand of the manipulator, the step may be started again from the sample reception step of S100. In addition, instead of the processing with the adhesion alleviation solution, the adhesive force may be weakened by using a substrate which alleviates the adhesion. For example, the substrate which alleviates the adhesion may be one that alleviates the adhesion in response to temperature or light irradiation.

Next, in S160, the information processing device 170 receives, via the input unit 180, an input regarding liquid replacement or addition processing from the manipulator. For example, when it is desired to regulate an attachment force between the organism to be the manipulation target 35 and the air bubble, the information processing device 170 may receive an input to perform the liquid replacement or addition processing. When the information processing device 170 receives an instruction to perform the liquid replacement or addition processing, the information processing device 170 may proceed the processing to S600. When the information processing device 170 receives an instruction not to perform the liquid replacement or addition processing, the information processing device 170 may proceed the processing to S180.

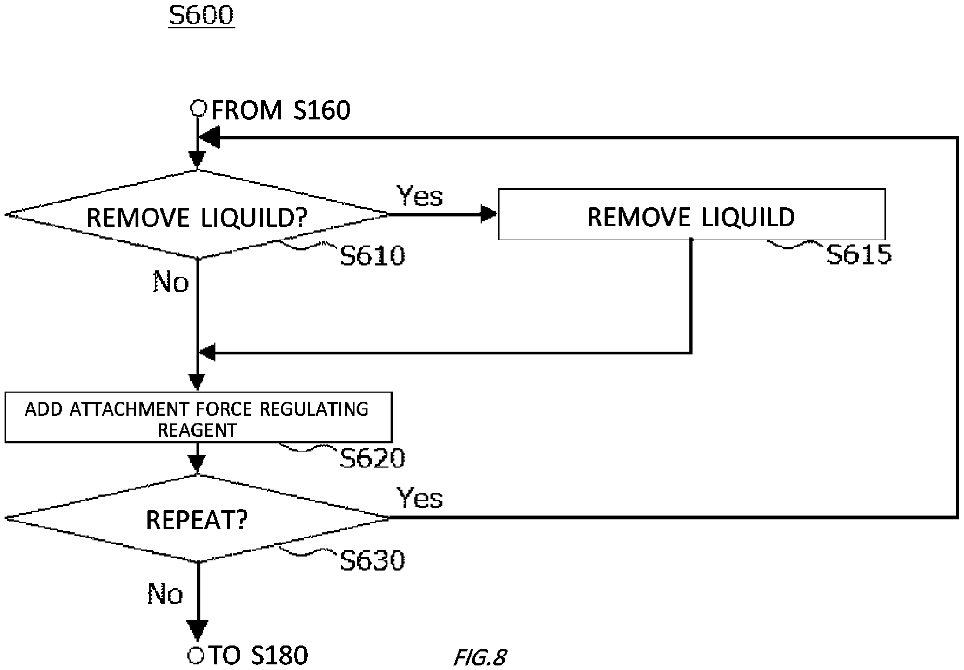

In S600, the liquid control unit 260 replaces the liquid in the vessel 25 accommodating the manipulation target 35 or adds another liquid to the liquid in the vessel 25. In S600, the step of performing the liquid replacement or addition processing includes steps S610 to S630 as illustrated in Fig. 8.

First, in S610, the information processing device 170 receives, via the input unit 180, an input regarding whether to remove the liquid from the manipulator. When the information processing device 170 receives an instruction to remove the liquid, the processing proceeds to S615. When the information processing device 170 receives an instruction not to remove the liquid, the processing proceeds to S620.

In S615, the liquid control unit 260 controls the liquid storage part 54 to remove the liquid. For example, the liquid control unit 260 sends an instruction to the liquid storage part 54 to recover, from the vessel 25, a preset amount of liquid accommodated in the vessel 25 and discard the liquid in the liquid discarding part of the liquid storage part 54. At this time, the liquid storage part 54 may recover the entire amount of the liquid and discard the liquid. Alternatively, the liquid storage part 54 may recover a part (for example, half amount) of the liquid and discard the liquid. After the liquid storage part 54 recovers the liquid, the liquid control unit 260 proceeds the processing to S620.

In S620, the liquid control unit 260 sends an instruction to the liquid storage part 54 to add an attachment force regulating reagent to the vessel 25. The attachment force regulating reagent is a reagent for regulating the attachment force between the organism and the air bubble. For example, the attachment force regulating reagent may change the concentration of inorganic salts and/or the concentration of amphipathic substances in the liquid. As an example, the attachment force regulating reagent may be a buffer solution containing or not containing at least one of calcium ions or magnesium ions, may be a basal medium, may be a complete medium, or may be a chelating agent. At this time, the liquid storage part 54 replenishes the vessel 25 with the attachment force regulating reagent housed in the liquid housing part of the liquid storage part 54.

In the above example, an example has been described in which the vessel 25 is replenished with the attachment force regulating reagent, but instead of adding the attachment force regulating reagent to the vessel 25, the attachment force between the organism and the air bubble may be regulated by attaching an inorganic salt, an amphipathic substance, or the like contained in the liquid to a filter or the like to remove the inorganic salt, the amphipathic substance, or the like.

In S630, the information processing device 170 receives, via the input unit 180, an instruction regarding whether to repeat the above-described series of manipulations from the manipulator. When the information processing device 170 receives an instruction to repeat the series of manipulations, the information processing device 170 proceeds the processing to S610, and the liquid control unit 260 sends an instruction to the liquid storage part 54 to remove the liquid in the vessel 25. When the information processing device 170 receives an instruction not to repeat the series of manipulations, the information processing device 170 proceeds the processing to S180. Note that the steps and sub-steps of S600 may be performed by the hand of the manipulator, or in this case, the processing may start again from the sample reception step of S100.

In S180, the nozzle actuator 40 installs the nozzle 49. For example, the information processing device 170 receives, from the manipulator via the input unit 180, an instruction to install the nozzle 49 to the nozzle actuator 40. According to the instruction, the flow channel control unit 250 takes out the nozzle 49 from the flow channel housing part of the flow channel exchange part 53, and sends an instruction to the flow channel exchange part 53 to install the nozzle 49 to the nozzle actuator 40. At this time, the suitable nozzle 49 may be selected according to the size of the manipulation target 35, the type of manipulation, and the like. The selection of the nozzle 49 may be designated by the manipulator via the input unit 180, or may be automatically designated by the flow channel control unit 250. After the nozzle actuator 40 installs the nozzle 49, the flow channel control unit 250 proceeds the processing to S200. Note that when the nozzle 49 does not need to be installed in the nozzle actuator 40, for example, in a state where the nozzle actuator 40 is installed in the nozzle actuator 40 in advance or a state where the nozzle 49 and the nozzle 49 are integrally formed, the step of S180 may be omitted.

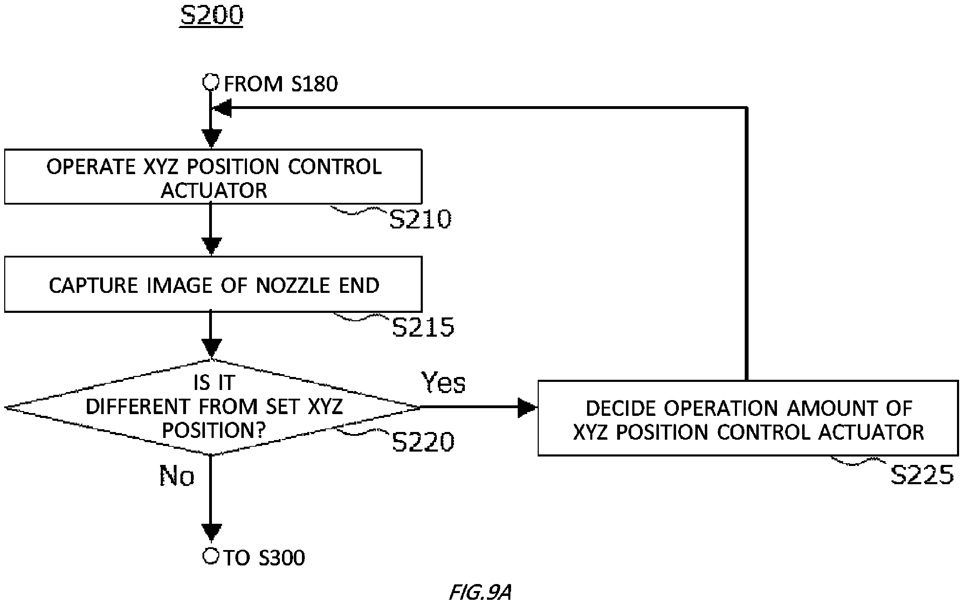

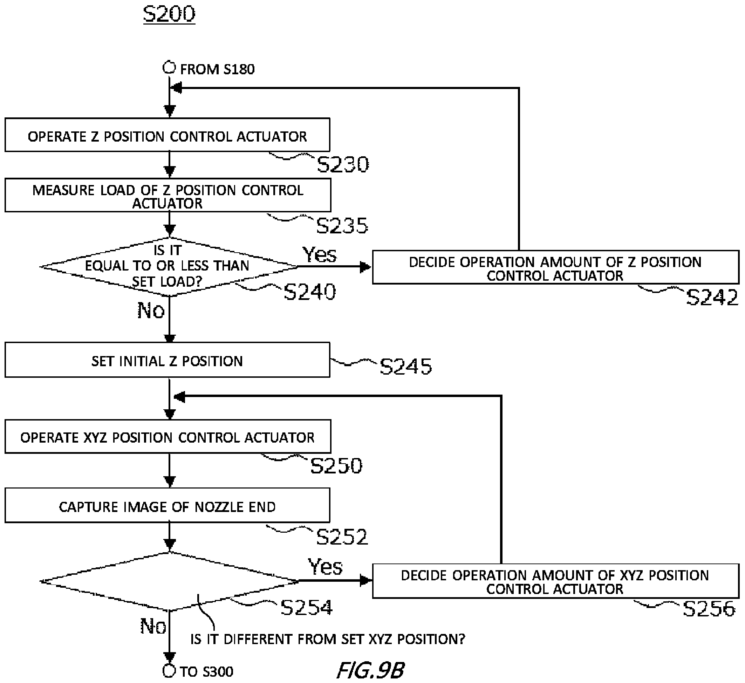

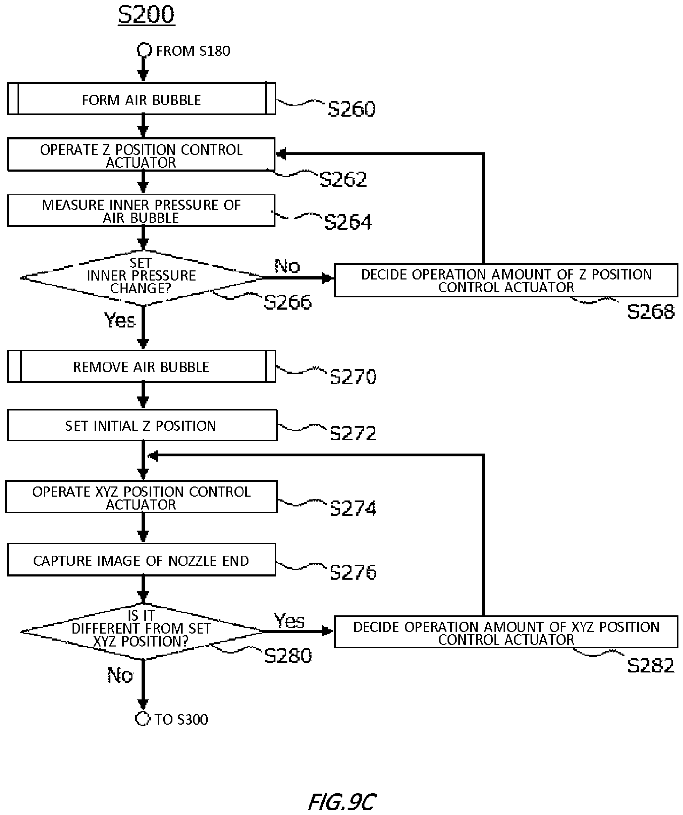

Next, in S200, the nozzle actuator 40 moves the relative position between the nozzle 49 and the manipulation target 35. For example, the air bubble forming unit 200 sends an instruction to the nozzle actuator 40 to move the relative position between the nozzle 49 and the manipulation target 35. In S200, a step of moving the relative position includes steps of S210 to S225, as illustrated in Fig. 9A, steps of S230 to S256, as illustrated in Fig. 9B, or steps of S260 to S282, as illustrated in Fig. 9C.

Fig. 9A is an example of a flow in which the relative position between the nozzle 49 and the manipulation target 35 is moved on the basis of an image obtained by imaging the position of the end part 254 of the nozzle 49.

First, in S210, the air bubble forming unit 200 sends an instruction to the nozzle actuator 40 to operate the nozzle 49 to a preset position. The nozzle actuator 40 may be an actuator which controls an xyz position. A z position may be a position in a vertical direction (also referred to as a direction along gravity, an up-and-down direction, and a z direction), an x position may be a position in an arbitrary x direction (also referred to as a longitudinal direction) perpendicular to the z direction, and a y position may be a position in the x direction and a y direction (also referred to as a lateral direction) perpendicular to the z direction.

In the position of the nozzle 49, the z position may be set by first focusing the camera 60 and/or the camera 70 on the bottom surface of the vessel 25, then moving the focus of the camera 60 and/or the camera 70 upward by an arbitrary distance, and then the nozzle actuator 40 aligning the edge of the nozzle 49 with the focus of the camera 60 and/or the camera 70. For example, the arbitrary distance may be less than or equal to the radius of the air bubble formed at the end part 254 of the nozzle 49. When a distance between the edge of the nozzle 49 and the bottom surface of the vessel 25 is equal to or less than the radius of the air bubble to be formed, the air bubble comes into contact with the bottom surface, so that the organism positioned on the bottom surface can be manipulated by using the interface of the air bubble. In this case, on the basis of an image obtained by imaging the manipulation target 35 by using the camera 60 and/or the camera 70, the xy position of the nozzle 49 can be set by using the nozzle actuator 40 or the sample actuator 41.

In addition, the position of the nozzle 49 may be set by using the flow channel imaging camera 42 instead of the camera 60 and/or the camera 70 or together with the camera 60 and/or the camera 70. For example, in the regulation of the z position of the nozzle 49, the edge of the nozzle 49 and the bottom surface of the vessel 25 may be imaged from the lateral direction of the nozzle 49 by using the flow channel imaging camera 42 to set the z position. Furthermore, the shape of the air bubble, the liquid amount in the flow channel 51, or the like may be confirmed by imaging from the lateral direction of the nozzle 49 by using the flow channel imaging camera 42. After the nozzle actuator 40 moves the nozzle 49 to the preset position, the air bubble forming unit 200 proceeds the processing to S215.

Next, in S215, the flow channel imaging camera 42 captures an image of the end part 254 of the nozzle 49. The flow channel imaging camera 42 sends the captured image to the image processing unit 300. The image processing unit 300 may record the image in the recording unit 190 and/or output the image to the air bubble forming unit 200.

Next, in S220, on the basis of the captured image of the end part 254 of the nozzle 49, the air bubble forming unit 200 determines whether the position of the nozzle 49 is different from the preset position. For example, the air bubble forming unit 200 calculates a difference in position from the captured image of the end part 254 of the nozzle 49 and the image of the end part 254 of the nozzle 49 at a preset set xyz position (that is, an initial position), and determines that the position of the nozzle 49 is different from the initial position when the difference is equal to or larger than a threshold.

When it is determined that the position of the nozzle 49 is different from the initial position, the air bubble forming unit 200 proceeds the processing to S225, and if not, proceeds the processing to S300.

In S225, the air bubble forming unit 200 decides the operation amount of the nozzle actuator 40. For example, the air bubble forming unit 200 decides the operation amount of the nozzle actuator 40 in order to operate the nozzle 49 to the preset xyz position (that is, the initial position), and sends an instruction to the nozzle actuator 40 to operate by the decided operation amount. For example, the air bubble forming unit 200 may decide the operation amount of an amount according to the level of the difference calculated in S220. The nozzle actuator 40 receives the instruction, and the processing proceeds to S210. In S210 of the second and subsequent times, the air bubble forming unit 200 sends an instruction to the nozzle actuator 40 to perform an operation by the amount according to the operation amount. For example, the air bubble forming unit 200 may send an instruction to the nozzle actuator 40 to move the relative position between the nozzle 49 and the manipulation target 35 close to each other. At this time, the nozzle actuator 40 can move the relative position between the nozzle 49 (flow channel 51) and the manipulation target 35 closer to each other.

Fig. 9B is an example of a flow of moving the relative position between the nozzle 49 and the manipulation target 35 on the basis of the load sensed by the nozzle actuator 40.

First, in S230, the air bubble forming unit 200 sends an instruction to the nozzle actuator 40 to operate the nozzle 49 to the preset position. The nozzle actuator 40 may be an actuator which controls the z position. In this case, the z position is controlled on the basis of the value of the load sensed by the nozzle actuator 40, and contact or proximity information. The nozzle 49 may be positioned above a region where no organism is present on the bottom surface of the vessel 25. After the nozzle actuator 40 moves the nozzle 49 to the preset position, the air bubble forming unit 200 proceeds the processing to S235.

Next, in S235, the sensor unit 48 measures the applied load and the contact or proximity information of the nozzle actuator 40, and sends the measured value to the air bubble forming unit 200. As an example of the load detection, when the nozzle 49 reaches the bottom portion of the vessel 25, the load sensed by the nozzle actuator 40 rapidly increases. Therefore, by measuring the value of the load sensed by the nozzle actuator 40, the air bubble forming unit 200 can determine whether the nozzle 49 has reached the bottom portion of the vessel 25. In addition, as another example of the load detection, the sensor unit 48 may sense the load while the nozzle actuator 40 moves the nozzle 49 downward. Note that instead of the sensor unit 48, the nozzle actuator 40 may send, to the air bubble forming unit 200, the value of the load to be sensed.

Next, in S240, the air bubble forming unit 200 determines whether the value of the measured load is equal to or less than a set load. When the value of the measured load is equal to or less than the set load, the air bubble forming unit 200 proceeds the processing to S242, and if not, proceeds the processing to S245. As described above, a difference between the load set by the air bubble forming unit 200 and the measured load is calculated, and when the value of the difference is equal to or larger than a threshold, it is determined that the nozzle 49 has not reached the bottom portion of the vessel 25.

In S242, the air bubble forming unit 200 decides the operation amount of the nozzle actuator 40. For example, the air bubble forming unit 200 decides the operation amount of the nozzle actuator 40 in order to operate the nozzle 49 to the preset position, and sends an instruction to the nozzle actuator 40 to operate by the decided operation amount. For example, the air bubble forming unit 200 may decide the operation amount of an amount according to the level of the difference calculated in S240. The nozzle actuator 40 receives the instruction, and the processing proceeds to S230. In S230 of the second and subsequent times, the air bubble forming unit 200 sends an instruction to the nozzle actuator 40 to perform an operation by the amount according to the operation amount.

In S245, the air bubble forming unit 200 sets the initial z position of the nozzle 49. For example, the air bubble forming unit 200 may set the z position of the nozzle 49 as the initial z position without moving the z position after the last S230. Alternatively, the air bubble forming unit 200 may move the nozzle 49 in the z direction by a preset arbitrary distance from the bottom surface of the vessel 25 and set the position thereof as the initial z position. As a result, the initial z position of the nozzle 49 is positioned above the bottom surface by the arbitrary distance. For example, the arbitrary distance may be less than or equal to the radius of the air bubble formed at the end part 254 of the nozzle 49. After the air bubble forming unit 200 sets the initial z position of the nozzle 49, the air bubble forming unit 200 proceeds the processing to S250.

Next, in S250, the air bubble forming unit 200 sends an instruction to the nozzle actuator 40 to operate the nozzle 49 to the preset set xyz position (that is, the initial position). The nozzle 49 may move on a xy plane. In this case, the control of the z position is performed on the basis of the value of the load sensed by the nozzle actuator 40. Furthermore, the movement of the nozzle 49 may include moving in the z direction as necessary in addition to the movement on the xy plane. After the nozzle actuator 40 moves the nozzle 49 to the set xyz position, the air bubble forming unit 200 proceeds the processing to S252.

Next, in S252, the flow channel imaging camera 42 captures an image of the end part 254 of the nozzle 49. The flow channel imaging camera 42 sends the captured image to the image processing unit 300. The image processing unit 300 may record the image in the recording unit 190 and/or output the image to the air bubble forming unit 200.

Next, in S254, on the basis of the captured image of the end part 254 of the nozzle 49, the air bubble forming unit 200 determines whether the position of the nozzle 49 is different from the preset position. For example, the air bubble forming unit 200 calculates a difference in position from the captured image of the end part 254 of the nozzle 49 and the image of the end part 254 of the nozzle 49 at the preset set xyz position (that is, the initial position), and when the difference is equal to or larger than a threshold, the air bubble forming unit 200 determines that the position of the nozzle 49 is different from the initial position.

When it is determined that the position of the nozzle 49 is different from the initial position, the air bubble forming unit 200 proceeds the processing to S256, and if not, proceeds the processing to S300.

In S256, the air bubble forming unit 200 decides the operation amount of the nozzle actuator 40. For example, the air bubble forming unit 200 decides the operation amount of the nozzle actuator 40 in order to operate the nozzle 49 to the preset set xyz position (that is, the initial position), and sends an instruction to the nozzle actuator 40 to operate by the decided operation amount. For example, the air bubble forming unit 200 may decide the operation amount of an amount according to the level of the difference calculated in S254. The nozzle actuator 40 receives the instruction, and the air bubble forming unit 200 proceeds the processing to S250. In S250 of the second and subsequent times, the air bubble forming unit 200 sends an instruction to the nozzle actuator 40 to perform an operation by the amount according to the operation amount. For example, the air bubble forming unit 200 may send an instruction to the nozzle actuator 40 to move the relative position between the nozzle 49 and the manipulation target 35 close to each other. At this time, the nozzle actuator 40 can move the relative position between the nozzle 49 (flow channel 51) and the manipulation target 35 closer to each other.

Fig. 9C is an example of a flow in which the relative position between the nozzle 49 and the manipulation target 35 is moved on the basis of the inner pressure of the air bubble formed at the end part 254 of the nozzle 49.

First, in S260, the air bubble forming unit 200 controls the pressure generating unit 47 to form the air bubble at the end part 254 of the nozzle 49. Prior to forming the air bubble, the air bubble forming unit 200 may send an instruction to the nozzle actuator 40 to operate the end part 254 of the nozzle 49 into the liquid. The step and sub-step of forming the air bubble in S260 may be the same as the step and sub-step of S300 described below.

Next, in S262, the air bubble forming unit 200 sends an instruction to the nozzle actuator 40 to operate the nozzle 49 to the preset position. The nozzle actuator 40 may be an actuator which controls the z position. In this case, the z position is controlled on the basis of the value of the inner pressure measured by the sensor unit 48. The nozzle 49 may be positioned above a region where no organism is present on the bottom surface of the vessel 25. After the nozzle actuator 40 moves the nozzle 49 to the preset position, the air bubble forming unit 200 proceeds the processing to S264.

In S262, the edge of the nozzle 49 may detect a liquid level and control the z position on the basis of the value of the inner pressure measured by the sensor unit 48. When the volume control part 200 maintains the inner pressure of the nozzle to be equal to or higher than the atmospheric pressure or equal to or lower than the atmospheric pressure, and the edge of the nozzle 49 reaches the liquid level, the value of the inner pressure measured by the sensor unit 48 changes due to an external force caused by the deformation of the gas-liquid interface due to the contact with the liquid level. Therefore, the volume control part 200 can determine whether the edge of the nozzle 49 has reached the liquid level by measuring the value of the inner pressure of the air bubble. In this manner, even when the position to which the nozzle 49 is moved is not preset, the volume control part 200 can send, to the nozzle actuator 40, an instruction of the z position control with the liquid level as a reference position to move the position of the nozzle 49.

Next, in S264, the sensor unit 48 measures the inner pressure of the formed air bubble, and sends the measured value of the inner pressure of the air bubble to the air bubble forming unit 200. When the air bubble reaches the bottom portion of the vessel 25, the air bubble shape is deformed by interaction with the bottom portion, and the inner pressure of the air bubble rapidly changes. Therefore, the air bubble forming unit 200 can determine whether the air bubble has reached the bottom portion of the vessel 25 by measuring the value of the inner pressure of the air bubble. In addition, as another example of the inner pressure measurement, the sensor unit 48 may measure the inner pressure while the nozzle actuator 40 moves the nozzle 49 downward, the volume control part 200 may control the pressure, or the pressure generating unit 47 may operate. By simultaneously operating in this manner, it is possible to increase the speed of the detection of the bottom portion or the like, or it is possible to use, as a detection index, a change process of the pressure in the process of forming the air bubble. Note that, instead of the sensor unit 48, the nozzle actuator 40 may measure the inner pressure of the air bubble and send the measured value of the inner pressure to the air bubble forming unit 200.

Next, in S266, the air bubble forming unit 200 determines whether the measured value of the inner pressure of the air bubble is within a preset inner pressure range. When the measured value of the inner pressure is out of the preset range of the inner pressure, the air bubble forming unit 200 proceeds the processing to S268, and if not, proceeds the processing to S270. As described above, an absolute value of a difference between the inner pressure set by the air bubble forming unit 200 and the measured inner pressure of the air bubble is calculated, and when the difference is equal to or larger than the threshold, the air bubble forming unit 200 determines that the nozzle 49 has reached the bottom portion of the vessel 25.

In S268, the air bubble forming unit 200 decides the operation amount of the nozzle actuator 40. For example, the air bubble forming unit 200 decides the operation amount of the nozzle actuator 40 in order to operate the nozzle 49 to the preset position, and sends an instruction to the nozzle actuator 40 to operate by the decided operation amount. For example, the air bubble forming unit 200 may decide the operation amount of an amount according to the level of the difference calculated in S266. The nozzle actuator 40 receives the instruction, and the processing proceeds to S262. In S262 of the second and subsequent times, the air bubble forming unit 200 sends an instruction to the nozzle actuator 40 to perform an operation by the amount according to the operation amount.

In S270, the air bubble forming unit 200 controls the pressure generating unit 47 to remove the air bubble from the end part 254 of the nozzle 49. The step and sub-step of removing the air bubble in S270 may be the same as the step and sub-step of S500 described below.

Next, in S272, the air bubble forming unit 200 sets the initial z position of the nozzle 49. The step of S272 may be the same as the step of S245. After the air bubble forming unit 200 sets the initial z position of the nozzle 49, the air bubble forming unit 200 proceeds the processing to S274.

Next, in S274, the air bubble forming unit 200 sends an instruction to the nozzle actuator 40 to operate the nozzle 49 to the preset set xyz position (that is, the initial position). The nozzle 49 may move on a xy plane. In this case, the control of the z position is performed on the basis of the value of the inner pressure measured by the nozzle actuator 40. Furthermore, the movement of the nozzle 49 may include moving in the z direction as necessary in addition to the movement on the xy plane. After the nozzle actuator 40 moves the nozzle 49 to the preset set xyz position, the air bubble forming unit 200 proceeds the processing to S276.

Next, in S276, the flow channel imaging camera 42 captures an image of the end part 254 of the nozzle 49. The flow channel imaging camera 42 sends the captured image to the image processing unit 300. The image processing unit 300 may record the image in the recording unit 190 and/or output the image to the air bubble forming unit 200.

Next, in S280, on the basis of the captured image of the end part 254 of the nozzle 49, the air bubble forming unit 200 determines whether the position of the nozzle 49 is different from the preset position. For example, the air bubble forming unit 200 may calculate a difference in position from the captured image of the end part 254 of the nozzle 49 and the image of the nozzle end part 254 at the preset set xyz position (that is, the initial position), and when the difference is equal to or larger than the threshold, the air bubble forming unit 200 may determine that the position of the nozzle 49 is different from the initial position.

When it is determined that the position of the nozzle 49 is different from the initial position, the air bubble forming unit 200 proceeds the processing to S282, and if not, proceeds the processing to S300.

In S282, the air bubble forming unit 200 decides the operation amount of the nozzle actuator 40. For example, the air bubble forming unit 200 decides the operation amount of the nozzle actuator 40 in order to operate the nozzle 49 to the preset set xyz position (that is, the initial position), and sends an instruction to the nozzle actuator 40 to operate by the decided operation amount. For example, the air bubble forming unit 200 may decide the operation amount of an amount according to the level of the difference calculated in S280. The nozzle actuator 40 receives the instruction, and the air bubble forming unit 200 proceeds the processing to S274. In S274 of the second and subsequent times, the air bubble forming unit 200 sends an instruction to the nozzle actuator 40 to perform an operation by the amount according to the operation amount. For example, the air bubble forming unit 200 may send an instruction to the nozzle actuator 40 to move the relative position between the nozzle 49 and the manipulation target 35 close to each other. At this time, the nozzle actuator 40 can move the relative position between the nozzle 49 (flow channel 51) and the manipulation target 35 closer to each other.

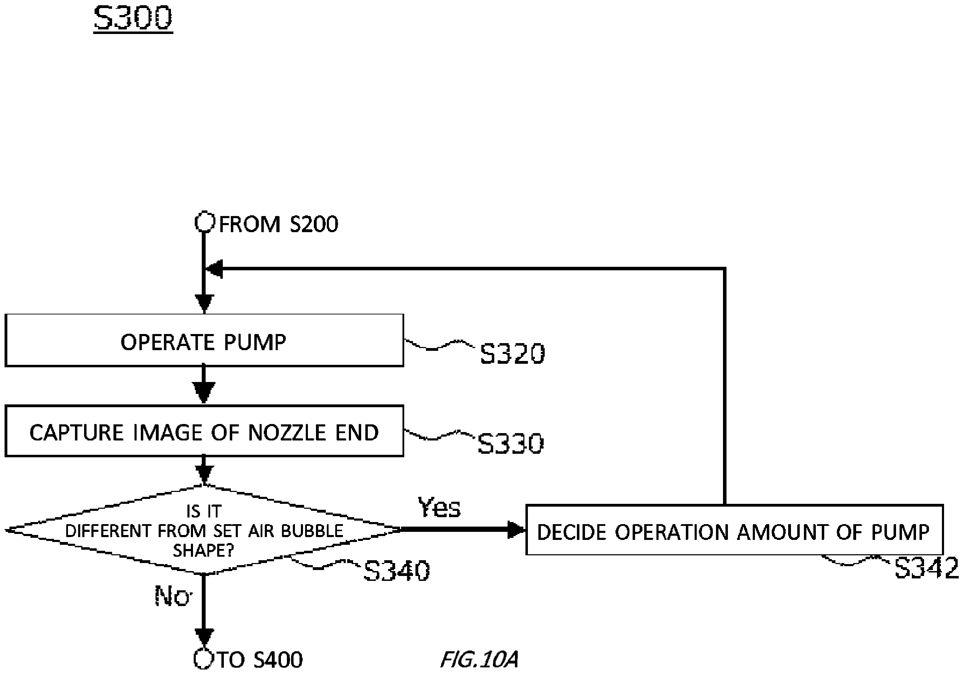

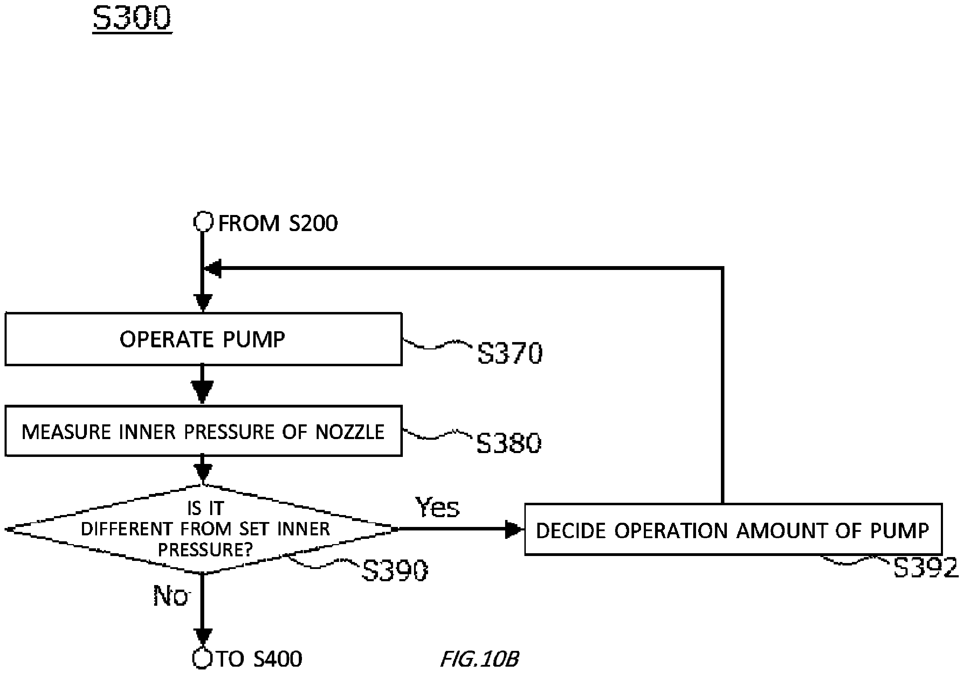

In S300, the air bubble forming unit 200 controls the pressure generating unit 47 to enlarge the gas-liquid interface 255. For example, enlarging the gas-liquid interface 255 may include forming the air bubble. Prior to forming the air bubble, the air bubble forming unit 200 may send an instruction to the nozzle actuator 40 to operate the end part 254 of the nozzle 49 into the liquid. In S300, the step of enlarging the gas-liquid interface 255 includes steps of S320 to S342 as illustrated in Fig. 10A, or includes steps of S370 to S392 as illustrated in Fig. 10B.

Fig. 10 is an example of a flow of enlarging the gas-liquid interface 255 on the basis of the image obtained by capturing the position of the end part 254 of the nozzle 49.

In S320, the air bubble forming unit 200 sends an instruction to the pressure generating unit 47, which is connected to the flow channel 51, to enlarge (form the air bubble) the gas-liquid interface 255 at the edge of the flow channel 51. For example, the air bubble forming unit 200 sends an instruction to the actuator of the pressure generating unit 47 to push the plunger of the syringe pump of the pressure generating unit 47 by a preset distance or to push the plunger of the syringe pump until the pressure reaches a preset pressure. As a result, the gas pushed out from the syringe pump is supplied to the flow channel 51, and the gas is released from the end part 254, whereby the gas-liquid interface 255 at the edge of the flow channel 51 is enlarged (the air bubble is formed). After the gas-liquid interface 255 is enlarged (the air bubble is formed), the air bubble forming unit 200 proceeds the processing to S330.

Next, in S330, the flow channel imaging camera 42 captures an image of the air bubble formed at the end part 254 of the nozzle 49. The flow channel imaging camera 42 sends the captured image to the image processing unit 300. The image processing unit 300 may record the image in the recording unit 190 and/or output the image to the air bubble forming unit 200.

Then, in S340, the air bubble forming unit 200 determines whether the shape of the formed air bubble is different from a preset air bubble shape, based on the image of the air bubble that is captured. The air bubble forming unit 200 predicts the shape of the air bubble formed on the end part 254 of the nozzle 49, from information such as a set inner pressure in the nozzle 49, an inner diameter of the end part 254 of the nozzle 49, wettability of the nozzle 49 (a contact angle of the liquid), the type of the liquid, and the type of the gas. For example, the air bubble forming unit 200 may determine whether the shape of the formed air bubble is different from the set air bubble shape, by comparing the image of the air bubble that is captured, with the shape of the air bubble predicted from the above-described information.

If the shape of the formed air bubble is different from the set air bubble shape, the air bubble forming unit 200 proceeds the processing to S342, and if not, proceeds the processing to S400.

In S342, the air bubble forming unit 200 decides an operation amount of the plunger of the syringe pump of the pressure generating unit 47. For example, the air bubble forming unit 200 decides an operation amount of the plunger of the syringe pump of the pressure generating unit 47 (for example, the distance of pushing or drawing the plunger of the syringe pump) such that the air bubble formed on the edge of the nozzle 49 is formed in a preset shape. The air bubble forming unit 200 sends an instruction to the pressure generating unit 47 to operate by the decided operation amount. For example, the air bubble forming unit 200 may decide an operation amount of an amount according to the level of the difference calculated in S340.

The operation amount may be an operation amount of the actuator of the pressure generating unit 47, or may be an additional pressure loaded on the syringe pump. The pressure generating unit 47 receives the instruction, and the air bubble forming unit 200 proceeds the processing to S320. In S320 of the second and subsequent times, the pressure generating unit 47 performs an operation of an amount according to the operation amount.

FIG. 10B is an example of a flow of enlarging the gas-liquid interface 255, based on an inner pressure in the nozzle 49.

The step of S370 may be the same as the step of S320. Upon completion of S370, the air bubble forming unit 200 proceeds the processing to S380.

Then, in S380, the sensor unit 48 measures an inner pressure in the nozzle 49, and sends a measured value of the inner pressure in the nozzle 49 to the air bubble forming unit 200. Note that, instead of the sensor unit 48, the nozzle actuator 40 may measure an inner pressure in the nozzle 49, and send a measured value of the inner pressure to the air bubble forming unit 200.

Then, in S390, the air bubble forming unit 200 determines whether the measured value of the inner pressure in the nozzle 49 is within a preset range of the inner pressure. If the measured value of the inner pressure is outside the set range of the inner pressure, the air bubble forming unit 200 proceeds the processing to S392, and if not, proceeds the processing to S400. For example, the air bubble forming unit 200 calculates a difference between the preset inner pressure and the measured inner pressure in the nozzle 49, and if the difference is equal to or greater than a threshold, may determine that the set inner pressure has not been achieved.

In S392, the air bubble forming unit 200 decides an operation amount of the plunger of the syringe pump of the pressure generating unit 47 (for example, the distance of pushing or drawing the plunger of the syringe pump) to achieve the set inner pressure in the nozzle 49. The air bubble forming unit 200 sends an instruction to the pressure generating unit 47 to operate by the decided operation amount. For example, the air bubble forming unit 200 may decide an operation amount of an amount according to the level of the difference calculated in S390.

The operation amount may be an operation amount of the actuator of the pressure generating unit 47, or may be an additional pressure loaded on the syringe pump. The pressure generating unit 47 receives the instruction, and the air bubble forming unit 200 proceeds the processing to S370. In S370 of the second and subsequent times, the pressure generating unit 47 performs an operation of an amount according to the operation amount.

In S400, the gas-liquid interface manipulation unit 101 performs a manipulation on the manipulation target 35.

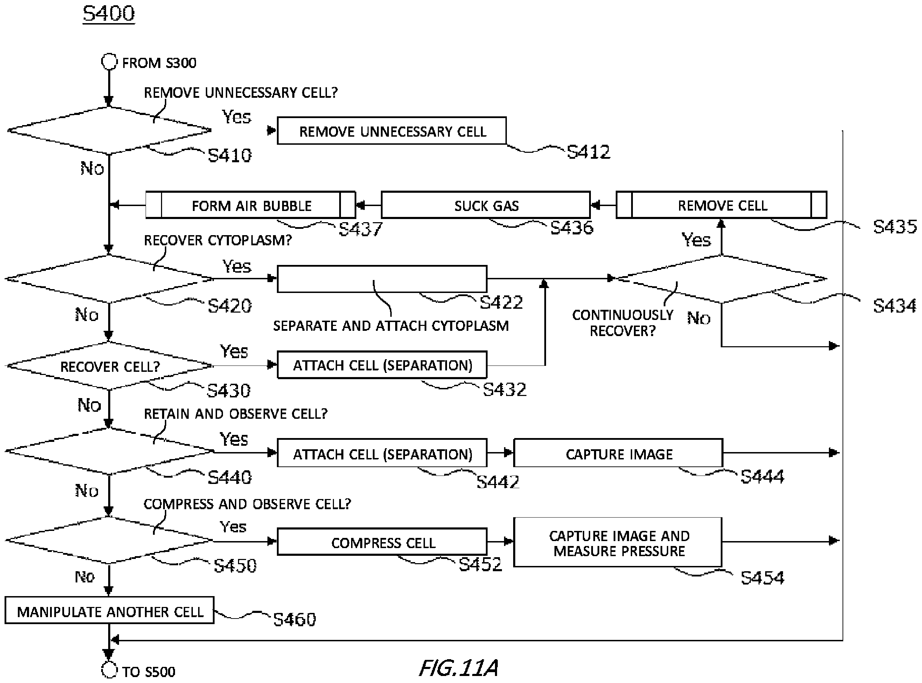

For example, the air bubble forming unit 200 sends an instruction to the gas-liquid interface manipulation unit 101 to perform a manipulation on the manipulation target 35, based on an instruction received via the input unit 180. In S400, the step of performing the manipulation includes the steps from S410 to S460 as illustrated in FIG. 11A. For example, the manipulation may be removal of an unnecessary cell, recovery or movement of a cytoplasm and/or a cell membrane, recovery of a cell, retention of a cell, or compression of a cell.

FIG. 11A is an example of a flow of performing a manipulation on the manipulation target 35. FIG. 11A describes an example when the manipulation target 35 is a cell. Note that, the manipulation target 35 may be other organisms, not limited to cells.

First, in S410, if an instruction to remove an unnecessary cell has been received in S140, the information processing device 170 proceeds the processing to S412. In S410, if an instruction not to remove an unnecessary cell has been received in S140, the information processing device 170 proceeds the processing to S420.

In S412, the air bubble forming unit 200 attaches the cell to the formed air bubble for removal.

For example, the air bubble forming unit 200 sends an instruction to the nozzle actuator 40 to move a position of the nozzle 49, in the x, y, and z directions, to a position where a target cell is present. After the nozzle actuator 40 has moved the nozzle 49 to the target position, the air bubble forming unit 200 may move the nozzle 49 and/or the stage, and bring the gas-liquid interface 255 of the air bubble into contact with the cell.

As an example, the nozzle actuator 40 or the sample actuator 41 identifies the position where the target cell is present, from an image that is obtained by imaging the target cell with the camera 60 or the camera 70, and moves the center of the nozzle 49 to match the target position. After the cell is brought into contact with the gas-liquid interface 255 of the air bubble, as illustrated in 290a to 290c of FIG. 4, the nozzle actuator 40 may recover the target cell in the flow channel 51, and the liquid storage part 54 may discard the cell to the liquid discarding part of the liquid storage part 54.

In addition, as another example, the air bubble forming unit 200 may form the gas-liquid interface 255 in a size that is predetermined in the step of S300 and the sub-step, and may attach the cell to the gas-liquid interface 255 and separate the cell by performing a control to enlarge the gas-liquid interface 255. After the liquid control unit 260 has removed the unnecessary cell, the air bubble forming unit 200 proceeds the processing to S500.

In S420, if an instruction to recover a cytoplasm and/or a cell membrane has been received in S140, the air bubble forming unit 200 proceeds the processing to S422, and if not, proceeds the processing to S430.

In S422, the air bubble forming unit 200 uses the formed air bubble to separate the cytoplasm and/or the cell membrane, and attach these to the air bubble for recovery. For example, the air bubble forming unit 200 controls the pressure generating unit 47 to form an air bubble on the edge of the flow channel 51, and attaches the target cell to the air bubble. Then, the air bubble forming unit 200 compresses the cell with the air bubble, and cuts off only the cytoplasm and/or the cell membrane portion, and attaches it to the air bubble. For example, the air bubble forming unit 200 compresses the cell by raising the inner pressure of the air bubble or enlarging the air bubble by controlling the pressure generating unit 47, or by pressing the air bubble against the cell by controlling the nozzle actuator 40 to move the nozzle 49 toward the cell. Subsequently, the air bubble forming unit 200 moves a part of the cell swollen to the outside of the cell due to the compression, in a manner such that it is cut off from the cell on the gas-liquid interface, thereby cutting off the cytoplasm and/or the cell membrane portion from the cell. In this manner, the air bubble forming unit 200 controls the nozzle actuator 40 or the pressure generating unit 47 to cut out a necessary cytoplasm and/or cell membrane among the manipulation target 35.

For example, the air bubble forming unit 200 may send an instruction to the nozzle actuator 40 to move the nozzle 49 from the initial position, in the x, y, and z directions, to a position where the target cell is present. After the nozzle actuator 40 has moved the nozzle 49 to the target position, the air bubble forming unit 200 may move the nozzle 49 and/or the stage with the nozzle actuator 40 or the sample actuator 41, and may bring the gas-liquid interface 255 of the air bubble into contact with the cell. As an example, the air bubble forming unit 200 identifies the position where the target cell is present from an image that is obtained by imaging the target cell with the camera 60 or the camera 70, and controls the nozzle actuator 40 and moves the nozzle 49 to match the center of the nozzle 49 with the target position. The position where the target cell is present may be identified by the manipulator. In this case, the air bubble forming unit 200 may receive, from the input unit 180, an input by the manipulator as to the position where the target cell is present, and identify the position.

A cell membrane has been known to have a portion showing a relatively soft physical property and a portion showing a relatively hard physical property, due to a difference in the composition of lipids constituting the membrane component. The air bubble forming unit 200 controls the nozzle actuator 40 or the pressure generating unit 47, and compresses the cell with the air bubble. The cell may be compressed with the air bubble by the air bubble forming unit 200 forming the gas-liquid interface 255 of a predetermined size in the step of S300 and the sub-step, and performing a control to enlarge the gas-liquid interface 255, thereby attaching the cell to the gas-liquid interface 255. Then, by using swelling of the portion showing a relatively soft physical property of the cell membrane to the outside, the gas-liquid interface 255 may be used to attach the swollen portion, and move it in a direction away from the cell to cut off the cell membrane, thereby recovering the cell membrane to the flow channel 51 while it is attached to the gas-liquid interface 255. In addition, when cutting out the cell membrane in this manner, since the cell membrane swells by being pushed from the inside by the cytoplasm, the cut-off cell membrane includes a cytoplasmic component on the inside, and thus the cytoplasmic component can also be recovered to the flow channel 51. After the nozzle actuator 40 has cut off the necessary cytoplasm and/or cell membrane portion, the air bubble forming unit 200 proceeds the processing to S434.

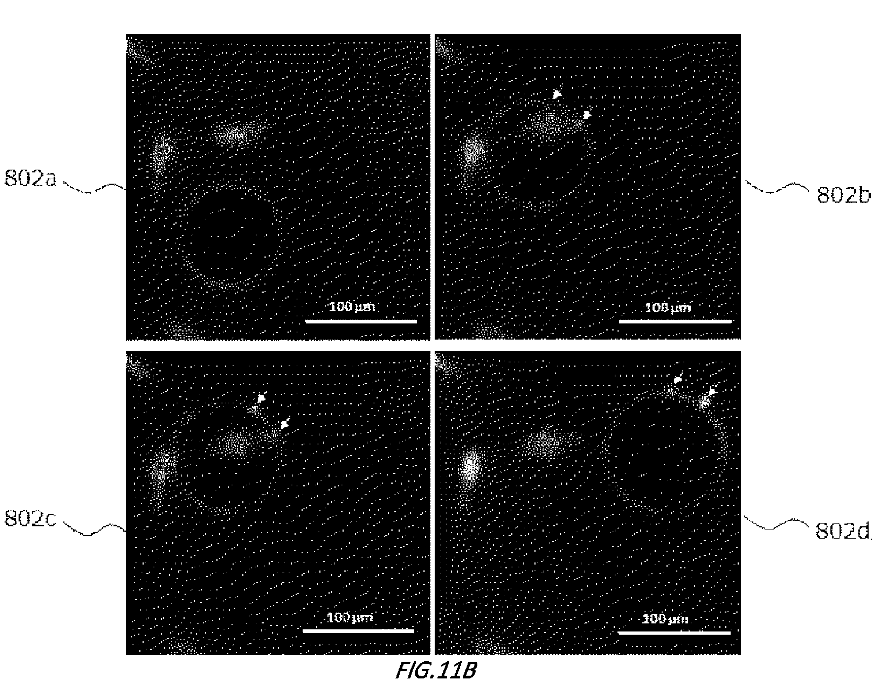

FIG. 11B illustrates a behavior of recovering a cytoplasm and a cell membrane from a cell, according to the present embodiment. To bring a HeLa cell (human cervical cancer cell) cultured on a solid phase of the vessel 25 into contact with the gas-liquid interface 255 of an air bubble, with the air bubble forming unit 200 controlling the pressure generating unit 47 and the nozzle actuator 40, a relative position between the cell and the nozzle 49 was brought close (802a). Then, with the air bubble forming unit 200 performing a control to press the gas-liquid interface 255 against the cell, the cell was compressed (802b). At this time, due to the compression, a behavior that a soft portion of the cell membrane swells to the outside was observed (the arrow of 802b). Then, regarding this swollen portion, a cytoplasm and a cell membrane in the swollen portion were cut off by moving the gas-liquid interface 255 in a direction away from the cell (the arrow of 802c). Finally, the cut-off cytoplasm and cell membrane were recovered by being attached to the gas-liquid interface 255 (802d). Note that, when performing the operation of FIG. 11B, the operation may be performed by enlarging the gas-liquid interface 255, or may be performed by moving the nozzle 49, or may be performed by moving the stage.

Then, in S434, the air bubble forming unit 200 determines whether the instruction received in S140 includes continuously recovering the cytoplasm and/or the cell membrane of the manipulation target 35. The air bubble forming unit 200 proceeds the processing to S435 if the determination is positive, and to S500 if the determination is negative.

In S435, the air bubble forming unit 200 controls the pressure generating unit 47 to remove the air bubble formed on the end part 254 of the nozzle 49. When removing the air bubble, recovery of the manipulation target 35 may be performed at the same time. For example, the manipulation target 35 may be recovered into liquid existing in the flow channel 51. The step of removing the air bubble in S435 and the sub-step may be the same as the step of S500 and the sub-step, and the details thereof will be described below.

Then, in S436, the air bubble forming unit 200 controls the pressure generating unit 47 and the nozzle actuator 40 to perform intake of gas for forming a new air bubble on the end part 254 of the nozzle 49. The air bubble forming unit 200 sends an instruction to the nozzle actuator 40 to take out the nozzle 49 from the liquid. After the nozzle actuator 40 has taken out the nozzle 49 from the liquid, the pressure generating unit 47 may draw the plunger of the syringe pump to take in a necessary amount of the gas.

Then, in S437, the air bubble forming unit 200 controls the pressure generating unit 47 to form a new air bubble on the end part 254 of the nozzle 49. For the step of S437 and the sub-step, the content of S300 that is already described may be applied. After the pressure generating unit 47 has formed an air bubble on the end part 254 of the nozzle 49, the air bubble forming unit 200 proceeds the processing to the step of S420.

In S430, the air bubble forming unit 200 determines whether the instruction to recover a cell has been received in S140. The air bubble forming unit 200 proceeds the processing to S432 if the determination is positive, and the air bubble forming unit 200 proceeds the processing to S440 if the determination is negative.

In S432, the air bubble forming unit 200 controls the pressure generating unit 47 to form an air bubble, and attaches the cell to the air bubble. The air bubble forming unit 200 may, as necessary, separate the cell attached to the air bubble from the solid phase.

For example, the air bubble forming unit 200 sends an instruction to the nozzle actuator 40 to move the nozzle 49 from the initial position, in the x, y, and z directions, to a position where a target cell is present. After the nozzle actuator 40 has moved the nozzle 49 to the target position, the air bubble forming unit 200 controls the pressure generating unit 47 to supply gas to the flow channel 51, thereby forming an air bubble on the edge of the flow channel 51. Then, the air bubble forming unit 200 may control the nozzle actuator 40 or the sample actuator 41 to move the nozzle 49 or the stage, thereby bringing the gas-liquid interface 255 of the air bubble into contact with the cell.

As an example, the nozzle actuator 40 or the sample actuator 41 identifies the position where the target cell is present, from an image that is obtained by imaging the target cell with the camera 60 or the camera 70, and moves the center of the nozzle 49 to match the target position. After the nozzle actuator 40 or the sample actuator 41 has moved the nozzle 49 to the target position, the air bubble forming unit 200 controls the pressure generating unit 47 to supply gas to the flow channel 51, thereby forming an air bubble on the edge of the flow channel 51. Then, the air bubble forming unit 200 may move the nozzle 49 and/or the stage with the nozzle actuator 40 or the sample actuator 41, and bring the gas-liquid interface 255 of the air bubble into contact with the cell. For example, after bringing the cell into contact with the gas-liquid interface 255 of the air bubble, the nozzle actuator 40 may separate the cell, as necessary, by a means such as moving the gas-liquid interface 255.

After the air bubble forming unit 200 has controlled the pressure generating unit 47 to form an air bubble, and attached a cell to the air bubble, the air bubble forming unit 200 proceeds the processing to S434. For the steps of S434 and onwards, the content that is already described may be applied. Note that, in this case, in the step of S434, an object to be continuously recovered is not limited to only a cytoplasm or only a cell, but it may be both the cytoplasm and the cell.

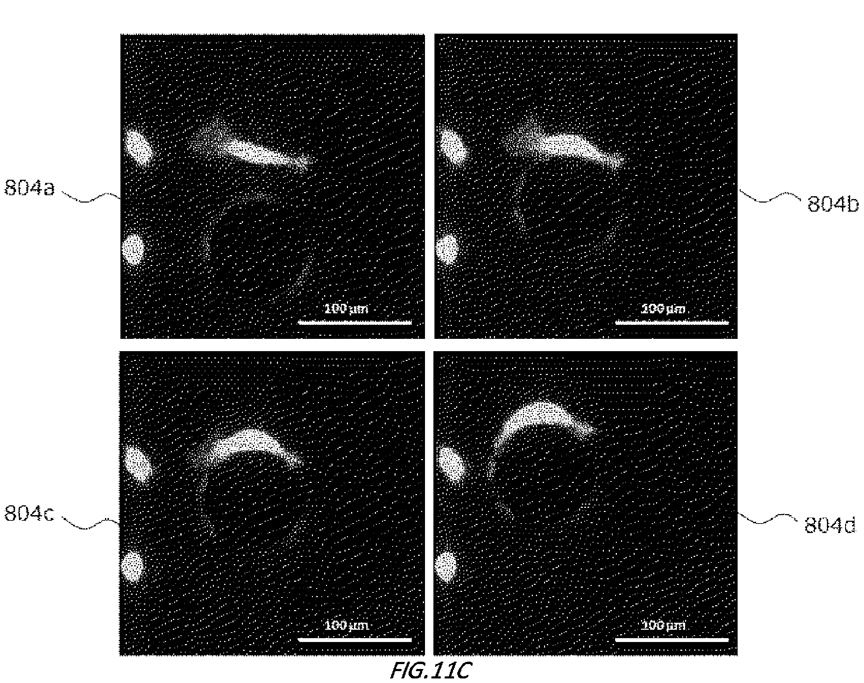

FIG. 11C illustrates a behavior of attaching an established cell line to an air bubble and separating the established cell line, according to the present embodiment. To bring a HeLa cell cultured on a solid phase of the vessel 25 into contact with the gas-liquid interface 255 of an air bubble, with the air bubble forming unit 200 controlling the pressure generating unit 47 and the nozzle actuator 40 (or the sample actuator 41 instead of the nozzle actuator 40), a relative position between the cell and the nozzle 49 was brought close (804a). Then, the cell contacted the gas-liquid interface 255 of the air bubble (804b). Then, the nozzle actuator 40 moved the gas-liquid interface 255 (804c), and the cell was attached to and separated from the gas-liquid interface 255 (804d). Note that, when performing the operation of FIG. 11C, the operation may be performed by enlarging the gas-liquid interface 255, or may be performed by moving the nozzle 49, or may be performed by moving the stage.

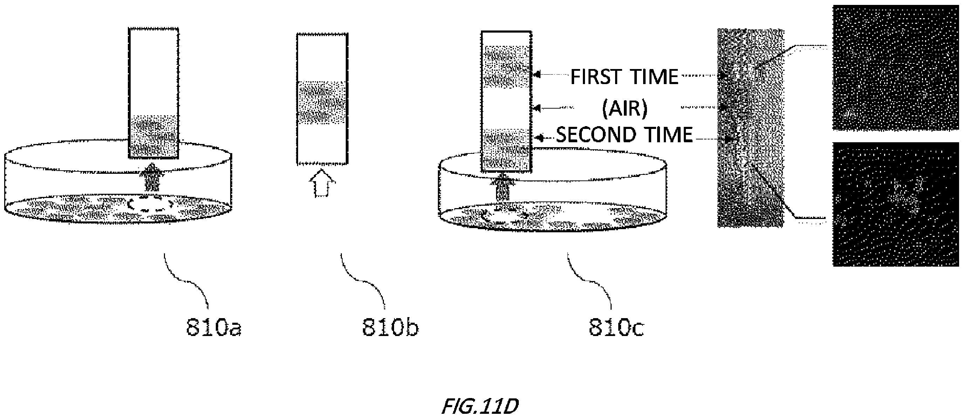

FIG. 11D illustrates a schematic view when repeating S430 → S432 → S434 → S435 → S436 → S437. When continuously recovering a cytoplasm and/or a cell membrane, and when continuously recovering a cell, these may be performed as shown in the schematic view of FIG. 11D. In 810a, after the nozzle actuator 40 has put the nozzle 49 in liquid, the air bubble forming unit 200 controls the pressure generating unit 47, and forms an air bubble on the end part 254 of the nozzle 49 by supplying gas to the syringe pump (not illustrated). Then, a cell adhered on a bottom surface of the vessel 25 is attached to the gas-liquid interface 255 of the air bubble and separated, and is recovered to the flow channel 51 by taking in the gas with the syringe pump. Then, in 810b, the nozzle actuator 40 pulls the nozzle 49 up from the liquid, and the syringe pump suctions the gas (for example, the air) to the flow channel 51. Then, in 810c, after the nozzle actuator 40 has put the nozzle 49 in the liquid, the syringe pump supplies gas to the end part 254 of the nozzle 49 to form a new air bubble, and a cell adhered on a bottom surface of the vessel 25 is recovered by being attached to the gas-liquid interface 255 of the air bubble. In this manner, with the air bubble forming unit 200 controlling the pressure generating unit 47 and the nozzle actuator 40, two cells (a population) can be continuously recovered in the flow channel 51, via the gas, without being mixed. A photograph showing two cells (a population) that are actually recovered with the air placed therebetween, by using this method, is also presented. Note that, FIG. 11D described the example of moving the nozzle 49, but instead of moving the nozzle 49, the operation of FIG. 11D may be performed by moving the stage.

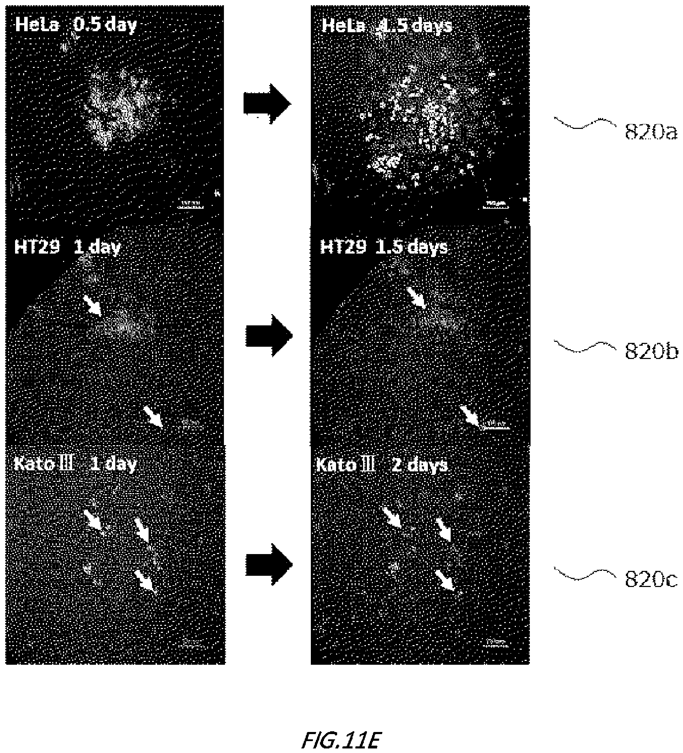

FIG. 11E illustrates a behavior of subculture in which an established cell line is recovered, and the recovered established cell line is cultured, according to the present embodiment. With the air bubble forming unit 200 controlling the pressure generating unit 47 and the nozzle actuator 40, a HeLa cell (human cervical cancer cell, 820a), a HT29 cell (human large bowel cancer cell, 820b), and a Kato III cell (human gastric signet ring cell cancer cell, 820c) that are established cell lines were attached to, and separated and recovered from a solid phase of the vessel 25 by using the gas-liquid interface 255 of an air bubble, and were released to a medium in another vessel to be cultured for 1.5 day (820a and 820b) and for 2 days (820c). For each cell, a cell proliferation was confirmed after the subculture. That is, according to the present embodiment, even when an established cell line was separated by using the gas-liquid interface 255 of an air bubble, the cell could be proliferated without damaging the viability of the cell. Note that, when performing the operation of FIG. 11E, the operation may be performed by moving the nozzle 49, or may be performed by moving the stage.

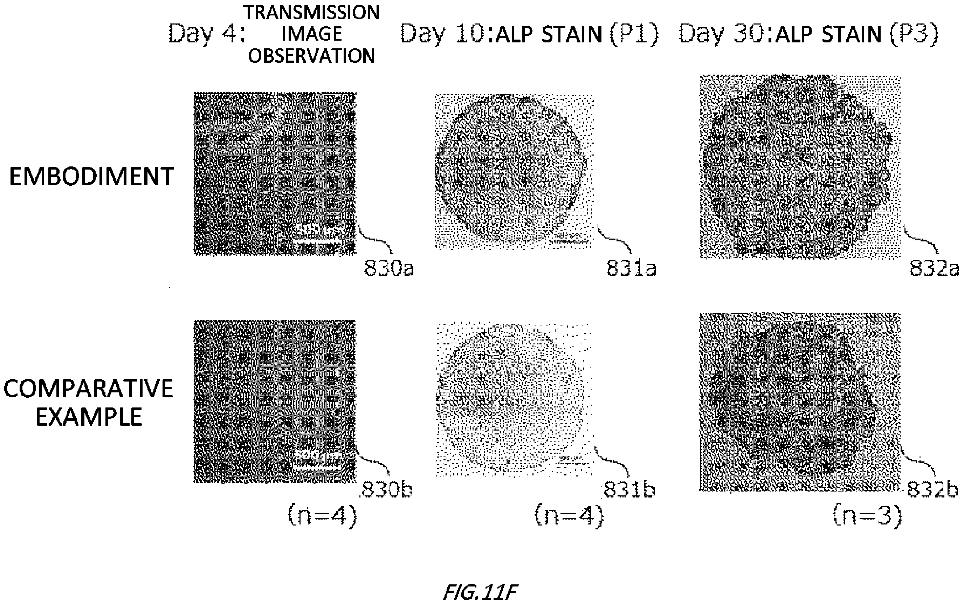

FIG. 11F illustrates a behavior of recovering an iPS cell, and subculturing the recovered iPS cell, according to the present embodiment. With the air bubble forming unit 200 controlling the pressure generating unit 47 and the nozzle actuator 40, a colony of the cultured iPS cells was attached to, and separated and recovered from a solid phase of the vessel 25 by using the gas-liquid interface 255 of an air bubble, and was released in a liquid culture medium in another vessel for observation of a transmission image after culturing for 4 days. In addition, as a comparative example, cells obtained by subculturing an iPS cell with an ordinary cell subculturing method (mechanical passage) was used. As a result, no morphological difference was recognized between the iPS cells of the present embodiment (830a) and the iPS cells of the comparative example (830b). In addition, after culturing for 10 days, staining of alkaline phosphatase was performed on the iPS cells of the present embodiment and the comparative example. Furthermore, after subculturing the iPS cell of the present embodiment and the comparative example for three times, and culturing the iPS cell for 30 days, staining of alkaline phosphatase was performed on the iPS cells of the present embodiment and the comparative example. As a result, no difference in stainability was recognized between the iPS cells of the present embodiment (831a is those cultured for 10 days, and 832a is those cultured for 30 days) and the iPS cells of the comparative example (831b is those cultured for 10 days, and 832b is those cultured for 30 days). It has been known that alkaline phosphatase is highly expressed in an iPS cell maintaining a self-replication ability that is undifferentiated. That is, according to the present embodiment, even when an iPS cell was separated and recovered by using the gas-liquid interface 255, the iPS cell could be proliferated while maintaining an undifferentiated state, without affecting maintainability of undifferentiation ability. Note that, when performing the operation of FIG. 11F, the operation may be performed by moving the nozzle 49, or may be performed by moving the stage.

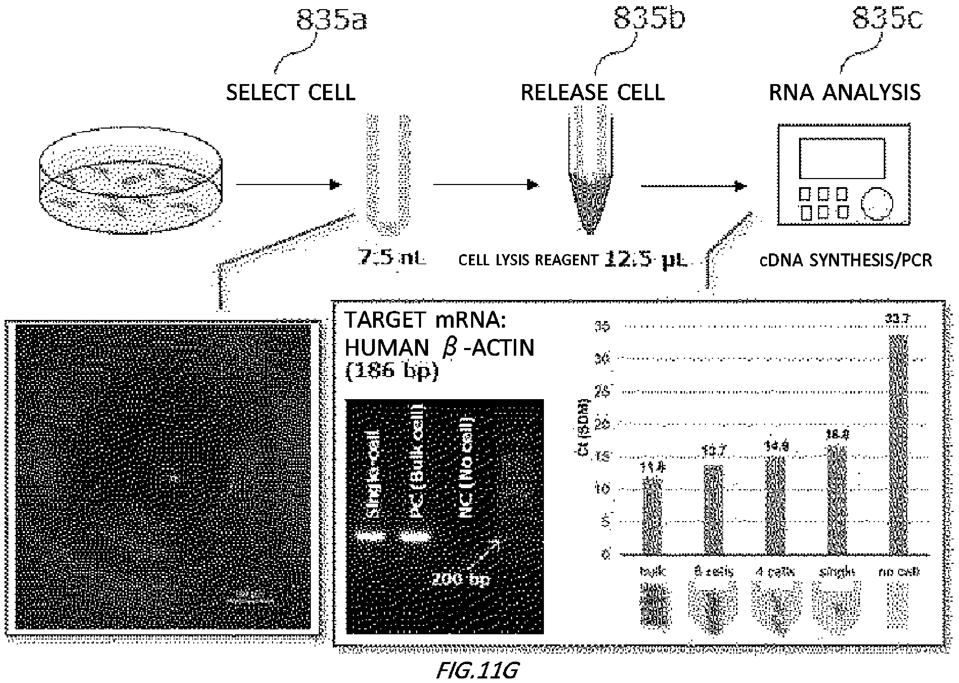

FIG. 11G illustrates a behavior of recovering an established cell line, and analyzing the recovered cell, according to the present embodiment. A HeLa cell that is an established cell line was separated and recovered from a solid phase of the vessel 25 by using the gas-liquid interface 255 of an air bubble. With the air bubble forming unit 200 controlling the pressure generating unit 47 and the nozzle actuator 40, one cell, four cells, and eight cells of the HeLa cells were selected and separated from the solid phase by using the gas-liquid interface 255 of the air bubble, and these cells were each recovered together with a liquid culture medium of 7.5 nL (835a). Then, the recovered HeLa cells were released to a 12.5 μL cytolytic reagent, and the cells were lysed (835b). In the cell lysate in which the HeLa cells are lysed, from mRNA of β-actin, a cDNA was synthesized, and a PCR reaction was performed (835c). As a result, an amount of cDNAs generally proportional to the number of recovered cells could be detected. That is, according to the present embodiment, cells could be recovered by one cell or an optional number of cells by using the gas-liquid interface 255, and a molecular biological analysis could be performed. Note that, when performing the operation of FIG. 11G, the operation may be performed by moving the nozzle 49, or may be performed by moving the stage.

Then, in S440, the air bubble forming unit 200 determines whether the instruction to retain and image the cell has been received in S140. The air bubble forming unit 200 proceeds the processing to S442 if the determination is positive, and to S450 if the determination is negative.

In S442, the air bubble forming unit 200 performs a control to attach the cell to the formed air bubble, and retain the attached cell. For example, the air bubble forming unit 200 sends an instruction to the nozzle actuator 40 to move the nozzle 49 from the initial position, in the x, y, and z directions, to a position where a target cell is present. After the nozzle actuator 40 has moved the nozzle 49 to the target position, the air bubble forming unit 200 controls the pressure generating unit 47 to supply gas to the flow channel 51, thereby forming an air bubble on the edge of the flow channel 51. The air bubble forming unit 200 may move the nozzle 49 and/or the stage with the nozzle actuator 40 or the sample actuator 41, and may bring the gas-liquid interface 255 of the air bubble into contact with the cell. As an example, the nozzle actuator 40 or the sample actuator 41 identifies the position where the target cell is present, from an image that is obtained by imaging the target cell with the camera 60 or the camera 70, and moves the center of the nozzle 49 to match the target position. After the cell has been brought into contact with the gas-liquid interface 255 of the air bubble, the air bubble forming unit 200 proceeds the processing to S444.

The position where the target cell is present may be identified by the manipulator. In this case, the air bubble forming unit 200 may receive, from the input unit 180, an input by the manipulator as to the position where the target cell is present, and identify the position. In addition, an edge surface of the air bubble is brought into contact with the cell in the above-described example, but the contact between the air bubble and the cell may be performed by bringing a side surface of the air bubble into contact with the cell. In this case, the nozzle actuator 40 or the sample actuator 41 may move the center of the nozzle 49 to match a vicinity of the target cell. In addition, for example, after bringing the cell into contact with the gas-liquid interface 255 of the air bubble, the nozzle actuator 40 may separate the cell as necessary, by a means such as moving the gas-liquid interface 255.

Then, in S444, the imaging control unit 171 sends an instruction to the camera 60 or the camera 70 to image the cell retained in the air bubble. The camera 60 or the camera 70 captures an image, and sends the image to the image processing unit 300. The image processing unit 300 may record the image in the recording unit 190 and/or output the image to the air bubble forming unit 200.

After the camera 60 or the camera 70 has imaged the retained cell, the air bubble forming unit 200 proceeds the processing to S500.

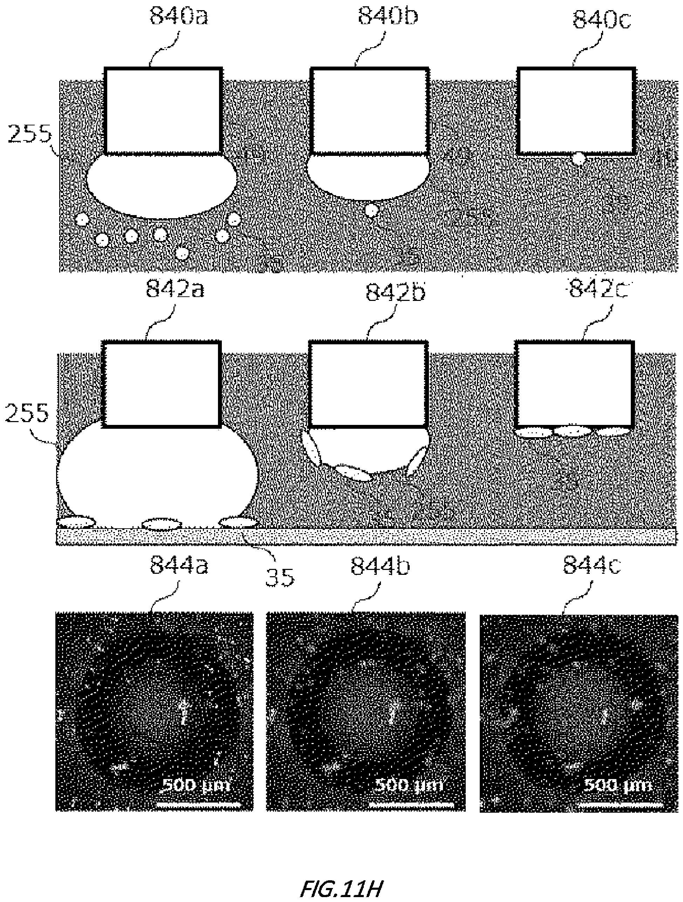

FIG. 11H is a schematic view showing a behavior of retaining an established cell line in the gas-liquid interface 255 of an air bubble, and performing an observation. Since a floating cell or a cell that is weakly adhered freely moves in a culture solution due to a slight vibration or the like, it is difficult to observe such cell as it is by using a microscope or the like. In 840a, the air bubble forming unit 200 controls the nozzle actuator 40 to put the end part 254 of the nozzle 49 in a liquid culture medium in the vessel 25 where a floating cell (the manipulation target 35) is cultured. Then, the pressure generating unit 47 supplies gas to the flow channel 51 to form an air bubble on the edge of the nozzle 49, thereby forming the gas-liquid interface 255. The air bubble forming unit 200 attaches a floating cell to become the manipulation target 35 to the gas-liquid interface 255, which is formed by controlling the nozzle actuator 40 or the pressure generating unit 47. Then, in 840b, the pressure generating unit 47 controls an inner pressure of the air bubble to temporarily retain the cell on the gas-liquid interface 255 of the air bubble. Then, in 840c, the pressure generating unit 47 takes in the gas from the flow channel 51 to shrink the air bubble. The cell retained in that state can be observed by using a microscope or the like. Alternatively, the pressure generating unit 47 may retain the cell without shrinking the air bubble, and the retained cell may be observed by using a microscope or the like. In this manner, according to the present embodiment, by retaining a cell on the gas-liquid interface 255 of an air bubble, an observation can be performed without moving the cell.

In addition, in the case of adherent cells that are dispersed on a solid phase of the vessel 25, when performing an observation with a microscope or the like, it is necessary to move the stage and observe with a wide field. In 842a, the air bubble forming unit 200 controls the nozzle actuator 40 to put the end part 254 of the nozzle 49 in a liquid culture medium of the vessel 25 where an adherent cell (the manipulation target 35) is cultured. Then, the pressure generating unit 47 supplies gas to the flow channel 51 to form an air bubble on the edge of the nozzle 49, thereby forming the gas-liquid interface 255. Then, the air bubble forming unit 200 controls the nozzle actuator 40 or the pressure generating unit 47 to control the gas-liquid interface 255, thereby attaching the cells to the gas-liquid interface 255 and separating the cells. Then, in 842b, the pressure generating unit 47 temporarily retains the cells on the gas-liquid interface 255 of the air bubble. Then, in 842c, the pressure generating unit 47 takes in the gas from the flow channel 51 to shrink the air bubble. The cells retained in such state are present in a narrow range on a plane surface of the same z position, and thus the cells can be observed all at once by using a microscope or the like. In this manner, according to the present embodiment, a field to be observed can be reduced by retaining cells on a gas-liquid interface of an air bubble.

As an example of such observation technique, Kato III cells that are floating cells were dispersed on a solid phase, and some of the cells were attached to the gas-liquid interface 255 (844a). Subsequently, the air bubble forming unit 200 controls an inner pressure of the air bubble via the pressure generating unit 47 to retain the cells on the gas-liquid interface 255 while shrinking the air bubble, and when the retained cells were focused, surrounding cells became out of focus (844b). At this time, when moving the stage, the surrounding cells will move but the cells retained on the gas-liquid interface 255 will not move, and thus the retained cells can be easily observed with a microscope (844c).

In S450, the air bubble forming unit 200 determines whether the instruction to compress and image the cell has been received in S140. The air bubble forming unit 200 proceeds the processing to S452 if the determination is positive, and to S460 if the determination is negative.

In S452, the air bubble forming unit 200 controls the pressure generating unit 47 and the nozzle actuator 40 to compress the cell by using the air bubble. For example, the air bubble forming unit 200 controls the pressure generating unit 47 to form an air bubble on the edge of the flow channel 51, and brings the air bubble into contact with the cell to become the manipulation target 35. Note that, when performing the operation of FIG. 11H, the operation may be performed by moving the nozzle 49, or may be performed by moving the stage.

As an example, the air bubble forming unit 200 sends an instruction to the nozzle actuator 40 to move the nozzle 49 from the initial position, in the x, y, and z directions, to a position where the target cell is present. As an example, the air bubble forming unit 200 identifies the position where the target cell is present from an image that is obtained by imaging the target cell with the camera 60 or the camera 70, and controls the nozzle actuator 40 and moves the nozzle 49 to match the center of the nozzle 49 with the target position.

The position where the target cell is present may be identified by the manipulator. In this case, the air bubble forming unit 200 may receive, from the input unit 180, an input by the manipulator as to the position where the target cell is present, and identify the position. In addition, the contact between the air bubble and the cell may be performed by bringing an edge surface of the air bubble into contact with the cell, or may be performed by bringing a side surface of the air bubble into contact with the cell. When bringing the edge surface of the air bubble into contact with the cell, the nozzle actuator 40 or the sample actuator 41 may move the center of the nozzle 49 to match immediately above the target cell. When bringing the side surface of the air bubble into contact with the cell, the nozzle actuator 40 or the sample actuator 41 may move the center of the nozzle 49 to match a vicinity of the target cell, and in this case, an air bubble can be formed on the side of the cell, and the cell can be gradually compressed from the side by moving the nozzle 49.

Then, after the nozzle actuator 40 has moved the nozzle 49 to the target position, the air bubble forming unit 200 controls the pressure generating unit 47 to supply gas to the flow channel 51, thereby forming an air bubble on the edge of the flow channel 51 in the target position. The air bubble forming unit 200 may move the nozzle 49 and/or the stage with the nozzle actuator 40 or the sample actuator 41, and may bring the gas-liquid interface 255 of the air bubble into contact with the cell. When the position of the nozzle 49 is fixed, the gas-liquid interface 255 and the cell may be brought into contact by moving the stage. The cell may be brought into contact with the gas-liquid interface 255 with a control by the air bubble forming unit 200 to enlarge the gas-liquid interface 255 via the pressure generating unit 47.

As an example, the air bubble forming unit 200 operates the plunger of the syringe pump of the pressure generating unit 47 with a preset operation amount to form an air bubble of a preset volume, and then uses the nozzle actuator 40 or the sample actuator 41 to move the nozzle 49 and/or the stage such that the nozzle 49 is positioned at a position where the air bubble compresses the cell. Then, the air bubble forming unit 200 may compress the cell by enlarging the air bubble formed on the edge of the flow channel 51 by controlling the pressure generating unit 47, or by pressing the air bubble against the cell by controlling the nozzle actuator 40 to move the nozzle 49 toward the cell.

In addition, as an example, the air bubble forming unit 200 may compress the cell by, after moving the nozzle 49 very near the cell, controlling the pressure generating unit 47 to form an air bubble of a preset volume on the edge of the flow channel 51.

Then, in S454, the imaging control unit 171 sends an instruction to the camera 60 or the camera 70 to image the compressed cell. The camera 60 or the camera 70 captures an image, and sends the image to the image processing unit 300. The image processing unit 300 may record the image in the recording unit 190 and/or output the image to the output unit 160.

Furthermore, instead of/in addition to this, the sensor unit 48 or the nozzle actuator 40 may measure a pressure of compressing the cell with the air bubble, and send a measured value of the pressure to the air bubble forming unit 200. The camera 60 or the camera 70 may repeat capturing of an image while changing a pressure of compressing the cell with the air bubble forming unit 200. The pressure of compressing the cell can be changed by the air bubble forming unit 200 controlling the pressure generating unit 47 and changing an inner pressure and/or a volume of the air bubble.

Then, in S340, the air bubble forming unit 200 determines whether the shape of the formed air bubble is different from a preset air bubble shape, based on the image of the air bubble that is captured. The air bubble forming unit 200 predicts the shape of the air bubble formed on the end part 254 of the nozzle 49, from information such as a set inner pressure in the nozzle 49, an inner diameter of the end part 254 of the nozzle 49, wettability of the nozzle 49 (a contact angle of the liquid), the type of the liquid, and the type of the gas. For example, the air bubble forming unit 200 may determine whether the shape of the formed air bubble is different from the set air bubble shape, by comparing the image of the air bubble that is captured, with the shape of the air bubble predicted from the above-described information.

If the shape of the formed air bubble is different from the set air bubble shape, the air bubble forming unit 200 proceeds the processing to S342, and if not, proceeds the processing to S400.

In S342, the air bubble forming unit 200 decides an operation amount of the plunger of the syringe pump of the pressure generating unit 47. For example, the air bubble forming unit 200 decides an operation amount of the plunger of the syringe pump of the pressure generating unit 47 (for example, the distance of pushing or drawing the plunger of the syringe pump) such that the air bubble formed on the edge of the nozzle 49 is formed in a preset shape. The air bubble forming unit 200 sends an instruction to the pressure generating unit 47 to operate by the decided operation amount. For example, the air bubble forming unit 200 may decide an operation amount of an amount according to the level of the difference calculated in S340.

The operation amount may be an operation amount of the actuator of the pressure generating unit 47, or may be an additional pressure loaded on the syringe pump. The pressure generating unit 47 receives the instruction, and the air bubble forming unit 200 proceeds the processing to S320. In S320 of the second and subsequent times, the pressure generating unit 47 performs an operation of an amount according to the operation amount.

FIG. 10B is an example of a flow of enlarging the gas-liquid interface 255, based on an inner pressure in the nozzle 49.

The step of S370 may be the same as the step of S320. Upon completion of S370, the air bubble forming unit 200 proceeds the processing to S380.

Then, in S380, the sensor unit 48 measures an inner pressure in the nozzle 49, and sends a measured value of the inner pressure in the nozzle 49 to the air bubble forming unit 200. Note that, instead of the sensor unit 48, the nozzle actuator 40 may measure an inner pressure in the nozzle 49, and send a measured value of the inner pressure to the air bubble forming unit 200.

Then, in S390, the air bubble forming unit 200 determines whether the measured value of the inner pressure in the nozzle 49 is within a preset range of the inner pressure. If the measured value of the inner pressure is outside the set range of the inner pressure, the air bubble forming unit 200 proceeds the processing to S392, and if not, proceeds the processing to S400. For example, the air bubble forming unit 200 calculates a difference between the preset inner pressure and the measured inner pressure in the nozzle 49, and if the difference is equal to or greater than a threshold, may determine that the set inner pressure has not been achieved.

In S392, the air bubble forming unit 200 decides an operation amount of the plunger of the syringe pump of the pressure generating unit 47 (for example, the distance of pushing or drawing the plunger of the syringe pump) to achieve the set inner pressure in the nozzle 49. The air bubble forming unit 200 sends an instruction to the pressure generating unit 47 to operate by the decided operation amount. For example, the air bubble forming unit 200 may decide an operation amount of an amount according to the level of the difference calculated in S390.

The operation amount may be an operation amount of the actuator of the pressure generating unit 47, or may be an additional pressure loaded on the syringe pump. The pressure generating unit 47 receives the instruction, and the air bubble forming unit 200 proceeds the processing to S370. In S370 of the second and subsequent times, the pressure generating unit 47 performs an operation of an amount according to the operation amount.

In S400, the gas-liquid interface manipulation unit 101 performs a manipulation on the manipulation target 35. For example, the air bubble forming unit 200 sends an instruction to the gas-liquid interface manipulation unit 101 to perform a manipulation on the manipulation target 35, based on an instruction received via the input unit 180. In S400, the step of performing the manipulation includes the steps from S410 to S460 as illustrated in FIG. 11A. For example, the manipulation may be removal of an unnecessary cell, recovery or movement of a cytoplasm and/or a cell membrane, recovery of a cell, retention of a cell, or compression of a cell.

FIG. 11A is an example of a flow of performing a manipulation on the manipulation target 35. FIG. 11A describes an example when the manipulation target 35 is a cell. Note that, the manipulation target 35 may be other organisms, not limited to cells.

First, in S410, if an instruction to remove an unnecessary cell has been received in S140, the information processing device 170 proceeds the processing to S412. In S410, if an instruction not to remove an unnecessary cell has been received in S140, the information processing device 170 proceeds the processing to S420.

In S412, the air bubble forming unit 200 attaches the cell to the formed air bubble for removal.

For example, the air bubble forming unit 200 sends an instruction to the nozzle actuator 40 to move a position of the nozzle 49, in the x, y, and z directions, to a position where a target cell is present. After the nozzle actuator 40 has moved the nozzle 49 to the target position, the air bubble forming unit 200 may move the nozzle 49 and/or the stage, and bring the gas-liquid interface 255 of the air bubble into contact with the cell.

As an example, the nozzle actuator 40 or the sample actuator 41 identifies the position where the target cell is present, from an image that is obtained by imaging the target cell with the camera 60 or the camera 70, and moves the center of the nozzle 49 to match the target position. After the cell is brought into contact with the gas-liquid interface 255 of the air bubble, as illustrated in 290a to 290c of FIG. 4, the nozzle actuator 40 may recover the target cell in the flow channel 51, and the liquid storage part 54 may discard the cell to the liquid discarding part of the liquid storage part 54.

In addition, as another example, the air bubble forming unit 200 may form the gas-liquid interface 255 in a size that is predetermined in the step of S300 and the sub-step, and may attach the cell to the gas-liquid interface 255 and separate the cell by performing a control to enlarge the gas-liquid interface 255. After the liquid control unit 260 has removed the unnecessary cell, the air bubble forming unit 200 proceeds the processing to S500.

In S420, if an instruction to recover a cytoplasm and/or a cell membrane has been received in S140, the air bubble forming unit 200 proceeds the processing to S422, and if not, proceeds the processing to S430.

In S422, the air bubble forming unit 200 uses the formed air bubble to separate the cytoplasm and/or the cell membrane, and attach these to the air bubble for recovery. For example, the air bubble forming unit 200 controls the pressure generating unit 47 to form an air bubble on the edge of the flow channel 51, and attaches the target cell to the air bubble. Then, the air bubble forming unit 200 compresses the cell with the air bubble, and cuts off only the cytoplasm and/or the cell membrane portion, and attaches it to the air bubble. For example, the air bubble forming unit 200 compresses the cell by raising the inner pressure of the air bubble or enlarging the air bubble by controlling the pressure generating unit 47, or by pressing the air bubble against the cell by controlling the nozzle actuator 40 to move the nozzle 49 toward the cell. Subsequently, the air bubble forming unit 200 moves a part of the cell swollen to the outside of the cell due to the compression, in a manner such that it is cut off from the cell on the gas-liquid interface, thereby cutting off the cytoplasm and/or the cell membrane portion from the cell. In this manner, the air bubble forming unit 200 controls the nozzle actuator 40 or the pressure generating unit 47 to cut out a necessary cytoplasm and/or cell membrane among the manipulation target 35.

For example, the air bubble forming unit 200 may send an instruction to the nozzle actuator 40 to move the nozzle 49 from the initial position, in the x, y, and z directions, to a position where the target cell is present. After the nozzle actuator 40 has moved the nozzle 49 to the target position, the air bubble forming unit 200 may move the nozzle 49 and/or the stage with the nozzle actuator 40 or the sample actuator 41, and may bring the gas-liquid interface 255 of the air bubble into contact with the cell. As an example, the air bubble forming unit 200 identifies the position where the target cell is present from an image that is obtained by imaging the target cell with the camera 60 or the camera 70, and controls the nozzle actuator 40 and moves the nozzle 49 to match the center of the nozzle 49 with the target position. The position where the target cell is present may be identified by the manipulator. In this case, the air bubble forming unit 200 may receive, from the input unit 180, an input by the manipulator as to the position where the target cell is present, and identify the position.

A cell membrane has been known to have a portion showing a relatively soft physical property and a portion showing a relatively hard physical property, due to a difference in the composition of lipids constituting the membrane component. The air bubble forming unit 200 controls the nozzle actuator 40 or the pressure generating unit 47, and compresses the cell with the air bubble. The cell may be compressed with the air bubble by the air bubble forming unit 200 forming the gas-liquid interface 255 of a predetermined size in the step of S300 and the sub-step, and performing a control to enlarge the gas-liquid interface 255, thereby attaching the cell to the gas-liquid interface 255. Then, by using swelling of the portion showing a relatively soft physical property of the cell membrane to the outside, the gas-liquid interface 255 may be used to attach the swollen portion, and move it in a direction away from the cell to cut off the cell membrane, thereby recovering the cell membrane to the flow channel 51 while it is attached to the gas-liquid interface 255. In addition, when cutting out the cell membrane in this manner, since the cell membrane swells by being pushed from the inside by the cytoplasm, the cut-off cell membrane includes a cytoplasmic component on the inside, and thus the cytoplasmic component can also be recovered to the flow channel 51. After the nozzle actuator 40 has cut off the necessary cytoplasm and/or cell membrane portion, the air bubble forming unit 200 proceeds the processing to S434.

FIG. 11B illustrates a behavior of recovering a cytoplasm and a cell membrane from a cell, according to the present embodiment. To bring a HeLa cell (human cervical cancer cell) cultured on a solid phase of the vessel 25 into contact with the gas-liquid interface 255 of an air bubble, with the air bubble forming unit 200 controlling the pressure generating unit 47 and the nozzle actuator 40, a relative position between the cell and the nozzle 49 was brought close (802a). Then, with the air bubble forming unit 200 performing a control to press the gas-liquid interface 255 against the cell, the cell was compressed (802b). At this time, due to the compression, a behavior that a soft portion of the cell membrane swells to the outside was observed (the arrow of 802b). Then, regarding this swollen portion, a cytoplasm and a cell membrane in the swollen portion were cut off by moving the gas-liquid interface 255 in a direction away from the cell (the arrow of 802c). Finally, the cut-off cytoplasm and cell membrane were recovered by being attached to the gas-liquid interface 255 (802d). Note that, when performing the operation of FIG. 11B, the operation may be performed by enlarging the gas-liquid interface 255, or may be performed by moving the nozzle 49, or may be performed by moving the stage.

Then, in S434, the air bubble forming unit 200 determines whether the instruction received in S140 includes continuously recovering the cytoplasm and/or the cell membrane of the manipulation target 35. The air bubble forming unit 200 proceeds the processing to S435 if the determination is positive, and to S500 if the determination is negative.

In S435, the air bubble forming unit 200 controls the pressure generating unit 47 to remove the air bubble formed on the end part 254 of the nozzle 49. When removing the air bubble, recovery of the manipulation target 35 may be performed at the same time. For example, the manipulation target 35 may be recovered into liquid existing in the flow channel 51. The step of removing the air bubble in S435 and the sub-step may be the same as the step of S500 and the sub-step, and the details thereof will be described below.

Then, in S436, the air bubble forming unit 200 controls the pressure generating unit 47 and the nozzle actuator 40 to perform intake of gas for forming a new air bubble on the end part 254 of the nozzle 49. The air bubble forming unit 200 sends an instruction to the nozzle actuator 40 to take out the nozzle 49 from the liquid. After the nozzle actuator 40 has taken out the nozzle 49 from the liquid, the pressure generating unit 47 may draw the plunger of the syringe pump to take in a necessary amount of the gas.

Then, in S437, the air bubble forming unit 200 controls the pressure generating unit 47 to form a new air bubble on the end part 254 of the nozzle 49. For the step of S437 and the sub-step, the content of S300 that is already described may be applied. After the pressure generating unit 47 has formed an air bubble on the end part 254 of the nozzle 49, the air bubble forming unit 200 proceeds the processing to the step of S420.

In S430, the air bubble forming unit 200 determines whether the instruction to recover a cell has been received in S140. The air bubble forming unit 200 proceeds the processing to S432 if the determination is positive, and the air bubble forming unit 200 proceeds the processing to S440 if the determination is negative.

In S432, the air bubble forming unit 200 controls the pressure generating unit 47 to form an air bubble, and attaches the cell to the air bubble. The air bubble forming unit 200 may, as necessary, separate the cell attached to the air bubble from the solid phase.

For example, the air bubble forming unit 200 sends an instruction to the nozzle actuator 40 to move the nozzle 49 from the initial position, in the x, y, and z directions, to a position where a target cell is present. After the nozzle actuator 40 has moved the nozzle 49 to the target position, the air bubble forming unit 200 controls the pressure generating unit 47 to supply gas to the flow channel 51, thereby forming an air bubble on the edge of the flow channel 51. Then, the air bubble forming unit 200 may control the nozzle actuator 40 or the sample actuator 41 to move the nozzle 49 or the stage, thereby bringing the gas-liquid interface 255 of the air bubble into contact with the cell.

As an example, the nozzle actuator 40 or the sample actuator 41 identifies the position where the target cell is present, from an image that is obtained by imaging the target cell with the camera 60 or the camera 70, and moves the center of the nozzle 49 to match the target position. After the nozzle actuator 40 or the sample actuator 41 has moved the nozzle 49 to the target position, the air bubble forming unit 200 controls the pressure generating unit 47 to supply gas to the flow channel 51, thereby forming an air bubble on the edge of the flow channel 51. Then, the air bubble forming unit 200 may move the nozzle 49 and/or the stage with the nozzle actuator 40 or the sample actuator 41, and bring the gas-liquid interface 255 of the air bubble into contact with the cell. For example, after bringing the cell into contact with the gas-liquid interface 255 of the air bubble, the nozzle actuator 40 may separate the cell, as necessary, by a means such as moving the gas-liquid interface 255.

After the air bubble forming unit 200 has controlled the pressure generating unit 47 to form an air bubble, and attached a cell to the air bubble, the air bubble forming unit 200 proceeds the processing to S434. For the steps of S434 and onwards, the content that is already described may be applied. Note that, in this case, in the step of S434, an object to be continuously recovered is not limited to only a cytoplasm or only a cell, but it may be both the cytoplasm and the cell.

FIG. 11C illustrates a behavior of attaching an established cell line to an air bubble and separating the established cell line, according to the present embodiment. To bring a HeLa cell cultured on a solid phase of the vessel 25 into contact with the gas-liquid interface 255 of an air bubble, with the air bubble forming unit 200 controlling the pressure generating unit 47 and the nozzle actuator 40 (or the sample actuator 41 instead of the nozzle actuator 40), a relative position between the cell and the nozzle 49 was brought close (804a). Then, the cell contacted the gas-liquid interface 255 of the air bubble (804b). Then, the nozzle actuator 40 moved the gas-liquid interface 255 (804c), and the cell was attached to and separated from the gas-liquid interface 255 (804d). Note that, when performing the operation of FIG. 11C, the operation may be performed by enlarging the gas-liquid interface 255, or may be performed by moving the nozzle 49, or may be performed by moving the stage.

FIG. 11D illustrates a schematic view when repeating S430 → S432 → S434 → S435 → S436 → S437. When continuously recovering a cytoplasm and/or a cell membrane, and when continuously recovering a cell, these may be performed as shown in the schematic view of FIG. 11D. In 810a, after the nozzle actuator 40 has put the nozzle 49 in liquid, the air bubble forming unit 200 controls the pressure generating unit 47, and forms an air bubble on the end part 254 of the nozzle 49 by supplying gas to the syringe pump (not illustrated). Then, a cell adhered on a bottom surface of the vessel 25 is attached to the gas-liquid interface 255 of the air bubble and separated, and is recovered to the flow channel 51 by taking in the gas with the syringe pump. Then, in 810b, the nozzle actuator 40 pulls the nozzle 49 up from the liquid, and the syringe pump suctions the gas (for example, the air) to the flow channel 51. Then, in 810c, after the nozzle actuator 40 has put the nozzle 49 in the liquid, the syringe pump supplies gas to the end part 254 of the nozzle 49 to form a new air bubble, and a cell adhered on a bottom surface of the vessel 25 is recovered by being attached to the gas-liquid interface 255 of the air bubble. In this manner, with the air bubble forming unit 200 controlling the pressure generating unit 47 and the nozzle actuator 40, two cells (a population) can be continuously recovered in the flow channel 51, via the gas, without being mixed. A photograph showing two cells (a population) that are actually recovered with the air placed therebetween, by using this method, is also presented. Note that, FIG. 11D described the example of moving the nozzle 49, but instead of moving the nozzle 49, the operation of FIG. 11D may be performed by moving the stage.

FIG. 11E illustrates a behavior of subculture in which an established cell line is recovered, and the recovered established cell line is cultured, according to the present embodiment. With the air bubble forming unit 200 controlling the pressure generating unit 47 and the nozzle actuator 40, a HeLa cell (human cervical cancer cell, 820a), a HT29 cell (human large bowel cancer cell, 820b), and a Kato III cell (human gastric signet ring cell cancer cell, 820c) that are established cell lines were attached to, and separated and recovered from a solid phase of the vessel 25 by using the gas-liquid interface 255 of an air bubble, and were released to a medium in another vessel to be cultured for 1.5 day (820a and 820b) and for 2 days (820c). For each cell, a cell proliferation was confirmed after the subculture. That is, according to the present embodiment, even when an established cell line was separated by using the gas-liquid interface 255 of an air bubble, the cell could be proliferated without damaging the viability of the cell. Note that, when performing the operation of FIG. 11E, the operation may be performed by moving the nozzle 49, or may be performed by moving the stage.

FIG. 11F illustrates a behavior of recovering an iPS cell, and subculturing the recovered iPS cell, according to the present embodiment. With the air bubble forming unit 200 controlling the pressure generating unit 47 and the nozzle actuator 40, a colony of the cultured iPS cells was attached to, and separated and recovered from a solid phase of the vessel 25 by using the gas-liquid interface 255 of an air bubble, and was released in a liquid culture medium in another vessel for observation of a transmission image after culturing for 4 days. In addition, as a comparative example, cells obtained by subculturing an iPS cell with an ordinary cell subculturing method (mechanical passage) was used. As a result, no morphological difference was recognized between the iPS cells of the present embodiment (830a) and the iPS cells of the comparative example (830b). In addition, after culturing for 10 days, staining of alkaline phosphatase was performed on the iPS cells of the present embodiment and the comparative example. Furthermore, after subculturing the iPS cell of the present embodiment and the comparative example for three times, and culturing the iPS cell for 30 days, staining of alkaline phosphatase was performed on the iPS cells of the present embodiment and the comparative example. As a result, no difference in stainability was recognized between the iPS cells of the present embodiment (831a is those cultured for 10 days, and 832a is those cultured for 30 days) and the iPS cells of the comparative example (831b is those cultured for 10 days, and 832b is those cultured for 30 days). It has been known that alkaline phosphatase is highly expressed in an iPS cell maintaining a self-replication ability that is undifferentiated. That is, according to the present embodiment, even when an iPS cell was separated and recovered by using the gas-liquid interface 255, the iPS cell could be proliferated while maintaining an undifferentiated state, without affecting maintainability of undifferentiation ability. Note that, when performing the operation of FIG. 11F, the operation may be performed by moving the nozzle 49, or may be performed by moving the stage.

FIG. 11G illustrates a behavior of recovering an established cell line, and analyzing the recovered cell, according to the present embodiment. A HeLa cell that is an established cell line was separated and recovered from a solid phase of the vessel 25 by using the gas-liquid interface 255 of an air bubble. With the air bubble forming unit 200 controlling the pressure generating unit 47 and the nozzle actuator 40, one cell, four cells, and eight cells of the HeLa cells were selected and separated from the solid phase by using the gas-liquid interface 255 of the air bubble, and these cells were each recovered together with a liquid culture medium of 7.5 nL (835a). Then, the recovered HeLa cells were released to a 12.5 μL cytolytic reagent, and the cells were lysed (835b). In the cell lysate in which the HeLa cells are lysed, from mRNA of β-actin, a cDNA was synthesized, and a PCR reaction was performed (835c). As a result, an amount of cDNAs generally proportional to the number of recovered cells could be detected. That is, according to the present embodiment, cells could be recovered by one cell or an optional number of cells by using the gas-liquid interface 255, and a molecular biological analysis could be performed. Note that, when performing the operation of FIG. 11G, the operation may be performed by moving the nozzle 49, or may be performed by moving the stage.

Then, in S440, the air bubble forming unit 200 determines whether the instruction to retain and image the cell has been received in S140. The air bubble forming unit 200 proceeds the processing to S442 if the determination is positive, and to S450 if the determination is negative.

In S442, the air bubble forming unit 200 performs a control to attach the cell to the formed air bubble, and retain the attached cell. For example, the air bubble forming unit 200 sends an instruction to the nozzle actuator 40 to move the nozzle 49 from the initial position, in the x, y, and z directions, to a position where a target cell is present. After the nozzle actuator 40 has moved the nozzle 49 to the target position, the air bubble forming unit 200 controls the pressure generating unit 47 to supply gas to the flow channel 51, thereby forming an air bubble on the edge of the flow channel 51. The air bubble forming unit 200 may move the nozzle 49 and/or the stage with the nozzle actuator 40 or the sample actuator 41, and may bring the gas-liquid interface 255 of the air bubble into contact with the cell. As an example, the nozzle actuator 40 or the sample actuator 41 identifies the position where the target cell is present, from an image that is obtained by imaging the target cell with the camera 60 or the camera 70, and moves the center of the nozzle 49 to match the target position. After the cell has been brought into contact with the gas-liquid interface 255 of the air bubble, the air bubble forming unit 200 proceeds the processing to S444.

The position where the target cell is present may be identified by the manipulator. In this case, the air bubble forming unit 200 may receive, from the input unit 180, an input by the manipulator as to the position where the target cell is present, and identify the position. In addition, an edge surface of the air bubble is brought into contact with the cell in the above-described example, but the contact between the air bubble and the cell may be performed by bringing a side surface of the air bubble into contact with the cell. In this case, the nozzle actuator 40 or the sample actuator 41 may move the center of the nozzle 49 to match a vicinity of the target cell. In addition, for example, after bringing the cell into contact with the gas-liquid interface 255 of the air bubble, the nozzle actuator 40 may separate the cell as necessary, by a means such as moving the gas-liquid interface 255.

Then, in S444, the imaging control unit 171 sends an instruction to the camera 60 or the camera 70 to image the cell retained in the air bubble. The camera 60 or the camera 70 captures an image, and sends the image to the image processing unit 300. The image processing unit 300 may record the image in the recording unit 190 and/or output the image to the air bubble forming unit 200.

After the camera 60 or the camera 70 has imaged the retained cell, the air bubble forming unit 200 proceeds the processing to S500.

FIG. 11H is a schematic view showing a behavior of retaining an established cell line in the gas-liquid interface 255 of an air bubble, and performing an observation. Since a floating cell or a cell that is weakly adhered freely moves in a culture solution due to a slight vibration or the like, it is difficult to observe such cell as it is by using a microscope or the like. In 840a, the air bubble forming unit 200 controls the nozzle actuator 40 to put the end part 254 of the nozzle 49 in a liquid culture medium in the vessel 25 where a floating cell (the manipulation target 35) is cultured. Then, the pressure generating unit 47 supplies gas to the flow channel 51 to form an air bubble on the edge of the nozzle 49, thereby forming the gas-liquid interface 255. The air bubble forming unit 200 attaches a floating cell to become the manipulation target 35 to the gas-liquid interface 255, which is formed by controlling the nozzle actuator 40 or the pressure generating unit 47. Then, in 840b, the pressure generating unit 47 controls an inner pressure of the air bubble to temporarily retain the cell on the gas-liquid interface 255 of the air bubble. Then, in 840c, the pressure generating unit 47 takes in the gas from the flow channel 51 to shrink the air bubble. The cell retained in that state can be observed by using a microscope or the like. Alternatively, the pressure generating unit 47 may retain the cell without shrinking the air bubble, and the retained cell may be observed by using a microscope or the like. In this manner, according to the present embodiment, by retaining a cell on the gas-liquid interface 255 of an air bubble, an observation can be performed without moving the cell.

In addition, in the case of adherent cells that are dispersed on a solid phase of the vessel 25, when performing an observation with a microscope or the like, it is necessary to move the stage and observe with a wide field. In 842a, the air bubble forming unit 200 controls the nozzle actuator 40 to put the end part 254 of the nozzle 49 in a liquid culture medium of the vessel 25 where an adherent cell (the manipulation target 35) is cultured. Then, the pressure generating unit 47 supplies gas to the flow channel 51 to form an air bubble on the edge of the nozzle 49, thereby forming the gas-liquid interface 255. Then, the air bubble forming unit 200 controls the nozzle actuator 40 or the pressure generating unit 47 to control the gas-liquid interface 255, thereby attaching the cells to the gas-liquid interface 255 and separating the cells. Then, in 842b, the pressure generating unit 47 temporarily retains the cells on the gas-liquid interface 255 of the air bubble. Then, in 842c, the pressure generating unit 47 takes in the gas from the flow channel 51 to shrink the air bubble. The cells retained in such state are present in a narrow range on a plane surface of the same z position, and thus the cells can be observed all at once by using a microscope or the like. In this manner, according to the present embodiment, a field to be observed can be reduced by retaining cells on a gas-liquid interface of an air bubble.

As an example of such observation technique, Kato III cells that are floating cells were dispersed on a solid phase, and some of the cells were attached to the gas-liquid interface 255 (844a). Subsequently, the air bubble forming unit 200 controls an inner pressure of the air bubble via the pressure generating unit 47 to retain the cells on the gas-liquid interface 255 while shrinking the air bubble, and when the retained cells were focused, surrounding cells became out of focus (844b). At this time, when moving the stage, the surrounding cells will move but the cells retained on the gas-liquid interface 255 will not move, and thus the retained cells can be easily observed with a microscope (844c).

In S450, the air bubble forming unit 200 determines whether the instruction to compress and image the cell has been received in S140. The air bubble forming unit 200 proceeds the processing to S452 if the determination is positive, and to S460 if the determination is negative.

In S452, the air bubble forming unit 200 controls the pressure generating unit 47 and the nozzle actuator 40 to compress the cell by using the air bubble. For example, the air bubble forming unit 200 controls the pressure generating unit 47 to form an air bubble on the edge of the flow channel 51, and brings the air bubble into contact with the cell to become the manipulation target 35. Note that, when performing the operation of FIG. 11H, the operation may be performed by moving the nozzle 49, or may be performed by moving the stage.

As an example, the air bubble forming unit 200 sends an instruction to the nozzle actuator 40 to move the nozzle 49 from the initial position, in the x, y, and z directions, to a position where the target cell is present. As an example, the air bubble forming unit 200 identifies the position where the target cell is present from an image that is obtained by imaging the target cell with the camera 60 or the camera 70, and controls the nozzle actuator 40 and moves the nozzle 49 to match the center of the nozzle 49 with the target position.

The position where the target cell is present may be identified by the manipulator. In this case, the air bubble forming unit 200 may receive, from the input unit 180, an input by the manipulator as to the position where the target cell is present, and identify the position. In addition, the contact between the air bubble and the cell may be performed by bringing an edge surface of the air bubble into contact with the cell, or may be performed by bringing a side surface of the air bubble into contact with the cell. When bringing the edge surface of the air bubble into contact with the cell, the nozzle actuator 40 or the sample actuator 41 may move the center of the nozzle 49 to match immediately above the target cell. When bringing the side surface of the air bubble into contact with the cell, the nozzle actuator 40 or the sample actuator 41 may move the center of the nozzle 49 to match a vicinity of the target cell, and in this case, an air bubble can be formed on the side of the cell, and the cell can be gradually compressed from the side by moving the nozzle 49.

Then, after the nozzle actuator 40 has moved the nozzle 49 to the target position, the air bubble forming unit 200 controls the pressure generating unit 47 to supply gas to the flow channel 51, thereby forming an air bubble on the edge of the flow channel 51 in the target position. The air bubble forming unit 200 may move the nozzle 49 and/or the stage with the nozzle actuator 40 or the sample actuator 41, and may bring the gas-liquid interface 255 of the air bubble into contact with the cell. When the position of the nozzle 49 is fixed, the gas-liquid interface 255 and the cell may be brought into contact by moving the stage. The cell may be brought into contact with the gas-liquid interface 255 with a control by the air bubble forming unit 200 to enlarge the gas-liquid interface 255 via the pressure generating unit 47.

As an example, the air bubble forming unit 200 operates the plunger of the syringe pump of the pressure generating unit 47 with a preset operation amount to form an air bubble of a preset volume, and then uses the nozzle actuator 40 or the sample actuator 41 to move the nozzle 49 and/or the stage such that the nozzle 49 is positioned at a position where the air bubble compresses the cell. Then, the air bubble forming unit 200 may compress the cell by enlarging the air bubble formed on the edge of the flow channel 51 by controlling the pressure generating unit 47, or by pressing the air bubble against the cell by controlling the nozzle actuator 40 to move the nozzle 49 toward the cell.

In addition, as an example, the air bubble forming unit 200 may compress the cell by, after moving the nozzle 49 very near the cell, controlling the pressure generating unit 47 to form an air bubble of a preset volume on the edge of the flow channel 51.

Then, in S454, the imaging control unit 171 sends an instruction to the camera 60 or the camera 70 to image the compressed cell. The camera 60 or the camera 70 captures an image, and sends the image to the image processing unit 300. The image processing unit 300 may record the image in the recording unit 190 and/or output the image to the output unit 160.

Furthermore, instead of/in addition to this, the sensor unit 48 or the nozzle actuator 40 may measure a pressure of compressing the cell with the air bubble, and send a measured value of the pressure to the air bubble forming unit 200. The camera 60 or the camera 70 may repeat capturing of an image while changing a pressure of compressing the cell with the air bubble forming unit 200. The pressure of compressing the cell can be changed by the air bubble forming unit 200 controlling the pressure generating unit 47 and changing an inner pressure and/or a volume of the air bubble.

As an example, the air bubble forming unit 200 may move the nozzle 49 very close to the cell, then control the pressure generating unit 47 to form an air bubble at the edge of the flow channel 51, and compress the entire cell or various sites of the cell while changing the inner pressure and/or volume of the air bubble to maintain or change the pressure of compressing the cell. It is expected that various sites of the cell have different hardness depending on the composition or distribution of the cell membrane or the intracellular organelle. In this way, the composition or distribution of the cell membranes in the cell or the intracellular organelle can be analyzed by using, as indexes, the pressure and/or the observation image at the time of the compression by the air bubble. By compressing the cell, the thickness of the cell is reduced so that a structure in the deep portion of the cell can be clearly observed, and by expanding the cell in the lateral direction, adjacent structures are separated to be individually separated and observed. By observing the cell while compressing the cell, it is possible to obtain information on the force applied to the cell and the internal and external morphological change amounts of the cell, and it is possible to analyze the information on the mechanism of the cell. After the camera 60 or the camera 70 images the compressed cell, the air bubble forming unit 200 proceeds the processing to S500.

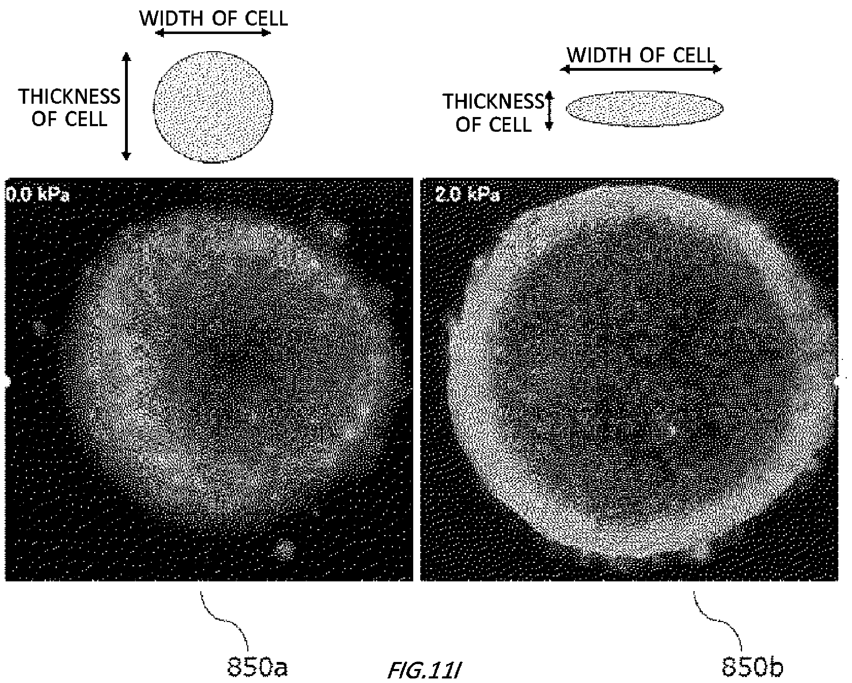

Fig. 11I illustrates a behavior of compressing the cell line by using the air bubble and observing the deep portion of the cell according to the present embodiment. By staining the nucleus and cytoplasm of a spheroid (850a) formed from a HT29 cell in a living state and performing compression by using the air bubble, it was possible to observe a structure, such as the nucleus, in the deep portion of the cell (850b). In particular, when comparison is performed with a thick central portion, the nucleus cannot be seen in the central portion in 850a before the compression, but the nucleus can be confirmed in the central portion in 850b obtained by observation with the compression. Conventionally, in order to observe a structure in the deep portion of the cell, observation can be performed by fixing the cell with formalin, methanol, or the like and thinly slicing the cell, or observation can be performed by using a special microscope specialized for deep portion observation. According to the present embodiment, the structure in the deep portion of the cell can be observed in a living state without using a special microscope. Furthermore, in the spheroid (850a) before the compression, the nuclei were in close contact with each other, and it was difficult to recognize individual nuclei, but in the spheroid (850b) obtained by observation with the compression, the spheroid expands in the longitudinal and lateral directions, so that a sufficient interval was generated between the nuclei, and the nuclei could be recognized independently. Conventionally, in order to independently recognize two or more adjacent organelles inside a cell, a microscopic system in which an optical system and a fluorescent labeling method are devised has been researched and developed, and called a super-resolution microscope. In these microscope technologies, a resolution increases, but an observation field becomes narrow, and an imaging time is lengthened. According to the present embodiment, without using a special microscope, two or more close organelles inside the cell can be independently recognized in the living state in a short imaging time while maintaining the observation field. In addition, the three-dimensional structure of the original cell can be reproduced from the observation image and the mechanism information of the compressed cell.

In S460, the air bubble forming unit 200 controls the gas-liquid interface manipulation unit 101 such that necessary manipulations other than S410 to S450 in the instruction received in S140 are performed on the manipulation target 35. For example, the manipulation may be evaluation of cell adhesiveness, induction of cell differentiation, and the like, which will be described below. After completing S460, the air bubble forming unit 200 proceeds the processing to S500.

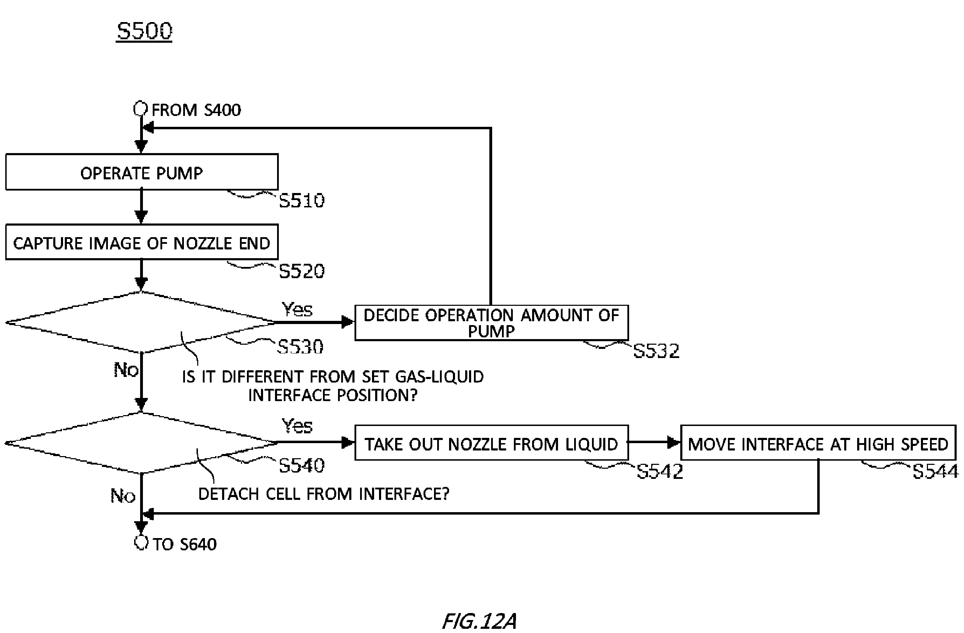

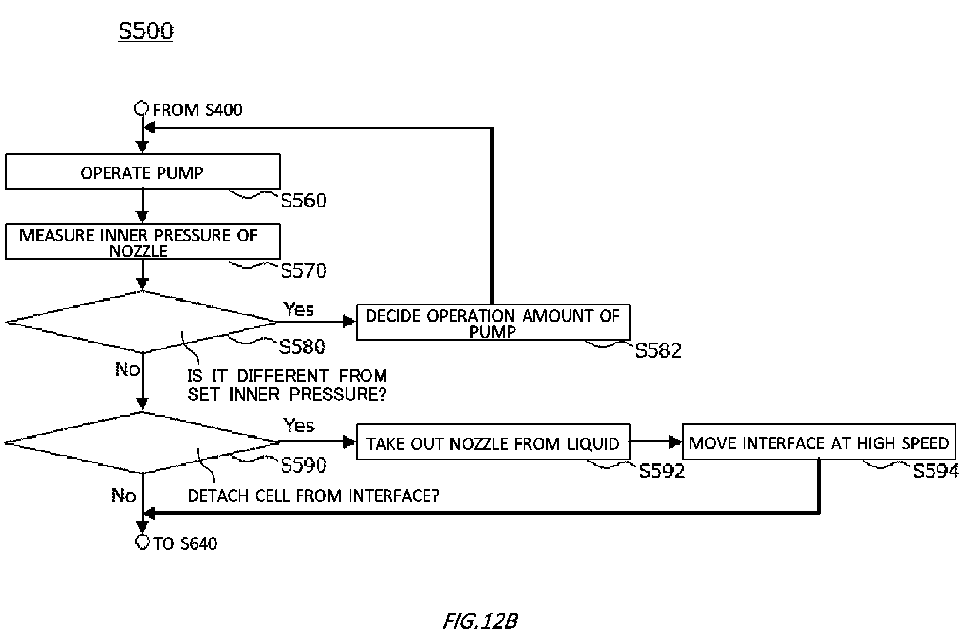

In S500, the air bubble forming unit 200 controls the pressure generating unit 47 to shrink the gas-liquid interface 255. Shrinking the gas-liquid interface 255 may include removing the air bubble. When the air bubble is shrunk or removed, the manipulation target 35 may be recovered simultaneously. In S500, the step of shrinking the gas-liquid interface 255 includes steps of S510 to S544 as illustrated in Fig. 12A, or includes steps of S560 to S594 as illustrated in Fig. 12B.

Fig. 12 is an example of a flow of shrinking the gas-liquid interface 255 on the basis of the image obtained by capturing the position of the end part 254 of the nozzle 49.

In S510, the air bubble forming unit 200 controls the pressure generating unit 47 to perform the suction operation on the flow channel 51 and take in the gas-liquid interface 255 from the edge of the flow channel 51. At this time, the liquid is also taken in simultaneously. The liquid to be taken in may be used for detaching the manipulation target 35 from the gas-liquid interface 255. The liquid to be taken in may be a liquid accommodated in the vessel 25 or the like (for example, a medium), or may be another liquid housed in the liquid storage part 54.

For example, the air bubble forming unit 200 sends an instruction to the actuator of the pressure generating unit 47 to draw the plunger of the syringe pump of the pressure generating unit 47 by a preset distance or to draw the plunger of the syringe pump until the pressure reaches a preset pressure. When receiving an instruction from the volume control part or the gas intake control part in the air bubble forming unit 200, the pressure generating unit 47 takes in gas. As a result, the gas-liquid interface 255 is shrunk (the air bubble is removed), and the gas-liquid interface 255 is taken into the flow channel 51. After the gas-liquid interface 255 is shrunk (the air bubble is removed), the air bubble forming unit 200 proceeds the processing to S520.

Next, in S520, the flow channel imaging camera 42 captures an image of the end part 254 of the nozzle 49, and sends the image to the image processing unit 300. The image processing unit 300 may record the image in the recording unit 190 and/or output the image to the air bubble forming unit 200.

Next, in S530, on the basis of the captured image of the end part 254 of the nozzle 49, the air bubble forming unit 200 determines whether the position of the gas-liquid interface 255 taken in by the flow channel 51 is different from the preset position in the air bubble forming unit 200. When the positions are different from each other, the air bubble forming unit 200 proceeds the processing to S532, and if not, proceeds the processing to S540. For example, the air bubble forming unit 200 may calculate a difference between the position of the gas-liquid interface 255 calculated on the basis of the captured image of the end part 254 of the nozzle 49 and the preset position, and if the difference is equal to or larger than a threshold, the air bubble forming unit 200 may determine that the position of the gas-liquid interface 255 taken in by the flow channel 51 is different from the set position.

In S532, the air bubble forming unit 200 decides the operation amount of the plunger of the syringe pump of the pressure generating unit 47. For example, the air bubble forming unit 200 decides the operation amount (for example, the distance of pushing or drawing the plunger of the syringe pump) of the plunger of the syringe pump of the pressure generating unit 47 in order to take the gas-liquid interface 255 to the preset position of the gas-liquid interface 255. The air bubble forming unit 200 sends an instruction to the pressure generating unit 47 to operate by the decided operation amount. For example, the air bubble forming unit 200 may decide the operation amount of an amount according to the level of the difference calculated in S530.

The operation amount may be the operation amount of the actuator of the pressure generating unit 47, or may be an additional pressure loaded on the syringe pump. The pressure generating unit 47 receives the instruction, and the air bubble forming unit 200 proceeds the processing to S510. In S510 of the second and subsequent times, the pressure generating unit 47 operates by the amount according to the operation amount.

In S540, when the instruction received in S140 includes detaching the cell from the interface (for example, cell recovery), the air bubble forming unit 200 proceeds the processing to S542, and if not, the air bubble forming unit 200 proceeds the processing to S640.

In S542, the air bubble forming unit 200 sends, to the nozzle actuator 40, an instruction to take out the nozzle 49 from the liquid. The nozzle actuator 40 moves the nozzle 49 upward by a preset distance to take out the nozzle 49 from the liquid, and then the air bubble forming unit 200 proceeds the processing to S544.

Next, in S544, the air bubble forming unit 200 controls the pressure generating unit 47 to move the gas-liquid interface 255 between the gas and the liquid in the flow channel 51 at a high speed. In this manner, the cell attached to the gas-liquid interface 255 is detached from the gas-liquid interface 255 and moves to the liquid. For example, the air bubble forming unit 200 causes the pressure generating unit 47 to rapidly perform the reciprocating operation of the plunger of the syringe pump to repeat the gas supply and the gas intake in the flow channel 51, thereby moving the gas-liquid interface 255 at a high speed. In addition, additionally/alternatively, the air bubble forming unit 200 may cause the nozzle actuator 40 to reciprocate the nozzle 49 at a high speed in the up-and-down direction ($\pm$ z direction) and/or the longitudinal and lateral directions ($\pm$ xy direction), thereby moving the gas-liquid interface 255 in the flow channel 51 at a high speed.

The air bubble forming unit 200 may cause the high-speed movement of the gas-liquid interface 255 to be performed on the spot (at the place where S542 is performed), or may cause the high-speed movement to be performed after the nozzle actuator 40 is caused to put the nozzle 49 into the liquid of a designated movement destination. The air bubble forming unit 200 can appropriately detach the cell attached to the gas-liquid interface 255 from the gas-liquid interface 255 by controlling the moving speed of the liquid or the like from the information such as the inner pressure of the nozzle 49 received from the sensor unit 48. In addition, the air bubble forming unit 200 may vibrate the gas-liquid interface 255 by forming an electromagnetic field with respect to the nozzle 49.

Furthermore, the air bubble forming unit 200 may control the cell to be detached from the gas-liquid interface 255 by bringing the air bubble into contact with the filter. The air bubble forming unit 200 may control the liquid storage part 54 to add a liquid for reducing the free energy of the interface, thereby detaching the cell from the gas-liquid interface 255. Additionally, the air bubble forming unit 200 may control the nozzle actuator 40 and the pressure generating unit 47 to form an air bubble at the edge of the nozzle 49 at the designated movement destination, and detach the cell by rubbing the cell against the bottom surface of the vessel 25 at the designated movement destination. In addition, the air bubble forming unit 200 may push out and detach the cell by controlling the pressure generating unit 47 to increase the inner pressure of the air bubble. In addition, the cell may be detached from the gas-liquid interface 255 by making the liquid at the movement destination into a liquid in which the interface free energy decreases. After the cell is detached from the interface, the air bubble forming unit 200 proceeds the processing to S640.

Fig. 12B is an example of a flow of shrinking the gas-liquid interface 255 on the basis of the inner pressure of the nozzle 49.

In S560, the air bubble forming unit 200 controls the pressure generating unit 47 to perform the suction operation on the flow channel 51 and take in the gas-liquid interface 255 from the edge of the flow channel 51. The step of S560 may be the same as the step of S510. Next, the air bubble forming unit 200 proceeds the processing to S570.

Next, in S570, the sensor unit 48 measures the inner pressure of the nozzle 49, and sends the measured value of the inner pressure of the nozzle 49 to the air bubble forming unit 200. Note that, instead of the sensor unit 48, the nozzle actuator 40 may measure the inner pressure of the nozzle 49, and send the measured value of the inner pressure to the air bubble forming unit 200.

Next, in S580, the air bubble forming unit 200 determines whether the measured value of the inner pressure of the nozzle 49 is within a preset inner pressure range. When the measured value of the inner pressure is out of the preset range of the inner pressure, the air bubble forming unit 200 proceeds the processing to S582, and if not, proceeds the processing to S590. For example, the air bubble forming unit 200 may calculate a difference between the preset inner pressure and the measured inner pressure of the nozzle 49, and when the difference is equal to or greater than a threshold, determine that the set inner pressure has not been reached.

In S582, the air bubble forming unit 200 decides the operation amount (for example, the distance of pushing or drawing the plunger of the syringe pump) of the plunger of the syringe pump of the pressure generating unit 47 in order to achieve the set inner pressure of the nozzle 49. The air bubble forming unit 200 sends an instruction to the pressure generating unit 47 to operate by the decided operation amount. For example, the air bubble forming unit 200 may decide the operation amount of an amount according to the level of the difference calculated in S580.

The operation amount may be the operation amount of the actuator of the pressure generating unit 47, or may be an additional pressure loaded on the syringe pump. The pressure generating unit 47 receives the instruction, and the air bubble forming unit 200 proceeds the processing to S560. In S560 of the second and subsequent times, the pressure generating unit 47 operates by the amount according to the operation amount.

In S590, when the instruction received in S140 includes detaching the cell from the gas-liquid interface 255 (for example, cell recovery), the air bubble forming unit 200 proceeds the processing to S592. The steps S590 to S594 may be the same as the steps S540 to S544. After completing S594, the air bubble forming unit 200 proceeds the processing to S640. When the instruction received in S140 does not include detaching the cell from the gas-liquid interface 255 from the gas-liquid interface 255, the air bubble forming unit 200 proceeds the processing to S640.

Next, in S640, the information processing device 170 receives, via the input unit 180, an input regarding the release of the manipulation target 35 from the manipulator. When the information processing device 170 has received the instruction to release the manipulation target 35, the information processing device 170 proceeds the processing to S645, and if not, proceeds the processing to S650.

In S645, the air bubble forming unit 200 may send, to the nozzle actuator 40, an instruction regarding the movement destination of the recovered manipulation target 35. For example, the movement destination of the manipulation target 35 may be designated by the manipulator in the display region of the GUI as illustrated in Fig. 7B. The nozzle actuator 40 may immerse, in the liquid of the movement destination, the nozzle 49 including the manipulation target 35 attached to the gas-liquid interface 255 or detached from the gas-liquid interface 255 and release the manipulation target to the liquid of the movement destination. After the nozzle actuator 40 releases the recovered cell to the liquid of the movement destination, the air bubble forming unit 200 proceeds the processing to S650. Note that the cell released into the liquid of the movement destination may be observed by using the microscope unit 50.

When there is another manipulation target 35 in S650, the air bubble forming unit 200 proceeds the processing to S660. When there is no other manipulation target 35 in S650, the air bubble forming unit 200 proceeds the processing to S680.

In S660, when the nozzle 49 needs to be exchanged in manipulating another manipulation target 35, the air bubble forming unit 200 proceeds the processing to S670, and when the nozzle 49 does not need to be exchanged, the air bubble forming unit proceeds the processing to S200.

In S670, the flow channel control unit 250 sends, to the flow channel exchange part 53, an instruction to remove the nozzle 49 installed in the nozzle actuator 40 and discard the nozzle to the nozzle discarding part of the flow channel exchange part 53. Note that the nozzle may be stored in a state where the cell is taken into the nozzle 49 without being released, and then the taken cell may be analyzed. In this case, the nozzle 49 may be housed in the nozzle housing part of the flow channel exchange part 53 without being discarded. After the flow channel exchange part 53 discards the nozzle 49, the processing proceeds to S180.

In S680, the nozzle 49 may be discarded in a procedure similar to S670. The flow channel exchange part 53 discards the nozzle 49, and the flow ends.

In the above flow, the case of removing unnecessary cells, recovering cytoplasm and/or a cell membrane, recovering and subculturing a cell, retaining the cell, and compressing the cell has been described as an example of manipulating the manipulation target 35. Examples of the manipulation include some other than those listed above.

An example of the manipulation includes applying a culture substrate or an agent to the solid phase on the bottom surface of the vessel 25 and evaluating the adhesiveness of the culture substrate or the agent to cells. Since the adhesiveness to cells can be evaluated by using, as indices, the inner pressure of the air bubble, the moving speed of the nozzle, and the load at the time of separating the cells, the effectiveness of the adhesion of the culture substrate and the agent to the cells can be evaluated.

Another example of the manipulation includes sorting cells. According to the above-described flow, the cells are compressed by the air bubble. It is conceivable that a cell membrane, an intracellular component, or a physical property varies depending on the type of the cell. Therefore, it is conceivable that the shape change process of the cell and the shape of the cell vary after the air bubble forming unit 200 controls the nozzle actuator 40 and/or the pressure generating unit 47 to start the compression of the cell by the air bubble, to stop the compression, or to release the cell. In addition, it is also conceivable that in a certain type of cell, a cell appears which ruptures when compressed. The cells can be sorted by using these as indices.

Another example of the manipulation includes observing a change in shape in the process of compressing the cell. According to the above-described flow, the cells are compressed by the air bubble. For example, in the process of compressing the cell, the entire cell or various sites of the cell may be compressed while changing the pressure of compressing the cell, and the change in the shape of the cell may be imaged.

Another example of the manipulation includes rupture or cleavage due to the compression of the cell. By applying a large pressure to the cell, the cell can be ruptured or cleaved. By rupturing or cleaving the cell, the cell membrane, the cytoplasm, the organelle, and/or the like can be recovered, and connection (for example, a synapse that is a connection between nerve cells) between cells can be cut.

Another example of the manipulation includes induction of cell differentiation. It is known that differentiation of osteoblasts, myocytes, vascular endothelial progenitor cells, and the like is induced by giving mechanical stimulation. The differentiation can be induced by the pressure generating unit 47 compressing these cells by using the air bubble according to the above-described flow.

Another example of the manipulation includes introducing a gene into the cell. According to the above-described flow, by using the air bubble, the air bubble forming unit 200 attaches a vesicle-shaped object such as a cell membrane to the gas-liquid interface 255 to be contact with the cell membrane, whereby the contents of the vesicle are taken into the cell by membrane fusion. At this time, by incorporating the gene in the vesicle, the gene can be taken into the cell. In addition, not only genes but also other macromolecules can be taken into the cell through a pore. In addition, according to the above-described flow, when the air bubble forming unit 200 uses the air bubble to compress the cell by the gas-liquid interface 255, a minute gap is likely to be generated in a part of the membrane in the process of deforming the cell. At this time, by adding the gene into the medium of the cell, the gene can be taken into the cell through the gap. In addition, not only genes but also other macromolecules can be taken into the cell through the gap.

Another example of the manipulation includes cooperation with an external device, for example, a cell culture device such as a fermenter or a cell analysis device such as a cell sorter. According to the above-described flow, the air bubble forming unit 200 may move the cell by using the air bubble to take the cell into the flow channel 51 and release the cell to a designated position of the external device to cooperate with. In addition, the cell may be moved by directly connecting the flow channel 51 to the external device to send, to the external device, the cell taken into the flow channel 51.

Another example of the manipulation includes the manipulation of an emulsion. The emulsion is a droplet in an oil liquid or an oil droplet in an aqueous solution. In order to stabilize the formed emulsion, the emulsion may contain a surfactant or the like which is an amphiphilic substance. The surfactant or the like are arranged to surround a droplet or an oil droplet, and form a monomolecular membrane on the interface. Such a monomolecular membrane is also found in a part of an intracellular organelle such as an endosome and a lipid droplet. According to the above-described flow, the air bubble forming unit 200 may use the air bubble to attach the emulsion to the gas-liquid interface 255, or may perform a further manipulation.

As a method of separating and/or recovering a cell, there are a method of locally denaturing a substrate to separate a cell by using a special substrate that reacts with temperature or light, and a method of separating a cell by ultrasonic waves, but these methods require a means of recovering the cell. However, the method of the present invention does not require a special substrate, and includes both the means for separating the cell and the means for recovering the cell. In addition, as a means for recovering the separated cell, there is a method of recovering the cell by a liquid flow, such as an aspirator, which sucks a liquid, but there is a possibility that the cell and a large amount of liquid are recovered simultaneously, or cells other than the target cell are involved. However, in the method of the present invention, the cell attached to the gas-liquid interface can be recovered by taking the gas-liquid interface into the nozzle, and the target cell can be easily recovered with a considerably small amount of liquid without involving cells other than the target cell. In addition, it is known that a strong liquid flow is applied at the time of sucking a liquid, so as to adversely affect cells, but such an adverse effect can be avoided by using the method of the present invention.

As described above, the method of manipulating the manipulation target 35 by using the gas-liquid interface 255 has been described. By stably controlling the formed gas-liquid interface 255, the manipulation target 35 can be easily and stably manipulated. Hereinafter, a method for stably controlling the gas-liquid interface 255 will be described in detail.

First, a theory for stably controlling the gas-liquid interface 255 will be described. In a state where the gas-liquid interface of the air bubble is stably maintained, an inner pressure $P_i$ of the air bubble and a Laplace pressure $P_m$ of the meniscus (gas-liquid interface 255) are balanced. The Laplace pressure $P_m$ is a pressure difference between the gas phase in the air bubble and the liquid phase of the liquid 261. That is, the following Mathematical Formula 1 is satisfied.

[Mathematical Formula 1]

$$P_i = P_m$$

In addition, a condition that a restoring force acts against a minute volume change is satisfied. When the restoring force does not act, the air bubble continues to expand, and it becomes difficult to maintain or control the air bubble. Specifically, when the following Mathematical Formula 2 is satisfied, the air bubble is stable. V represents the volume of the air bubble. In the following description, Mathematical Formula 2 is referred to as a stabilization formula.

[Mathematical Formula 2]

$$\frac{dP_i}{dV} \leq \frac{dP_m}{dV}$$

The left side of the above stabilization formula (Mathematical Formula 2) will be described. On the left side, when it is assumed that the air bubble inner pressure change with respect to a minute volume change is a quasi-static adiabatic process, the following Mathematical Formula 3 is satisfied. κ represents a specific heat ratio.

[Mathematical Formula 3]

$$\frac{dP_i}{dV} = -\kappa \frac{P_i}{V}$$

In the above Mathematical Formula 3, when V is brought close to 0 or V is diverged to infinity,

[Mathematical Formula 4]

$$\lim_{V \to 0} \frac{dP_i}{dV} = -\infty$$

$$\lim_{V \to \infty} \frac{dP_i}{dV} = -0$$

is satisfied.

Therefore, the left side of the stabilization formula (Mathematical Formula 2) can take a negative real value from 0. Therefore, in the stabilization formula (Mathematical Formula 2), as the volume V of the air bubble is decreased as much as possible, the left side of the stabilization formula (Mathematical Formula 2) becomes smaller, so that the stabilization formula (Mathematical Formula 2) is more easily satisfied, and the air bubble is stably maintained. Thus, it is shown that the volume V of the air bubble is preferably decreased in order to stably maintain the air bubble.

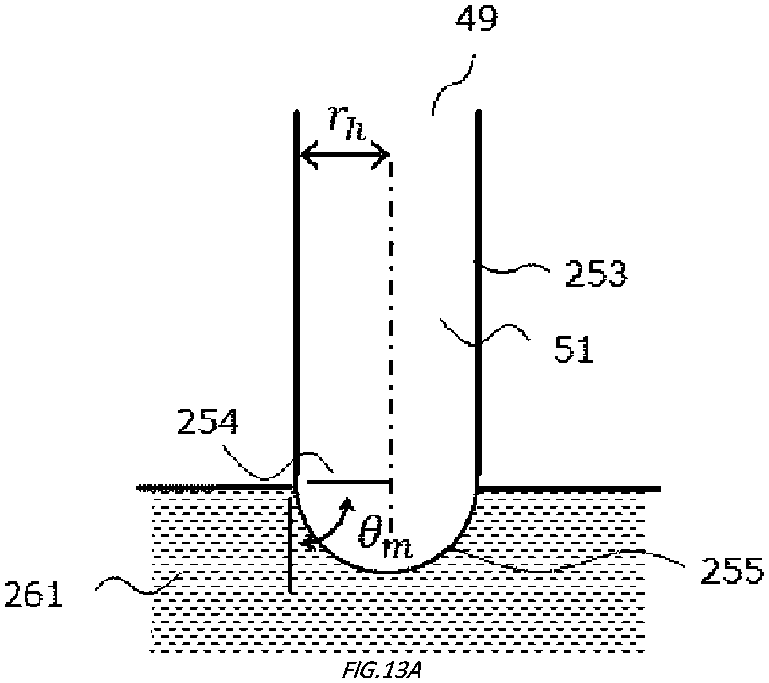

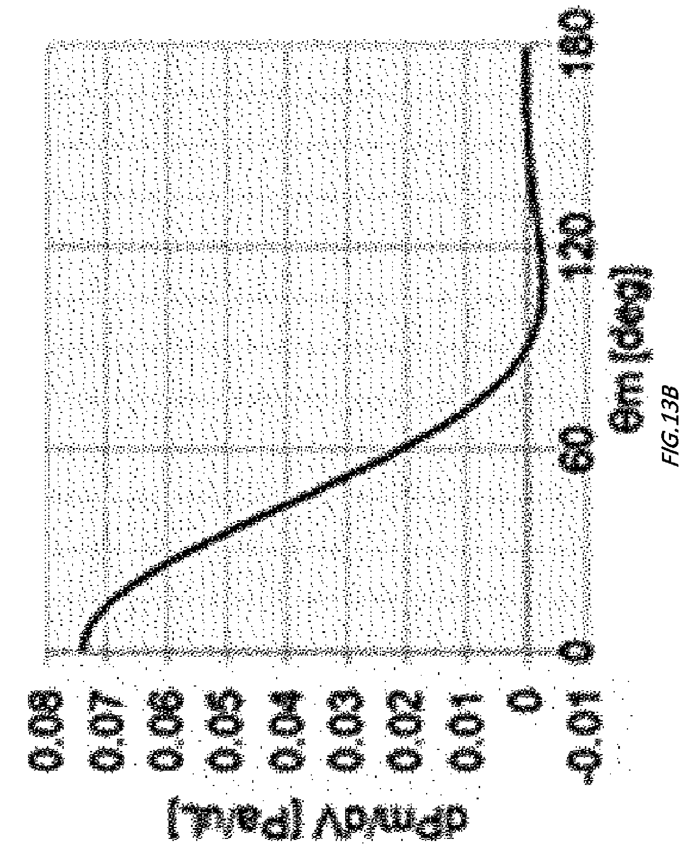

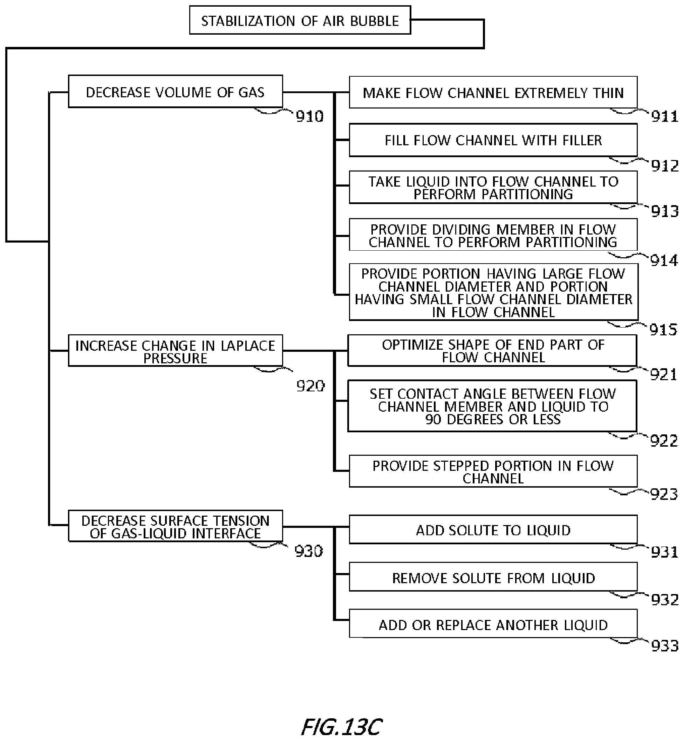

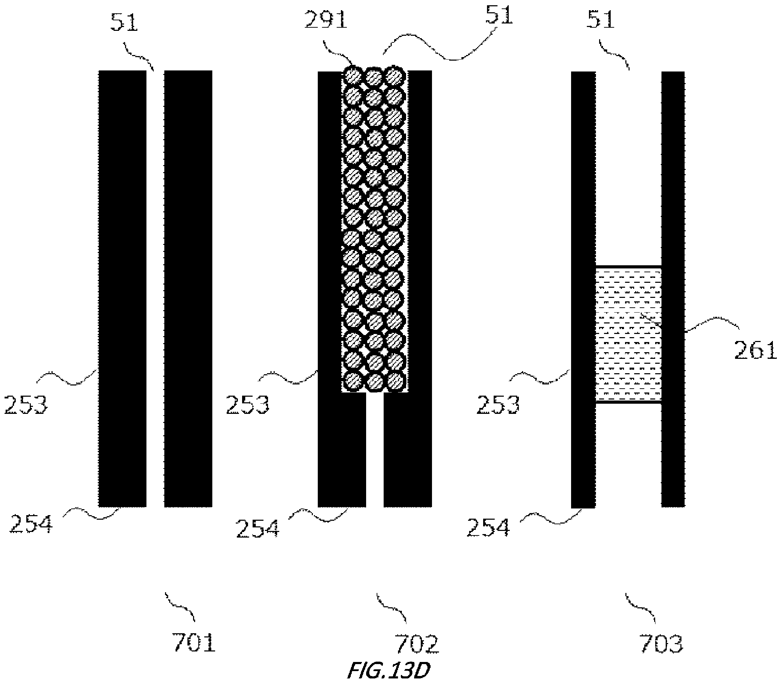

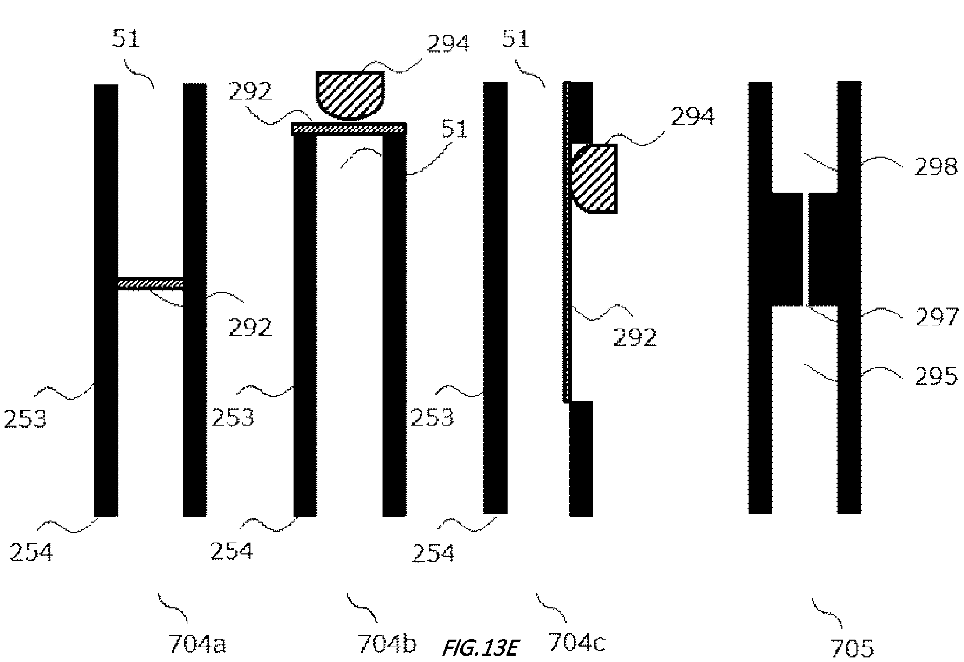

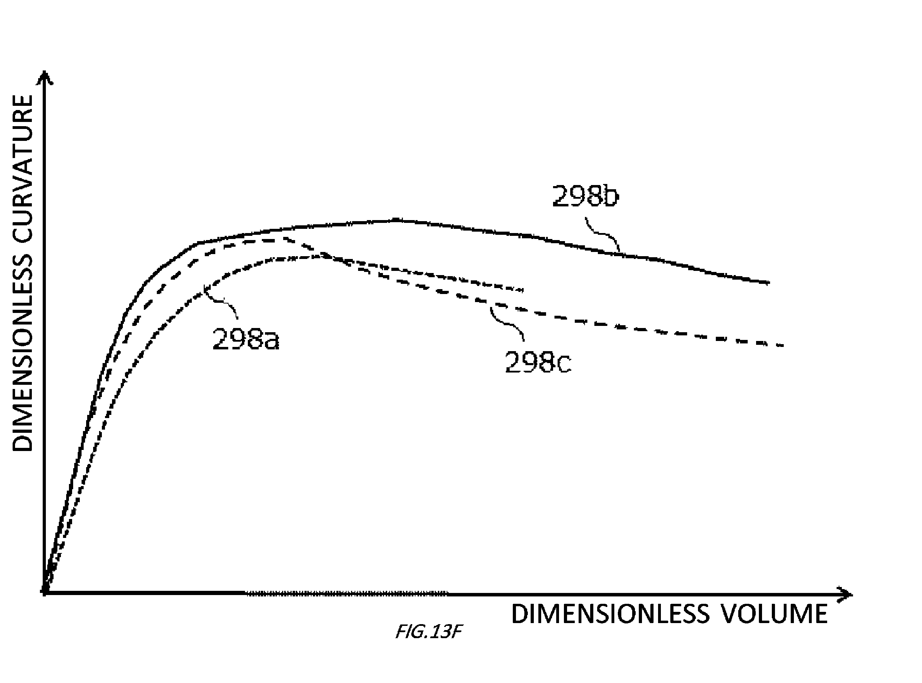

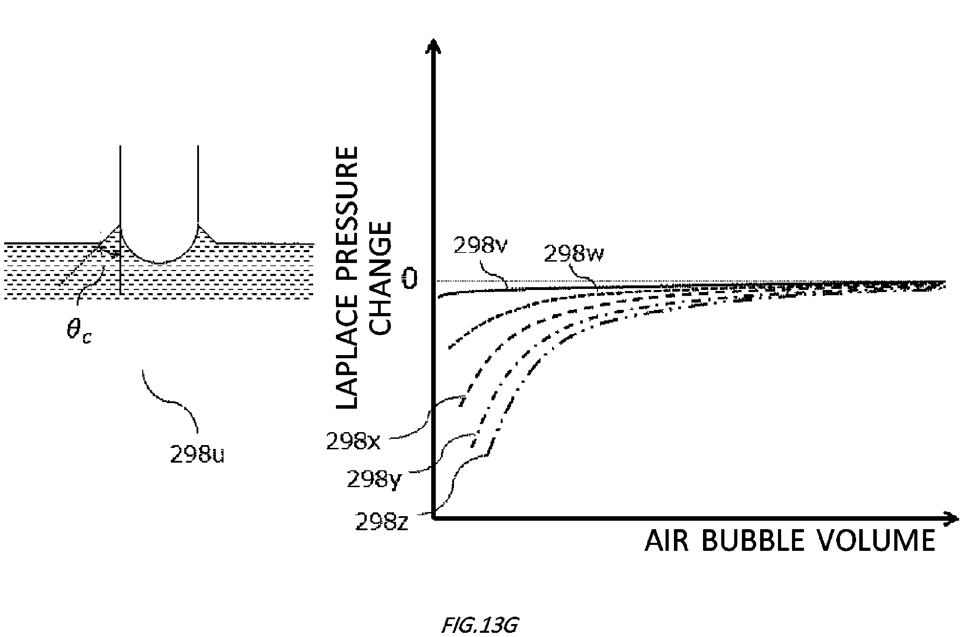

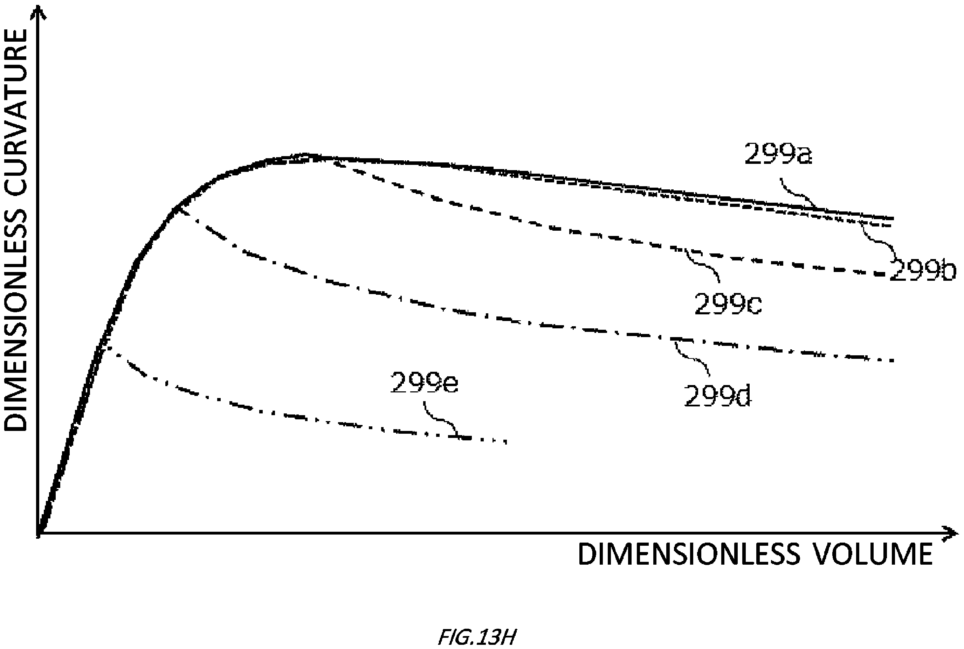

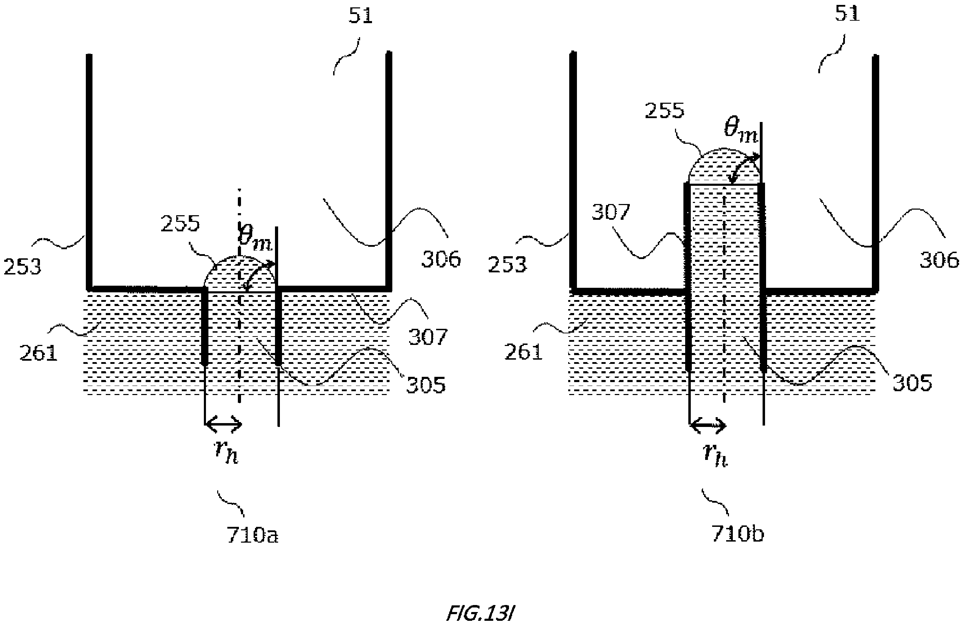

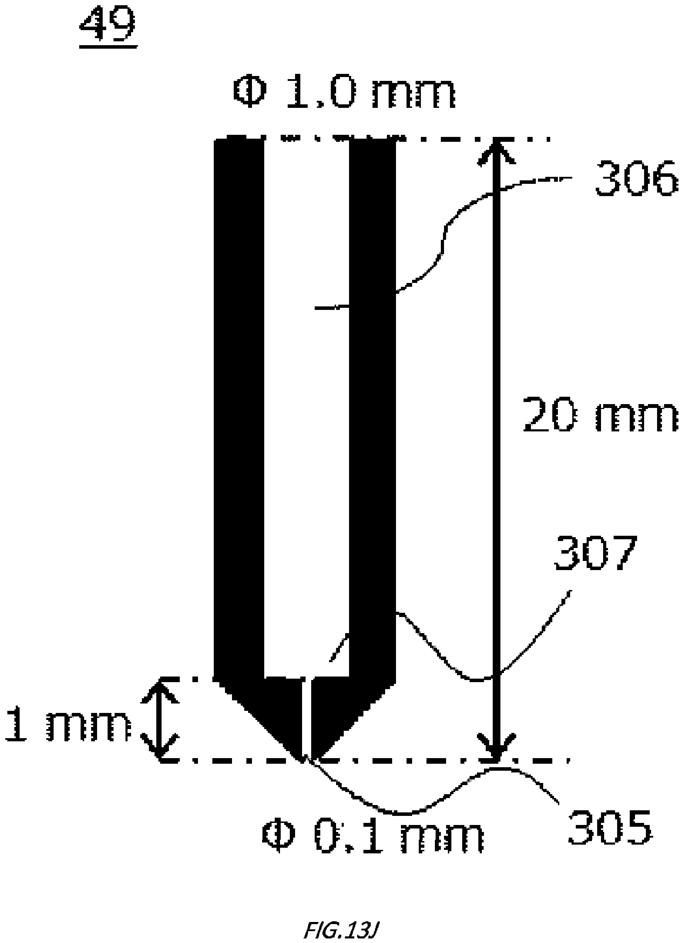

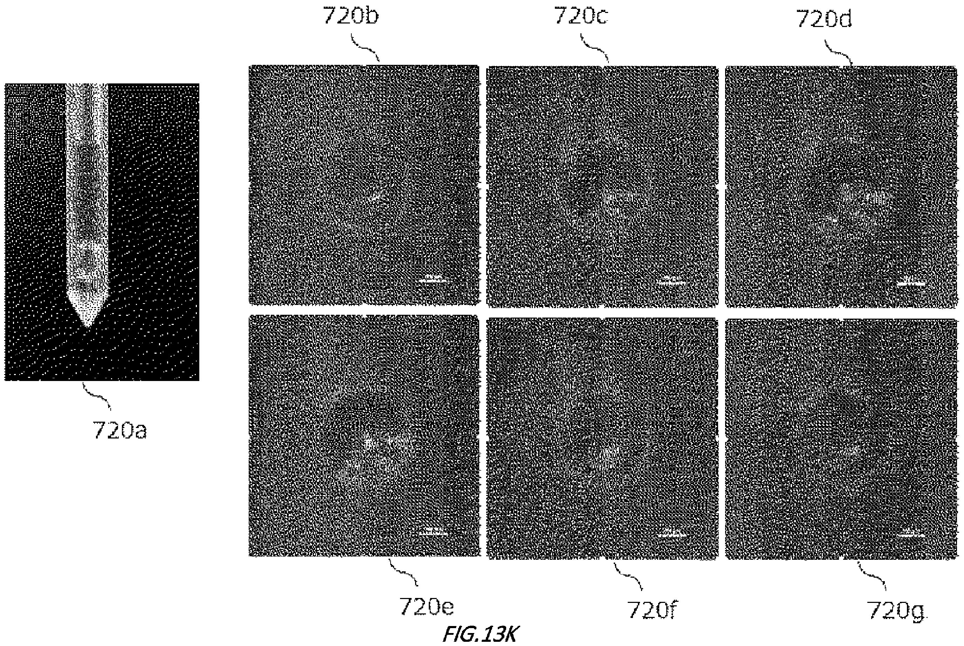

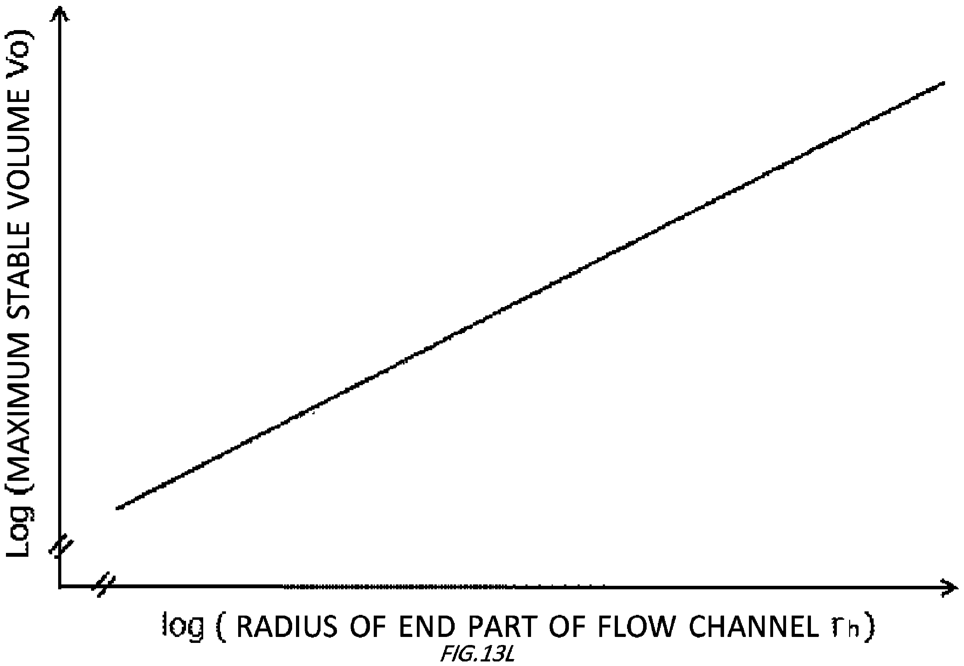

Next, the right side of the stabilization formula (Mathematical Formula 2) will be described with reference to Fig. 13A. In Fig. 13, a surface tension between the gas and the liquid 261 is y, the radius of the flow channel 51 of the nozzle 49 is $r_h$, and an angle formed by the end part 254 of the nozzle 49 and the meniscus (gas-liquid interface 255) is $\theta_m$. At this time, $P_m$ and V (described as $V_m$) on the right side of the stabilization formula (Mathematical Formula 2) are expressed by the following Mathematical Formula 5 as a function of $\theta_m$.

[Mathematical Formula 5]

$$P_m(\theta_m) = \frac{2\gamma \sin \theta_m}{r_h}$$

$$V_m(\theta_m) = \frac{\pi(\cos \theta_m - 1)^2(\cos \theta_m + 2)r_h{}^3}{3 \sin^3 \theta_m}$$

Therefore, the right side of the stabilization formula (Mathematical Formula 2) is expressed by the following Mathematical Formula 6 as a function of $\theta_m$.

[Mathematical Formula 6]

$$\frac{dP_m}{dV_m}(\theta_m) = \frac{2\gamma \cos \theta_m (\cos \theta_m + 1)^2}{\pi r_h{}^4}$$

Fig. 13B is a graph illustrating Mathematical Formula 6 described above, where a horizontal axis represents $\theta_m$, and a vertical axis represents the right side of the stabilization formula (Mathematical Formula 2), that is, Mathematical Formula 6. According to Fig. 13B, the right side of the stabilization formula (Mathematical Formula 2) is 0 or more when $\theta_m$ is 90 degrees or less, and is less than 0 when $\theta_m$ exceeds 90 degrees. Therefore, when $\theta_m$ is 90 degrees or less, the right side of the stabilization formula (Mathematical Formula 2) is 0 or more, and the left side of the stabilization formula (Mathematical Formula 2) is 0 or less as shown in Mathematical Formula 4, so that the stabilization formula (Mathematical Formula 2) is always satisfied.

As described above, when the angle $\theta_m$ formed by the end part 254 of the nozzle 49 and the meniscus (gas-liquid interface 255) is 90 degrees or less, the air bubble is stably maintained, and the control is facilitated. In addition, as the volume V of the air bubble is decreased as much as possible, the air bubble is stably maintained, and the control is facilitated.

When $\theta_m$ exceeds 90 degrees and the stabilization formula (Mathematical Formula 2) is not satisfied, the inner pressure $P_i$ of the air bubble becomes larger than the Laplace pressure $P_m$ of the meniscus (gas-liquid interface 255), and the air bubble rapidly enlarges due to the pressure difference. At this time, the inner pressure $P_i$ of the air bubble rapidly decreases due to the enlargement of the volume V of the air bubble. By detecting such a change in the inner pressure $P_i$ of the air bubble, it is also possible to experimentally detect a pressure at which the air bubble is stably maintained. For example, when the volume V of the air bubble is reduced and the inner pressure $P_i$ of the air bubble is increased, the inner pressure $P_i$ of the air bubble rapidly decreases due to the enlargement of the volume V of the air bubble at the moment when the air bubble becomes unstable, and thus, it can be known that the inner pressure $P_i$ of the air bubble immediately before the inner pressure $P_i$ of the air bubble decreases is a pressure at which the air bubble can be stably maintained. Such a method of detecting a pressure at which the air bubble can be stably maintained may be performed before the step of forming the air bubble (enlarging the gas-liquid interface 255), that is, before the step and the sub-step of S300 are performed, and the pressure at which the air bubble can be stably maintained may be used as the set inner pressure. Such a method for detecting, on the basis of the inner pressure of the air bubble, the pressure at which the air bubble can be stably maintained is also satisfied when the air bubble shape is not hemispherical, such as when the air bubble is in contact with the bottom surface.

Next, Fig. 13C is a diagram for explaining a method of stabilizing the gas-liquid interface 255 on the basis of the above description. The method for stabilizing the gas-liquid interface 255 described below may be performed before the step of forming the air bubble (enlarging the gas-liquid interface 255), that is, before the step and the sub-step of S300 are performed, but is not limited thereto. For example, the method for stabilizing the gas-liquid interface 255 can be performed in the middle of the step and the sub-step (the sub-step of S300) of forming the air bubble. A method, such as 911 described below, related to the flow channel structure may be executed in advance before S100 is performed.

For example, a case will be described in which a method of stabilizing the gas-liquid interface 255 is performed before performing the step of S300. The method for stabilizing the gas-liquid interface 255 may be performed automatically by the organism manipulation device 100, and in this case, first, the information processing device 170 may receive, from the manipulator via the input unit 180, an input regarding whether to stabilize the gas-liquid interface 255. When the information processing device 170 has received an instruction to stabilize the gas-liquid interface 255, the information processing device 170 performs any one or more of the methods indicating 910 to 933 described in Fig. 13C, and if not, the information processing device 170 proceeds the processing to S300. Note that the method of stabilizing the gas-liquid interface 255 may be performed by the hand of the manipulator instead of being automatically performed by the organism manipulation device 100. For example, when the method of stabilizing the gas-liquid interface 255 is performed by the hand of the manipulator immediately before performing the step of S300, the processing may be resumed from S300.

Figs. 13D and 13E are diagrams for explaining 910 in Fig. 13C, that is, a method of decreasing a gas volume occupied by a gas continuous from the air bubble 256 to the inside of the flow channel 51. As described above, the air bubble is stably maintained by decreasing the volume of the gas continuous with the air bubble. For example, when the flow channel 51 includes the nozzle 49 and the space formed inside the syringe pump connected to the nozzle 49, the syringe pump may reduce the flow channel volume to 10% or less, 8% or less, or 5% or less of the maximum capacity, but the present invention is not limited thereto.

An example in which 910 is specifically performed will be described. As an example, in 911, in 701 of Fig. 13D, the flow channel 51 in the nozzle 49 is made extremely thin to decrease the flow channel volume, so that the volume of the gas continuous with the air bubble is decreased, and the air bubble is stably maintained. For example, when the flow channel length of the flow channel 51 is 150 mm, the air bubble can be stably maintained by setting the flow channel diameter to 100 $\mu$m or less, 75 $\mu$m or less, or 50 $\mu$m or less. In this case, the method of 911 can be performed by installing the nozzle 49, which has the flow channel 51 made extremely thin, in the nozzle actuator 40 in advance. When it is necessary to attach the nozzle 49 to the nozzle actuator 40, the processing may start from the step of installing the nozzle in S180 again. The step of installing the nozzle 49 may be performed in the same manner as in S180. The nozzle 49 having the flow channel 51 made extremely thin may be housed in the nozzle housing part of the flow channel exchange part 53 in advance. When the gas-liquid interface manipulation unit 101 does not include the flow channel exchange part 53, the nozzle 49 having the flow channel 51 made extremely thin may be attached to the nozzle actuator 40 by the hand of the manipulator.

Another example in which 910 is specifically performed will be described. As an example, in 912, in 702 in Fig. 13D, by filling the flow channel 51 in the nozzle 49 with a filler (for example, beads 291) to block a partial inner space of the flow channel 51, the volume of the gas continuous with the air bubble is decreased, and the air bubble is stably controlled. The bead 291 may be made of metal, plastic, or rubber, but is not limited thereto. The size of the bead 291 may be an arbitrary size as long as the bead fits in the flow channel 51, and the shape of the bead 291 may be spherical, but the present invention is not limited thereto, and arbitrary size and shape can be used for the bead 291.

The total volume of the filler in the total volume of the flow channel 51 may be 95% or less. The total volume of the filler in the total volume of the flow channel 51 may be 30% or more, 60% or more, or 90% or more. The total volume of the flow channel 51 may be the volume of the space sealed between the end part 254 of the nozzle 49 and the plunger of the syringe pump connected to the nozzle 49.

The method of 912 can be performed by installing the nozzle 49, which is filled with the beads 291, in the nozzle actuator 40 in advance. When the nozzle 49 needs to be attached to the nozzle actuator 40, the step of installing the nozzle 49 in S180 may be started again. The step of installing the nozzle 49 may be performed in the same manner as in S180.

The nozzle 49 filled with the beads 291 may be housed in the nozzle housing part of the flow channel exchange part 53 after the flow channel 51 in the nozzle 49 is filled with the beads 291 in advance. Alternatively, the nozzle 49 not filled with the beads 291 may be housed in the nozzle housing part, and immediately before being installed in the nozzle actuator 40, the flow channel 51 in the nozzle 49 may be filled with the beads 291 by the hand of the manipulator, and then the nozzle 49 may be installed in the nozzle actuator 40 by the hand of the manipulator.

Another example in which 910 is specifically performed will be described. As an example, in 913, in 703 in Fig. 13D, the liquid 261 is taken into the flow channel 51 in the nozzle 49, and the gas inside the flow channel 51 is partitioned by the taken liquid 261. In this manner, by decreasing the flow channel volume related to air bubble formation, the volume of the gas continuous with the air bubble is decreased, the air bubble is stably maintained, and the control is facilitated. The liquid 261 taken into the flow channel 51 may be a liquid that fills the vessel 25 or may be the liquid housed in the liquid housing part of the liquid storage part 54, but is not limited thereto, and arbitrary liquid can be used.

The amount (volume) of the liquid 261 taken in may be 1% or more with respect to the total volume of the flow channel 51. The amount (volume) of the liquid 261 taken in may be 90% or less, 50% or less, or 20% or less with respect to the total volume of the flow channel 51. The total volume of the flow channel 51 may be the volume of the space sealed between the end part 254 of the nozzle 49 and the plunger of the syringe pump connected to the nozzle 49.

The method of 913 can be performed by installing the nozzle 49, which has taken in the liquid 261 in advance, in the nozzle actuator 40 in advance. When it is necessary to attach the nozzle 49 to the nozzle actuator 40, the processing may start from the step of installing the nozzle in S180 again. Alternatively, the liquid 261 may be taken into the flow channel 51 in the nozzle 49 after the nozzle 49 is attached to the nozzle actuator 40.

The liquid 261 may be taken into the flow channel 51 in the nozzle 49 in a manner similar to the step of S510. For example, the taking-in of the liquid 261 may be performed by the air bubble forming unit 200 controlling the pressure generating unit 47 to cause the suction operation to be performed on the flow channel 51 to take in the liquid 261 from the edge of the flow channel 51. After the liquid 261 is taken into the flow channel 51 in the nozzle 49, a gas may be further taken into the flow channel 51 in the nozzle 49. The gas may be taken in in a manner similar to the step of S436. For example, the air bubble forming unit 200 may control the pressure generating unit 47 and the nozzle actuator 40 to take in the gas in order to form a new air bubble at the end part 254 of the nozzle 49. The air bubble forming unit 200 may send an instruction to the nozzle actuator 40 to take out the nozzle 49 from the liquid 261. After the nozzle actuator 40 takes out the nozzle 49 from the liquid 261, the pressure generating unit 47 may draw the plunger of the syringe pump to take in a necessary amount of gas. The amount of the gas partitioned inside the flow channel 51 can be adjusted by using the amount of air taken in.

Another example in which 910 is specifically performed will be described. As an example, in 914, in 704a, 704b, and 704c of Fig. 13E, a dividing member (for example, a diaphragm 292) is provided in the flow channel 51 in the nozzle 49 to divide the inner space of the flow channel 51, the volume of the gas continuous with the air bubble is decreased, the air bubble is stably maintained, and the control is facilitated. The diaphragm 292 may be movable in the flow channel 51 by controlling the pressure generating unit 47 to cause the flow channel 51 to perform an intake or suction operation. The diaphragm 292 may be movable in the flow channel 51 by being pushed or drawn by an arbitrary distance by a presser 294. The presser 20 may move the diaphragm 292 in the flow channel 51 by pushing or drawing the diaphragm 292 by a preset distance by the nozzle actuator 40 or a presser actuator (not illustrated) on the basis of the control of the air bubble forming unit 200. The diaphragm 292 may be made of metal, plastic, or rubber, but is not limited thereto. In this case, the method of 914 can be performed by installing the nozzle 49, which is provided with the diaphragm 292, in the nozzle actuator 40 in advance. When the nozzle 49 needs to be attached to the nozzle actuator 40, the step of installing the nozzle 49 in S180 may be started again. The step of installing the nozzle 49 may be performed in the same manner as in S180.

The volume (for example, in the case of 704a, the volume of the space below the diaphragm 292) of the space divided by the dividing member in the total volume of the flow channel 51 may be 1% or more. The volume of the space divided by the dividing member in the total volume of the flow channel 51 may be 50% or less, 25% or less, or 10% or less. The total volume of the flow channel 51 may be the volume of the space sealed between the end part 254 of the nozzle 49 and the plunger of the syringe pump connected to the nozzle 49.

The nozzle 49 provided with the diaphragm 292 may be housed in the nozzle housing part of the flow channel exchange part 53 after the diaphragm 292 and the presser 294 are provided in the flow channel 51 in the nozzle 49 in advance. Alternatively, the nozzle 49 not provided with the diaphragm 292 and the presser 294 may be housed in the nozzle housing part, the diaphragm 292 and the presser 294 may be provided in the flow channel 51 in the nozzle 49 by the hand of the manipulator, and then the nozzle 49 may be installed in the nozzle actuator 40 by the hand of the manipulator.

Another example in which 910 is specifically performed will be described. As an example, in 915, in 705 in Fig. 13E, a structure is provided which includes a portion having a large flow channel diameter and a portion having a small flow channel diameter in the flow channel 51 in the nozzle 49. For example, in the flow channel 51, the structure including the portion having a large flow channel diameter and the portion having a small flow channel diameter may be an orifice structure in which a part of the flow channel 51 is narrowed. In addition, in the flow channel 51, one or more portions having a large flow channel diameter and one or more portions having a small flow channel diameter may be provided. In 705 of Fig. 13E, the nozzle 49 includes a portion 297 having a small flow channel diameter, a portion 295 having a large flow channel diameter, and a portion 298 having a large flow channel diameter, but the number of the portions having a small flow channel diameter and the portions having a large flow channel diameter included in the nozzle 49 is not limited thereto.

In this manner, by providing the portion 297 having a small flow channel diameter, when a gas is taken in the air bubble 256 through the flow channel 51, an effect can be obtained in which the volume of the gas present in the flow channel 51 related to the formation of the air bubble can be regarded as only the volume of 295 over time, and an effect can be obtained in which the volume of the gas continuous with the air bubble is decreased, the air bubble stably is stably maintained, and the control can be facilitated.

The flow channel diameter of the portion having a small flow channel diameter may be 1/10 or less, 1/20 or less, or 1/25 or less of the flow channel diameter of the portion having a large flow channel diameter, but is not limited thereto. When the flow channel diameter of the portion having a small flow channel diameter is 1/10 or less of the flow channel diameter of the portion having a large flow channel diameter, it is possible to bring about a sufficient effect for stably maintaining and controlling the air bubble.

In this case, the method of 915 can be performed by installing the nozzle 49, which includes the portion having a large flow channel diameter and the portion having a small flow channel diameter, in the nozzle actuator 40 in advance. When it is necessary to attach the nozzle 49 to the nozzle actuator 40, the processing may start from the step of installing the nozzle in S180 again. The step of installing the nozzle 49 may be performed in the same manner as in S180. The nozzle 49 including the portion having a large flow channel diameter and the portion having a small flow channel diameter may be housed in the nozzle housing part of the flow channel exchange part 53 in advance. When the gas-liquid interface manipulation unit 101 does not include the flow channel exchange part 53, the nozzle 49 including the portion having a large flow channel diameter and the portion having a small flow channel diameter may be attached to the nozzle actuator 40 by the hand of the manipulator.

Next, in 920, the air bubble can also be stabilized by increasing the right side of the above stabilization formula (Mathematical Formula 2). That is, the air bubble may be stably maintained by increasing the change in the Laplace pressure $P_m$ of the air bubble with respect to a minute volume change. For example, the air bubble can be stably maintained by changing the shape of the cross section in the flow channel 51 of the nozzle 49.

Fig. 13F is a graph illustrating a relationship between a dimensionless volume and a dimensionless curvature when an contact angle of a flow channel member forming the end part 254 of the nozzle 49 and the liquid 261 is set to 90 degrees, and on the basis of this, 921 will be described as an example of specifically performing 920. A horizontal axis represents the dimensionless volume of the air bubble, and a vertical axis represents the dimensionless curvature of the air bubble. A curve 298a (dotted line) indicates a case where the shape of the cross section in the flow channel 51 of the nozzle 49 is a square shape, a curve 298b (solid line) indicates a case where the shape of the cross section is a cross shape, and a curve 298c (broken line) indicates a case where the shape of the cross section is a circle shape. All cross-sectional areas of these cross sections are equal, and only the shape of the cross section is different.

The shape of the cross section in the flow channel 51 of the nozzle 49 increases the change in Laplace pressure with respect to the minute volume change of 920, thereby affecting the stable maintenance of the air bubble. Specifically, it is shown that when the cross section has a cross shape, the effect of increasing the change in Laplace pressure is the largest.

In the curve of the graph of Fig. 13F, the dimensionless curvature indicates the curvature of the air bubble, and the inner pressure of the air bubble increases as the curvature increases, and the inner pressure decreases as the curvature decreases. The fact that the dimensionless curvature increases with respect to the dimensionless volume change indicates that the air bubble inner pressure increases with respect to the volume change, and indicates that the Laplace pressure, which is a pressure difference from the liquid phase of the liquid 261, increases. That is, this indicates that the Laplace pressure increases with respect to a minute volume change, and the right side of the above stabilization formula (Mathematical Formula 2) increases. On the other hand, the fact that the dimensionless curvature decreases with respect to the dimensionless volume change indicates that the Laplace pressure decreases with respect to the minute volume change, and the right side of the above stabilization formula (Mathematical Formula 2) becomes smaller. From the above, when the dimensionless curvature turns from increase to decrease, the right side of the stabilization formula (Mathematical Formula 2) changes from plus to minus, so that the air bubble becomes unstable. Therefore, when the value of the dimensionless volume which brings the maximum value of the curve increases, the region of the volume in which the air bubble can be stably maintained and controlled is widened, and the air bubble can be stably maintained and controlled more easily. In the graph of Fig. 13F, compared to the curve 298a in the case of the square-shaped cross section and the curve 298c in the case of the circle-shaped cross section, the curve 298b in the case of the cross-shaped cross section brings the maximum value of the dimensionless curvature at a larger dimensionless volume, and thus it is conceivable that the cross-shaped cross section can contribute to the stability of the air bubble.

Fig. 13F illustrates the case of the cross-shaped cross section, but the shape contributing to stabilization is not limited to the cross shape. For example, when the cross-sectional shape is a shape having a protruding portion inward, that is, a shape having an internal angle, an effect similar to the cross shape can be obtained. For example, the shape having the protruding portion inward may be an asterisk shape, but is not limited thereto.

In addition, in 921, in the end part 254 of the nozzle 49, an angle formed by the inner side surface of the flow channel 51 and the surface on the end part side of the flow channel member forming the flow channel 51 increases the change in Laplace pressure with respect to the minute volume change of 920, thereby affecting the stable maintenance of the air bubble.

Fig. 13G is a graph illustrating a relationship between a Laplace pressure change rate and an air bubble volume when a chamfering angle ($\theta c$ of 298u) of the corner formed by the inner side surface of the flow channel 51 of the nozzle 49 and the surface on the end part side of the flow channel member forming the flow channel 51 is changed. A horizontal axis represents the volume of the air bubble, and a vertical axis represents the Laplace pressure change of the air bubble. A curve 298v (solid line) indicates a case where the chamfering angle is 15 degrees, a curve 298w (dotted line) indicates a case where the chamfering angle is 30 degrees, a curve 298x (broken line) indicates a case where the chamfering angle is 45 degrees, a curve 298y (one-dot chain line) indicates a case where the chamfering angle is 60 degrees, and a curve 298z (two-dot chain line) indicates a case where the chamfering angle is 75 degrees. From the graph, as the chamfering angle increases, the Laplace pressure change decreases with respect to the volume increase accompanying the air bubble formation, particularly in the initial volume increase stage, and thus it is conceivable that it becomes difficult to stably maintain and control the air bubble. When there is no chamfering, the angle formed by the inner side surface of the flow channel 51 and the surface on the end part side of the flow channel member forming the flow channel 51 is 90 degrees, and as illustrated in Fig. 13C, there is a region where the change in Laplace pressure increases with the air bubble formation, and there is a region where the air bubble can be stably maintained and controlled. That is, at the end part 254 of the nozzle 49, the angle formed by the inner side surface of the flow channel 51 and the surface on the end part side of the flow channel member forming the flow channel 51, that is, the angle of the cross section at the end part of the nozzle 49 is in a range of 90 $\pm$ 5 degrees, whereby the air bubble can be stably maintained and controlled.

The above-formed angle may be in a range of 90 $\pm$ 5 degrees, in a range of 90 $\pm$ 3 degrees, or even approximately 90 degrees. When the formed angle is in the vicinity of the right angle, the right side of the stabilization formula (Mathematical Formula 2) can be increased, which can contribute to the stability of the air bubble.

The method of 921 can be performed by previously installing the nozzle 49, which has the cross-sectional shape and/or angle as described above, in the nozzle actuator 40 in advance. When it is necessary to attach the nozzle 49 to the nozzle actuator 40, the processing may start from the step of installing the nozzle in S180 again. The step of installing the nozzle 49 may be performed in the same manner as in S180. The nozzle 49 having the cross-sectional shape and/or angle as described above may be housed in the nozzle housing part of the flow channel exchange part 53 in advance. When the gas-liquid interface manipulation unit 101 does not include the flow channel exchange part 53, the nozzle 49 may be attached to the nozzle actuator 40 by the hand of the manipulator.

The contact angle between the flow channel member forming the flow channel 51 of the nozzle 49 and the liquid increases the change in Laplace pressure with respect to the minute volume change of 920, thereby affecting the stable maintenance of the air bubble. Specifically, when the contact angle between the flow channel member and the liquid is 90 degrees or less, the air bubble can be stabilized.

Fig. 13H is a graph illustrating the relationship between the dimensionless volume and the dimensionless curvature when the contact angle of the flow channel member forming the end part 254 of the flow channel 51 of the nozzle 49 and the liquid 261 is changed, and on the basis of this, 922 will be described as another example of specifically performing 910 . A horizontal axis represents the dimensionless volume of the air bubble, and a vertical axis represents the dimensionless curvature of the air bubble. A curve 299a (solid line) indicates a case where the contact angle between the nozzle 49 and the liquid is 30 degrees, a curve 299b (dotted line) indicates a case where the contact angle is 60 degrees, a curve 299c (broken line) indicates a case where the contact angle is 90 degrees, a curve 299d (one-dot chain line) indicates a case where the contact angle is 120 degrees, and a curve 299e (two-dot chain line) indicates a case where the contact angle is 150 degrees.

Fig. 13F illustrates that, when the contact angle is 120 degrees or 150 degrees exceeding 90 degrees (the curve 299d and the curve 299e, respectively), the value of the dimensionless volume which brings the maximum value is largely shifted to the left side. That is, when the contact angle exceeds 90 degrees, the region where the air bubble is stably maintained becomes narrow, and thus it is conceivable that it becomes difficult to stably maintain and control the air bubble. Therefore, in order to contribute to the stable maintenance and control of the air bubble, the contact angle between the flow channel member forming the end part 254 of the flow channel 51 of the nozzle 49 and the liquid 261 is preferably 90 degrees or less. The contact angle between the flow channel member forming the end part 254 of the flow channel 51 of the nozzle 49 and the liquid 261 is preferably 60 degrees or less.

The contact angle between the flow channel member and the liquid 261 can be measured by a known method. For example, the contact angle may be measured by a droplet method. The droplet method may be a method in which the droplets of the liquid are brought into contact with the flow channel member to be deposited, and a static contact angle between the liquid and the flow channel member at that time is measured by using a contact angle meter. As the contact angle meter, a commercially available one can be used.

In this case, the method of 922 can be performed by installing the nozzle 49, which has having the above contact angle, in the nozzle actuator 40 in advance. When it is necessary to attach the nozzle 49 to the nozzle actuator 40, the processing may start from the step of installing the nozzle in S180 again. The step of installing the nozzle 49 may be performed in the same manner as in S180. The nozzle 49 having the above contact angle may be housed in the nozzle housing part of the flow channel exchange part 53 in advance. When the gas-liquid interface manipulation unit 101 does not include the flow channel exchange part 53, the nozzle 49 having the above contact angle may be attached to the nozzle actuator 40 by the hand of the manipulator.

Furthermore, another example in which 920 is specifically performed will be described. As an example, in 923, a stepped portion 307 is provided in the flow channel 51 in the nozzle 49, which can also contribute to the stabilization of the air bubble.

Fig. 13I is a view in which in the flow channel 51 in the nozzle 49, the stepped portion 307 is provided at a connection portion between an edge portion 305 provided on the end part side of the flow channel 51 and an inner portion 306 connected to the edge portion 305. The stepped portion may be a structure in which the inner diameter of the flow channel 51 changes in the axial direction of the cylindrical portion 253 of the nozzle 49. In addition, the stepped portion may have a structure in which inside the flow channel 51, a portion having a narrow flow channel diameter enters a portion having a wide flow channel diameter. For example, the edge portion 305 and the inner portion 306 may have different flow channel diameters. For example, the flow channel diameter of the edge portion 305 may be smaller than the flow channel diameter of the inner portion 306. As an example, the flow channel diameter of the edge portion 305 may be 1/10 or less, 1/20 or less, or 1/25 or less of the flow channel diameter of the inner portion 306, but is not limited thereto.

Examples of the stepped portion include a case where the portion having a wide flow channel diameter and the portion having a narrow flow channel diameter are continuously connected as illustrated in 710a, and a case where the portion having a narrow flow channel diameter enters the portion having a wide flow channel diameter and is connected as illustrated in 710b. In the case of 710b, the amount of a very small amount of liquid can be measured by adjusting the length of the portion having a narrow flow channel diameter entering the portion having a wide flow channel diameter.

At this time, the gas-liquid interface 255 is formed at the stepped portion 307 of the connection portion by taking the liquid 261 into the flow channel 51. In this manner, by providing the stepped portion 307 in the flow channel 51, the above-described stabilization formula (Mathematical Formula 2) is satisfied in the formed gas-liquid interface 255. That is, the gas-liquid interface 255 can be easily maintained and controlled at the position of the stepped portion 307. By using this, in the edge portion 305 having a small flow channel diameter, it is possible to measure a very small amount of liquid of only the edge portion.

In this case, the method of 923 can be performed by installing the nozzle 49, which is provided with the above stepped portion 307, in the nozzle actuator 40 in advance. When it is necessary to attach the nozzle 49 to the nozzle actuator 40, the processing may start from the step of installing the nozzle in S180 again. The step of installing the nozzle 49 may be performed in the same manner as in S180. The nozzle 49 provided with the above stepped portion 307 may be housed in the nozzle housing part of the flow channel exchange part 53 in advance. When the gas-liquid interface manipulation unit 101 does not include the flow channel exchange part 53, the nozzle 49 provided with the stepped portion 307 may be attached to the nozzle actuator 40 by the hand of the manipulator.

Fig. 13J illustrates the nozzle 49 provided with the stepped portion 307 in the flow channel 51. In the nozzle 49 illustrated in Fig. 13H, the flow channel diameter (diameter) of the edge portion 305 is 0.1 mm, the height of the edge portion 305 is 1 mm, the flow channel diameter (radius) of the inner portion 306 is 1.0 mm, and the total length of the nozzle 49 is 20 mm. In the nozzle 49, the stable gas-liquid interface 255 can be maintained at the stepped portion 307, and the liquid amount (about 7.5 nL) of only the edge portion can be measured.

Furthermore, in 930, the air bubble can also be stabilized by decreasing the surface tension (the interface free energy at the gas-liquid interface 255) of the liquid with the gas. When the surface tension is decreased, the air bubble easily expand with a small inner pressure change, so that the change in the inner pressure of the air bubble with respect to a minute volume change becomes smaller. That is, since the left side of the stabilization formula (Mathematical Formula 2) decreases, it is possible to more stably maintain and control the air bubble.

An example in which 930 is specifically performed will be described. As an example, in 931, the surface tension of the liquid with the gas can be decreased by adding, to the liquid, a solute for decreasing the surface tension of the liquid. For example, the solute may be a polar organic compound or a surfactant. The polar organic compound may be an organic compound having a highly polar functional group such as an amino group, a carboxyl group, or a hydroxyl group. For example, the polar organic compound may be an alcohol, a fatty acid, an amino acid, a peptide, a protein, a saccharide, or the like. Since the polar organic compound has a hydrophobic functional group and a hydrophilic functional group, the polar organic compound interacts with molecules of the gas and the liquid 261 forming the gas-liquid interface 255 and is attached to the gas-liquid interface 255. That is, by adding the polar organic compound to the solution, the gas-liquid interface 255 is covered with the molecules of the polar organic compound, and as a result, water molecules on the surface in contact with the gas phase are decreased, and the surface tension is decreased.

The surfactant may be an amphiphilic compound having both a hydrophobic portion and a hydrophilic portion in the molecule. For example, the surfactant may be a cationic surfactant (such as an inverted soap), an anionic surfactant (such as sodium fatty acid), an amphoteric surfactant (such as a betaine-based surfactant), or a nonionic surfactant (such as an octyl glycoside). Similarly to the polar organic compound, the surfactant also interact with the molecules of the gas and the liquid 261 forming the gas-liquid interface 255, but the surface tension is decreased at a much lower concentration extremely compared to the case of the polar organic compound. Therefore, by adding the surfactant to the liquid, the surface tension is significantly decreased. In such a step of adding a solute, for example, a solution containing a solute may be added by performing a manipulation similar to the step of S620.

Another example in which 930 is specifically performed will be described. As an example, in 932, the surface tension of the liquid with the gas can be decreased by removing, from the liquid, a solute for increasing the surface tension of the liquid. For example, the solute may be an inorganic salt. The inorganic salt may be a compound which ionizes into cations and anions in a liquid such as an aqueous solution. For example, the inorganic salt may be a metal salt such as sodium chloride, potassium chloride, phosphate, or alum. The inorganic salt is ionized in solution to become cations and anions, and these ions are surrounded by water molecules and hydrated to be stabilized. The water molecules are more stabilized when interacting with cations and anions having a Coulomb force than when interacting with other water molecules to form hydrogen bonds. Therefore, the cations and anions derived from the inorganic salt cannot be attached to the gas-liquid interface 255. As a result, in the inside of the air bubble, an interaction due to a stable and stronger Coulomb force occurs between the water molecules and the cations and anions derived from inorganic salts. On the other hand, since the gas-liquid interface 255 with the air bubble is in a state closer to pure water only by hydrogen bonding, an energy difference occurs between the inside of the liquid and the liquid on the surface of the gas-liquid interface 255, and as a result, the interface free energy of the gas-liquid interface 255 increases. That is, the surface tension is increased by adding the inorganic salt to the liquid. Therefore, the surface tension can be decreased by removing the inorganic salt from the liquid. The inorganic salt may be removed by adding a chelating agent such as EDTA to the liquid, performing column processing such as ion exchange resin on the liquid, or the like.

Another example in which 930 is specifically performed will be described. As an example, in 933, a liquid with a relatively smaller surface tension may be added to the liquid to decrease the surface tension of the liquid with the gas. Alternatively, by replacing the liquid with a liquid having a relatively smaller surface tension, the surface tension of the liquid with the gas may be decreased. For example, a liquid having a high concentration of amphipathic substances such as a polar organic compound or a surfactant in the liquid is added to the liquid with which the vessel 25 is filled, or the liquid is replaced with a liquid having a high concentration of amphipathic substances, whereby the surface tension may be decreased, and the air bubble may be stably maintained and controlled. For example, a liquid having a low concentration of inorganic salts in the liquid is added to the liquid with which the vessel 25 is filled, or the liquid is replaced with a liquid having a low concentration of inorganic salts, whereby the surface tension may be decreased, and the air bubble may be stably maintained and controlled.

Such a step of adding or replacing the liquid can be performed similarly to the step and the sub-step of S600. For example, with respect to the basic medium or buffer solution with which the vessel 25 is filled, all or at least some of the liquid may be replaced with a complete medium, or an appropriate amount of complete medium may be added to the liquid. By replacing or adding in this way, the surface tension can be decreased, and the air bubble can be stabilized. The basic medium may contain only a small portion of proteins or amino acids. As an example, the basic medium is DMEM (Dulbecco's Modified Eagle's Medium) or Ham's F-12 (Ham's F-12 Medium). The complete medium may be obtained by adding, a basic medium, a protein such as serum or a cell proliferation factor or an amino acid such as L-glutamine. The buffer solution may be a solution adjusted to a salt concentration, pH, or osmotic pressure suitable for the cell. As an example, the buffer solution may be PBS (phosphate buffered saline), HANKS buffer solution, or HEPES (hydroxyethyl piperazine ethanesulfonic acid) buffer solution.

As described with reference to Fig. 13B, when $\theta_m$ exceeds 90 degrees, the air bubble can become unstable, but even when $\theta_m$ exceeds 90 degrees, the air bubble can be stably maintained under specific conditions. At that time, the maximum volume of the gas (the gas continuous with the air bubble) related to the air bubble formation in which the air bubble is stably maintained is defined as $V_0$. By setting the volume of the gas continuous with the air bubble to $V_0$ or less, the air bubble can be stably maintained. Since the formula representing $V_0$ is complicated, the following was confirmed when an experiment was conducted by setting numerical values of parameters on the basis of actual use conditions.

Fig. 13K illustrates a behavior in which the above experiment is performed. In 720a, in the flow channel 51 of the nozzle 49, a minute gas space was formed with a liquid interposed therebetween. At this time, water was used as the liquid, the contact angle between the nozzle 49 and the liquid was 90 degrees, and air was used as the gas. The radius of the flow channel 51 of the nozzle 49 was 50 $\mu$m, and the maximum volume $V_0$, at which the air bubble was stably maintained, of the gas continuous with the air bubble was calculated to be 1.17 $\mu$L. In 720a, the volume of the minute gas space formed in the flow channel 51 of the nozzle 49 is about 0.9 $\mu$L, and the air bubble is stably maintained even when $\theta_m$ exceeds 90 degrees. Such a minute gas space was formed as follows. First, the edge part of the flow channel 51 of the nozzle 49 was put in water, a suction operation was performed by the syringe pump, and about 3.5 $\mu$L of water was taken into the flow channel 51. Subsequently, the nozzle 49 was taken from the water, the suction operation was performed by the syringe pump, and about 0.9 $\mu$L of air was taken into the flow channel 51. In this way, the amount by which the gas inside the flow channel 51 is partitioned can be adjusted. Such a minute gas space was formed, and the stability of the air bubble formation was confirmed as follows. The edge part of the flow channel 51 of the nozzle 49 having a minute gas space formed therein was put in water, and pressurization was started by using the syringe pump from the state of 720b (720c). At this time, although $\theta_m$ already exceeds 90 degrees, the air bubble was stably maintained, and the control was easy. Even when the pressurization was continued (720d) and the air bubble was further enlarged, the air bubble was stably maintained, and the control was easy. Thereafter, the pressurization was stopped, and the gas suction was started (720e). The air bubble was stably maintained even in the process of continuing the gas suction (720f) and gradually reducing the air bubble, and the control was easy. The gas suction was completed (720g), and the air bubble was taken into the flow channel 51 of the nozzle 49.

First, when the contact angle between the flow channel member forming the end part 254 of the flow channel 51 of the nozzle 49 and the liquid 261 is 90 degrees, $V_0$ is maximized. In addition, $V_0$ increases as the specific heat ratio $\kappa$ of the gas increases. For example, the value of the specific heat ratio K $\kappa$ is 5/3 for a monatomic molecule such as helium, 7/5 for a diatomic molecule such as nitrogen, and 8/6 for a polyatomic molecule such as carbon dioxide. Furthermore, $V_0$ increases as the surface tension $\gamma$ decreases. For example, the value of the surface tension $\gamma$ at an air temperature of 20°C is 72.8 mN/m for water, about 50 mN/m for a culture solution containing a protein, and about 5 to 15 mN/m for an aqueous solution containing a surfactant.

In this regard, a case was examined in which the contact angle between the nozzle 49 and the liquid is 90 degrees, and the gas is a monatomic molecule (specific heat ratio $\kappa$ is 5/3) or air (since nitrogen and oxygen mainly constituting air are diatomic molecules, air is considered as a diatomic molecule, and a specific heat ratio $\kappa$ is 7/5). At this time, a relationship between $V_o$ and the radius $r_h$ of the end part 254 of the flow channel 51 is indicated by a straight line when $V_o$ and $r_h$ are plotted on a double logarithmic graph.

Fig. 13L illustrates a double logarithmic graph when $r_h$ is taken on a horizontal axis and $V_o$ is taken on a vertical axis. The conditions under which the air bubble can be stably maintained and controlled can be defined by $V_o$. As illustrated in Fig. 13L, $V_o$ and $r_h$ have a relationship indicated by a straight line, and thus the inclination of the straight line can be calculated. By calculating the inclination of the straight line, $V_o$ and $r_h$ can be approximated by the following relational expression.

[Mathematical Formula 7]

$$V_o = a \times r_h{}^4 + b \times r_h{}^3$$

a and b are coefficients. Further, in the graph of Fig. 13L, the volume of the air bubble is equal to or less than $V_o$ in the region below the straight line, so that the air bubble can be stably maintained and controlled.

For example, in Fig. 13L, when the contact angle between the end part 254 of the nozzle 49 and the liquid 261 is 90 degrees, the gas is air (diatomic molecule, the value of the specific heat ratio $\kappa$ is 7/5), and the liquid is water (at this time, the value of the surface tension $\gamma$ is 72.8 mN/m at an air temperature of 20°C), a = $1.82 \times 10^8$, and b = $2.59 \times 10^2$.

Therefore, the volume V (m³) of the gas occupied by the gas continuous from the gas-liquid interface 255 to the inside of the flow channel 51 may satisfy the following formula with respect to the radius $r_h$ (m) of the end part 254 of the flow channel 51.

[Mathematical Formula 8]

$$V \leq a \times r_h{}^4 + b \times r_h{}^3$$

where a = $1.82 \times 10^8$ and b = $2.59 \times 10^2$.

When V satisfies the above Mathematical Formula 8 with respect to $r_h$, the air bubble is stably maintained and controlled.

For example, in Fig. 13L, in a case where the contact angle between the end part 254 of the nozzle 49 and the liquid 261 is 90 degrees, the gas is a monatomic molecule, and the liquid is an aqueous solution (at this time, the value of the surface tension $\gamma$ is 5 mN/m at an air temperature of 20°C) containing a surfactant, when the coefficients a and b are calculated, a = $3.21 \times 10^9$ and b = $3.15 \times 10^2$.

In addition, in Fig. 13L, when the contact angle between the end part 254 of the nozzle 49 and the liquid 261 is 90 degrees, the gas is air, and the liquid is a culture solution (at this time, the value of the surface tension $\gamma$ is 50 mN/m at an air temperature of 20°C) containing a protein, a = $2.65 \times 10^8$, and b = $2.59 \times 10^2$. That is, when these values are substituted into the coefficients a and b, the air bubble is stably maintained and controlled within a range satisfying Mathematical Formula 8. Note that in the case of the example illustrated in Fig. 13K, since the contact angle between the end part 254 of the nozzle 49 and the liquid 261 is 90 degrees, the gas is air, the liquid is water, and $r_h$ is 50 $\mu$m, $V_o$ is determined to be 1.17 $\mu$L. In Fig. 13K, since the volume of the gas is about 0.9 $\mu$L, it could be confirmed experimentally that the air bubble is stably maintained and controlled. Note that although the above-described relational expression shows an example in which the contact angle is 90 degrees, the contact angle is not limited to 90 degrees. For example, by setting a predetermined coefficient, the above-described relational expression is satisfied regardless of whether the contact angle is 80 degrees or 120 degrees.

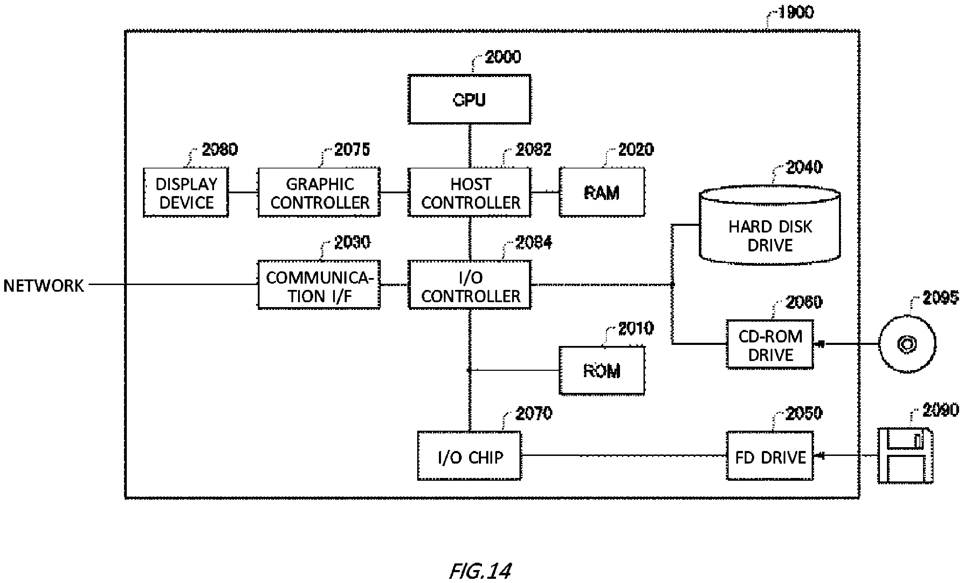

Fig. 14 illustrates an example of a hardware configuration of a computer 1900 which functions as the information processing device 170. The computer 1900 according to the present embodiment includes: a CPU-peripheral portion having a CPU 2000, a RAM 2020, a graphics controller 2075, and a display device 2080 interconnected by a host controller 2082; and an input/output unit having communication interface 2030, a hard disk drive 2040, and a CD-ROM drive 2060 connected to the host controller 2082 by an input/output controller 2084; and a legacy input/output unit having a ROM 2010, a flexible disk drive 2050, and an input/output chip 2070 connected to the input/output controller 2084.

The host controller 2082 connects the RAM 2020 with the CPU 2000 and the graphic controller 2075 accessing the RAM 2020 at a high transfer rate. The CPU 2000 operates on the basis of programs stored in the ROM 2010 and the RAM 2020, and controls each unit. The graphics controller 2075 is configured to acquire image data generated by the CPU 2000 or the like on a frame buffer provided inside the RAM 2020 and display the image data on the display device 2080. Alternatively, the graphics controller 2075 may include therein a frame buffer storing the image data generated by the CPU 2000 or the like. The display device 2080 can display various types of information (for example, an image, position information of the manipulation target 35, and the like) generated inside the information processing device 170.

The input/output controller 2084 connects the communication interface 2030, the hard disk drive 2040, and the CD-ROM drive 2060 which are relatively fast input/output devices to the host controller 2082. The communication interface 2030 is configured to communicate with other devices via a network by wire or wirelessly. In addition, the communication interface is configured to function as a hardware to perform communications. The hard disk drive 2040 stores a program and data to be used by the CPU 2000 in the computer 1900. The CD-ROM drive 2060 is configured to read a program or data from the CD-ROM 2095 and provide the hard disk drive 2040 through the RAM 2020.

In addition, the ROM 2010, and the flexible disk drive 2050 and input/output chip 2070, which are relatively low-speed input/output devices, are connected to the input/output controller 2084. The ROM 2010 stores a boot program performed when the computer 1900 starts up, and/or a program relying on the hardware of the computer 1900, and the like. The flexible disk drive 2050 is configured to read out a program or data from the flexible disk 2090, and provide it to the hard disk drive 2040 via the RAM 2020. The input/output chip 2070 connects the flexible disk drive 2050 to the input/output controller 2084, and connects various types of input/output devices to the input/output controller 2084, for example, via a parallel port, a serial port, a keyboard port, a mouse port, or the like.

The program provided to the hard disk drive 2040 via the RAM 2020 is stored in a recording medium, such as the flexible disk 2090, the CD-ROM 2095, or an IC card, and provided by a user. The program is read out from the recording medium, installed on the hard disk drive 2040 in the computer 1900 via the RAM 2020, and executed in the CPU 2000.

A program installed in the computer 1900 and causing the computer 1900 to function as the information processing device 170 includes an air bubble forming module, an energy control module, and a manipulation module. These programs or modules may work on the CPU 2000 or the like to cause the computer 1900 to function as the air bubble forming unit 200, the liquid control unit 260, or the like.

The information processing described in these programs is read by the computer 1900 to cause the computer to function as the air bubble forming unit 200, the liquid control unit 260, or the like which is a specific means realized by cooperation of software and the various types of hardware resources described above. These specific means implement operations or processing of information according to the intended use of the computer 1900 in the present embodiment, and the information processing device 170 is thereby constructed to be specific for the intended use.

As an example, when communication is performed between the computer 1900 and an external device or the like, the CPU 2000 is configured to execute the communication program loaded on the RAM 2020, and provide the communication interface 2030 with communication processing instructions on the basis of the content of the process written in the communication program. In response to the control by the CPU 2000, the communication interface 2030 is configured to read out the transmission data stored in the transmission buffer region or the like provided on the storage device, such as the RAM 2020, the hard disk drive 2040, the flexible disk 2090, the CD-ROM 2095, or the like, and transmit this transmission data to the network, and write reception data received from the network onto a reception buffer region or the like provided on the storage device. In this way, the communication interface 2030 may transfer transmission/reception data to the storage device through DMA (Direct Memory Access) scheme, and alternatively, the CPU 2000 may transfer the transmission/reception data by reading the data from the storage device or communication interface 2030 that are the origins of transfer, and writing the data onto the communication interface 2030 or the storage device that are the destinations of transfer.

In addition, the CPU 2000 causes all or necessary portions of files or database stored in an external storage device such as the hard disk drive 2040, the CD-ROM drive 2060 (CD-ROM 2095), and the flexible disk drive 2050 (flexible disk 2090) to be read into the RAM 2020 by means of DMA transfer or the like, and then performs various types of processing on the data in the RAM 2020. The CPU 2000 writes back the data on which processing is completed into an external storage device by DMA transfer or the like. In such processing, the RAM 2020 can be regarded as carrying contents of the external storage device temporarily, and thus the RAM 2020, the external storage device and the like are collectively called a memory, a recording unit, a storage device or the like in the present embodiment.

The storage device or the like stores information necessary for information processing of the information processing device 170, for example, moving image data or the like as necessary, and supplies the information to each component of the information processing device 170 as necessary.

Various types of information such as various types of programs, data, tables, databases or the like in the present embodiment according to the present embodiment are stored on such a storage device, and are subjected to information processing. Note that, the CPU 2000 can retain a part of the RAM 2020 in a cache memory and read from or write to the cache memory. In such a configuration as well, the cache memory serves a part of the function of the RAM 2020, and therefore the cache memory is also included with the RAM 2020, the memory, and/or the storage device in the present embodiment, except when it is shown with distinction.

In addition, the CPU 2000 is configured to execute various types of processing including various types of computations, information processing, conditional determination, information search/replacement, or the like described in the present embodiment for the data read from the RAM 2020, as specified by the instruction sequence of the program, and writes the result back onto the RAM 2020. For example, when performing conditional determination, the CPU 2000 compares various types of variables shown in the present embodiment to determine whether they satisfy conditions such as being larger than, smaller than, equal to or greater than, less than or equal to, equal to or like other variables or constants, and if a condition is satisfied (or if it is not satisfied) branches to a different instruction sequence or calls up a subroutine.

In addition, the CPU 2000 can search for information stored in a file in the storage device or the database, or the like. For example, if a plurality of entries, each having an attribute value of a second attribute associated with an attribute value of a first attribute, are stored in a storage device, the CPU 2000 searches, from among the plurality of entries stored in the storage device, an entry having an attribute value of the first attribute that matches a specified condition, and reads out the attribute value of the second attribute stored in the entry, and it is thereby possible to obtain the attribute value of the second attribute associated with the first attribute that satisfies a predetermined condition.

The programs or modules shown above may also be stored in an external recording medium. As a recording medium, other than the flexible disk 2090 and the CD-ROM 2095, an optical recording medium such as DVD or CD, a magneto-optical recording medium such as MO, a tape medium, a semiconductor memory, such as IC card, or the like can be used. Also, a storage device such as a hard disk or RAM that is provided with a server system connected to the Internet or a specialized communication network may be used as the recording medium to provide the programs to the computer 1900 via the network.

In the present disclosure, a configuration in which the information processing device 170 includes the CPU 2000 as a processor has been described, but the type of the processor is not particularly limited. For example, a GPU, an ASIA, an FPGA, or the like can be appropriately used as the processor. In addition, in the present disclosure, a configuration in which the information processing device 170 includes the hard disk drive 2040 as an auxiliary storage device has been described, but the type of the auxiliary storage device is not particularly limited. For example, instead of the hard disk drive 2040 or in addition to the hard disk drive 2040, another storage device such as a solid state drive may be used.

While the present invention has been described with the embodiments, the technical scope of the present invention is not limited to the above-described embodiments. It is apparent to persons skilled in the art that various alterations and improvements can be added to the above-described embodiments. It is also apparent from the scope of the claims that the embodiments added with such alterations or improvements can be included in the technical scope of the invention.

The operations, procedures, steps, and stages of each process performed by a device, system, program, and method shown in the claims, embodiments, or diagrams can be performed in arbitrary order as long as the order is not indicated by "prior to," "before," or the like and as long as the output from a previous process is not used in a later process. Even if the operation flow is described by using phrases such as "first" or "next" in the scope of the claims, specification, or drawings, it does not necessarily mean that the process must be performed in this order.

(Supplement)

(Item 1) A manipulation method of an organism including:

forming a gas-liquid interface, in which a restoring force acts against minute interface movement, in a flow channel or at an end part of the flow channel, with the end part immersed in a liquid in which an organism is immersed; and

manipulating the organism by using the gas-liquid interface.

(Item 2) The manipulation method according to item 1, wherein
the forming the gas-liquid interface includes releasing or sucking a gas from the end part.

(Item 3) The manipulation method according to item 1, wherein
the forming the gas-liquid interface includes releasing a gas from the end part and maintaining the gas-liquid interface, in which the restoring force acts against the minute volume change, at the end part.

(Item 4) The manipulation method according to item 2 or 3, wherein

in the forming the gas-liquid interface, a gas volume V ($m^3$) occupied by the gas continuous from the gas-liquid interface to an inside of the flow channel satisfies a following formula with respect to a radius $r_h$ (m) of the end part of the flow channel:

$$V \leq a \times r_h^4 + b \times r_h^3$$

(where a = $2.65 \times 10^8$ and b = $2.59 \times 10^2$ are satisfied).

(Item 5) The manipulation method according to any one of items 2 to 4, further including
decreasing a gas volume occupied by the gas continuous from the end part to an inside of the flow channel before the forming the gas-liquid interface.

(Item 6) The manipulation method according to item 5, wherein
the decreasing the gas volume includes taking the liquid into the flow channel and partitioning the gas inside the flow channel with the taken liquid.

(Item 7) The manipulation method according to item 5, wherein
the decreasing the gas volume includes filling the flow channel with a filler which blocks a partial inner space of the flow channel.

(Item 8) The manipulation method according to item 5, wherein

the flow channel includes a nozzle and a space formed inside a pump connected to the nozzle, and

the decreasing the gas volume includes reducing a capacity of the pump to 10% or less of a maximum capacity.

(Item 9) The manipulation method according to item 5, wherein
the flow channel is provided with a dividing member which divides an inner space of the flow channel.

(Item 10)The manipulation method according to any one of items 1 to 9, wherein
a cross-sectional shape of the end part of the flow channel is a shape having a protruding portion inward.

(Item 11)The manipulation method according to any one of items 1 to 10, wherein
at the end part of the flow channel, a corner is formed by an inner side surface of the flow channel and a surface on an end part side of a flow channel member forming the flow channel, and an angle formed by the corner is in a range of 90 ± 5 degrees.

(Item 12)The manipulation method according to any one of items 1 to 11, wherein
a contact angle between a flow channel member forming the flow channel and the liquid by a droplet method is 90 degrees or less.

(Item 13)The manipulation method according to any one of items 1 to 12, wherein
the flow channel includes:

a edge portion provided on an end part side; and

an inner portion which is connected to the edge portion and has a flow channel diameter different from a flow channel diameter of the edge portion, and

the flow channel forms a stepped portion at a connection portion between the edge portion and the inner portion.

(Item 14)The manipulation method according to any one of items 1 to 12, wherein

the flow channel includes

a portion having a large flow channel diameter and a portion having a small flow channel diameter, and

a flow channel diameter of the portion having a small flow channel diameter is 1/10 or less of a flow channel diameter of the portion having a large flow channel diameter.

(Item 15)The manipulation method according to any one of items 2 to 9 and claims 10 to 14 indirectly dependent on claim 2 or 3, further including
decreasing a surface tension of the liquid with the gas before the forming the gas-liquid interface.

(Item 16)The manipulation method according to any one of items 1 to 15, further including

before the forming the gas-liquid interface,

moving relative position of the flow channel and the organism closer to each other.

(Item 17)An organism manipulation device for manipulating an organism, including:

a flow channel having an end part immersed in a liquid in which the organism is immersed; and

a gas-liquid interface manipulation unit which forms a gas-liquid interface in which a restoring force acts against minute interface movement in the flow channel or at the end part and manipulates the organism by the gas-liquid interface.

(Item 18)The organism manipulation device according to item 17, wherein
the gas-liquid interface manipulation unit releases a gas into the liquid from the end part and maintains the gas-liquid interface, in which a restoring force acts against a minute volume change, at the end part.

(Item 19)The organism manipulation device according to item 18, wherein
a gas volume V ($m^3$) occupied by the gas continuous from the gas-liquid interface to an inside of the flow channel satisfies a following formula with respect to a radius $r_h$ (m) of the end part of the flow channel:

$$V \leq a \times r_h^4 + b \times r_h^3,$$

(where a = $2.65 \times 10^8$ and b = $2.59 \times 10^2$ are satisfied).

(Item 20)The organism manipulation device according to any one of items 17 to 19, wherein
the flow channel is filled with a filler which blocks a partial inner space of the flow channel.

(Item 21)The organism manipulation device according to any one of items 17 to 20, wherein
the flow channel is provided with a dividing member which divides an inner space of the flow channel.

(Item 22)The organism manipulation device according to any one of items 17 to 21, wherein
a cross-sectional shape of the end part of the flow channel is a shape having a protruding portion inward.

(Item 23)The organism manipulation device according to any one of items 17 to 22, wherein
at the end part of the flow channel, a corner is formed by an inner side surface of the flow channel and a surface on an end part side of a flow channel member forming the flow channel, and an angle formed by the corner is in a range of 90 ± 5 degrees.

(Item 24)The organism manipulation device according to any one of items 17 to 23, wherein
a contact angle between a flow channel member forming the flow channel and the liquid by a droplet method is 90 degrees or less.

(Item 25)The organism manipulation device according to any one of items 17 to 24, wherein the flow channel includes:

a edge portion provided on an end part side; and

an inner portion which is connected to the edge portion and has a flow channel diameter different from a flow channel diameter of the edge portion, and

the flow channel forms a stepped portion at a connection portion between the edge portion and the inner portion.

(Item 26)The organism manipulation device according to any one of items 17 to 25, wherein the flow channel includes:

a portion having a large flow channel diameter and a portion having a small flow channel diameter, and

a flow channel diameter of the portion having a small flow channel diameter is 1/10 or less of a flow channel diameter of the portion having a large flow channel diameter.

EXPLANATION OF REFERENCES

1: fluorescence image observation light source; 2: dichroic mirror; 3: optical deflector; 4: relay lens; 5: dichroic mirror; 6: objective lens; 7: condenser lens; 8: condensing lens; 9: band pass filter; 10: transmission image observation light source; 11: barrier filter; 12: projection lens; 13: barrier filter; 14: projection lens; 15: pinhole; 16: light source; 17: light source; 25: vessel; 31: Nomarski prism; 32: analyzer (polarizing plate); 35: manipulation target; 37: polarizer (polarizing plate); 38: Nomarski prism; 39: ring aperture; 40: nozzle actuator; 41: sample actuator; 42: flow channel imaging camera; 45: light source; 46: light source; 47: pressure generating unit; 48: sensor unit; 49: nozzle; 50: microscope unit; 51: flow channel; 51a: first flow channel; 51b: second flow channel; 53: flow channel exchange part; 54: liquid storage part; 58: sample lid; 59: sample lid housing part; 60: camera; 70: camera; 100: organism manipulation device; 101: gas-liquid interface manipulation unit; 111: display region; 112: display region; 113: display region; 114: display region; 115: display region; 160: output unit; 170: information processing device; 171: imaging control unit; 180: input unit; 190: recording unit; 200: air bubble forming unit; 250: flow channel control unit; 251: pump; 251a: first pump; 251b: second pump; 253: cylindrical portion; 253a: outer cylinder; 253b: inner cylinder; 254: end part; 255: gas-liquid interface; 256: air bubble; 260: liquid control unit; 261: liquid; 291: bead; 292: diaphragm; 294: presser; 295: portion having large flow channel diameter; 297: portion having small flow channel diameter; 298: portion having large flow channel diameter; 300: image processing unit; 305: edge portion; 306: inner portion; 307: stepped portion; 1900: computer; 2000: CPU; 2010: ROM; 2020: RAM; 2030: communication interface; 2040: hard disk drive; 2050: flexible disk drive; 2060: CD-ROM drive; 2070: input/output chip; 2075: graphics controller; 2080: display device; 2082: host controller; 2084: input/output controller; 2090: flexible disk; and 2095: CD-ROM.

**Claims**

1. A manipulation method of an organism comprising:

   forming a gas-liquid interface, in which a restoring force acts against minute interface movement, in a flow channel or at an end part of the flow channel, with the end part immersed in a liquid in which an organism is immersed; and
   manipulating the organism by using the gas-liquid interface.

2. The manipulation method according to claim 1, wherein
   the forming the gas-liquid interface includes releasing or sucking a gas from the end part.

3. The manipulation method according to claim 1, wherein
   the forming the gas-liquid interface includes releasing a gas from the end part and maintaining the gas-liquid interface, in which the restoring force acts against the minute volume change, at the end part.

4. The manipulation method according to claim 2 or 3, wherein

in the forming the gas-liquid interface, a gas volume V ($m^3$) occupied by the gas continuous from the gas-liquid interface to an inside of the flow channel satisfies a following formula with respect to a radius $r_h$ (m) of the end part of the flow channel:

$$V \leq a \times r_h^4 + b \times r_h^3$$

(where a = $2.65 \times 10^8$ and b = $2.59 \times 10^2$ are satisfied).

5. The manipulation method according to any one of claims 2 to 4, further comprising
decreasing a gas volume occupied by the gas continuous from the end part to an inside of the flow channel before the forming the gas-liquid interface.

6. The manipulation method according to claim 5, wherein
the decreasing the gas volume includes taking the liquid into the flow channel and partitioning the gas inside the flow channel with the taken liquid.

7. The manipulation method according to claim 5, wherein
the decreasing the gas volume includes filling the flow channel with a filler which blocks a partial inner space of the flow channel.

8. The manipulation method according to claim 5, wherein

the flow channel includes a nozzle and a space formed inside a pump connected to the nozzle, and
the decreasing the gas volume includes reducing a capacity of the pump to 10% or less of a maximum capacity.

9. The manipulation method according to claim 5, wherein
the flow channel is provided with a dividing member which divides an inner space of the flow channel.

10. The manipulation method according to any one of claims 1 to 9, wherein
a cross-sectional shape of the end part of the flow channel is a shape having a protruding portion inward.

11. The manipulation method according to any one of claims 1 to 10, wherein
at the end part of the flow channel, a corner is formed by an inner side surface of the flow channel and a surface on an end part side of a flow channel member forming the flow channel, and an angle formed by the corner is in a range of 90 $\pm$ 5 degrees.

12. The manipulation method according to any one of claims 1 to 11, wherein
a contact angle between a flow channel member forming the flow channel and the liquid by a droplet method is 90 degrees or less.

13. The manipulation method according to any one of claims 1 to 12, wherein
the flow channel includes:

a edge portion which is provided on an end part side; and
an inner portion which is connected to the edge portion and has a flow channel diameter different from a flow channel diameter of the edge portion, and
the flow channel forms a stepped portion at a connection portion between the edge portion and the inner portion.

14. The manipulation method according to any one of claims 1 to 12, wherein

the flow channel includes
a portion having a large flow channel diameter and a portion having a small flow channel diameter, and
a flow channel diameter of the portion having a small flow channel diameter is 1/10 or less of a flow channel diameter of the portion having a large flow channel diameter.

**15.** The manipulation method according to any one of claims 2 to 9 and claims 10 to 14 indirectly dependent on claim 2 or 3, further comprising
decreasing a surface tension of the liquid with the gas before the forming the gas-liquid interface.

**16.** The manipulation method according to any one of claims 1 to 15, further comprising

detecting a first pressure applied to the gas-liquid interface, wherein
in the detecting, a change in the first pressure when a gas is continuously released into the flow channel is detected, and a second pressure which allows formation of the gas-liquid interface is decided on a basis of a value of the first pressure that has been changed.

**17.** The manipulation method according to any one of claims 1 to 16, further comprising

detecting a first pressure applied to the gas-liquid interface, wherein
on a basis of a change in the first pressure, the end part of the flow channel having come into contact with the liquid is detected, or the gas-liquid interface formed at the end part of the flow channel having come into contact with a bottom portion of a vessel for culturing the organism is detected.

**18.** The manipulation method according to any one of claims 1 to 17, further comprising

before the forming the gas-liquid interface,
moving relative position of the flow channel and the organism closer to each other.

**19.** An organism manipulation device for manipulating an organism, comprising:

a flow channel having an end part immersed in a liquid in which the organism is immersed; and
a gas-liquid interface manipulation unit which forms a gas-liquid interface in which a restoring force acts against minute interface movement in the flow channel or at the end part and manipulates the organism by the gas-liquid interface.

**20.** The organism manipulation device according to claim 19, wherein
the gas-liquid interface manipulation unit releases a gas into the liquid from the end part and maintains the gas-liquid interface, in which a restoring force acts against a minute volume change, at the end part.

**21.** The organism manipulation device according to claim 20, wherein

a gas volume V ($m^3$) occupied by the gas continuous from the gas-liquid interface to an inside of the flow channel satisfies a following formula with respect to a radius $r_h$ (m) of the end part of the flow channel:

$$V \leq a \times r_h^4 + b \times r_h^3,$$

(where $a = 2.65 \times 10^8$ and $b = 2.59 \times 10^2$ are satisfied).

**22.** An organism manipulation device comprising:

a flow channel having an end part arranged in a liquid containing an organism;
a pump which introduces a gas into the flow channel to form a gas-liquid interface at the end part;
a pressure detector which detects a first pressure applied to the gas-liquid interface; and
a storing unit which stores a second pressure which allows maintenance of the gas-liquid interface, wherein
the pump changes an introduction amount of the gas on a basis of a change in the first pressure, and controls the gas introduced into the flow channel to maintain a state where the first pressure is equal to or lower than the second pressure.

*FIG.1A*

*FIG.1B*

<u>49</u>

251

51

253

254

51

FIG.2A

FIG.2B

<u>49</u>

251a

251b

253

51a

51b

254

253a

253b

*FIG.3A*

49

253
51a
51b
261
254
255

FIG.3B

FIG.4

IMAGING CONTROL UNIT
171

RECORDING UNIT
190

AIR BUBBLE FORMING UNIT
200

FLOW CHANNEL CONTROL UNIT
250

LIQUID CONTROL UNIT
260

IMAGE PROCESSING UNIT
300

INFORMATION PROCESSING DEVICE

170

*FIG.5*

```
                         START

                    RECEIVE SAMPLE  ~~~ S100

                    CAPTURE IMAGE  ~~~ S120

              RECEIVE MANIPULATION CONDITION  ~~~ S140

          IS THERE LIQUID REPLACEMENT/ADDITION PROCESSING?

                              Yes          LIQUID REPLACEMENT/
                                           ADDITION PROCESSING  ~~~ S600
                         No    S160

                    INSTALL NOZZLE  ~~~ S180

              MOVE RELATIVE POSITION
              BETWEEN NOZZLE AND CELL  ~~~ S200

                  FORM AIR BUBBLE  ~~~ S300

                EXECUTE MANIPULATION  ~~~ S400

                 REMOVE AIR BUBBLE  ~~~ S500

          IS THERE RELEASE OF MANIPULATION TARGET?
                              Yes    RELEASE TO DESIGNATED POSITION
                         No    S640                          S645

              IS THERE                     Yes
          ANOTHER MANIPULATION                    EXCHANGE NOZZLE?    No
             TARGET?                                              S660
                       S650           Yes
                 No

               REMOVE NOZZLE                      REMOVE NOZZLE
                       S680                                   S670

                         END
```

FIG.6

*FIG.7A*

FIG.7B

| I D | COORDINATES | SIZE | MOVEMENT DESTINATION |
|---|---|---|---|
| aaa | $(X_{aaa}, Y_{aaa})$ | AAA | A1 |
| bbb | $(X_{bbb}, Y_{bbb})$ | BBB | A2 |
| ccc | $(X_{ccc}, Y_{ccc})$ | CCC | A3 |
|  |  |  |  |
|  |  |  |  |

FIG.7C

EP 4 219 676 A1

▽ CELL MANIPULATION

  ⦿ CELL RECOVERY (SUBCULTURE)

  ◯ CELL RECOVERY (ANALYSIS)

  ◯ CELL REMOVAL, CUT

  ◯ CYTOPLASM RECOVERY

  ◯ CELL RETENTION, MOVEMENT

  ◯ CELL ADHESIVE FORCE MEASUREMENT

111

▽ CELL COMPRESSION

  ◯ CELL MECHANICS ANALYSIS

  ◯ DEEP CELL PORTION OBSERVATION

  ◯ CELL SUPER-RESOLUTION OBSERVATION

112

FIG.7D

APPLICATION

| CELL RECOVERY (ANALYSIS) ▼ |

⎫
⎬ 113
⎭

MANIPULATION CELL MORPHOLOGY

◯ CYTO-PLASM   ◉ SINGLE CELL   ◯ CELL POPULATION (COLONY)   ◯ SPHEROID

⎫
⎬ 114
⎭

MANIPULATION

◉ RECOVERY   ◯ RETENTION   ◯ REMOVAL   ◯ COMPRESSION

⎫
⎬ 115
⎭

*FIG.7E*

EP 4 219 676 A1

S600

FIG.8

EP 4 219 676 A1

S200

○ FROM S180

OPERATE XYZ POSITION CONTROL ACTUATOR — S210

CAPTURE IMAGE OF NOZZLE END — S215

IS IT DIFFERENT FROM SET XYZ POSITION? — S220

Yes → DECIDE OPERATION AMOUNT OF XYZ POSITION CONTROL ACTUATOR — S225

No → ○ TO S300

FIG.9A

## S200

○ FROM S180

OPERATE Z POSITION CONTROL ACTUATOR ~ S230

MEASURE LOAD OF Z POSITION CONTROL ACTUATOR ~ S235

IS IT EQUAL TO OR LESS THAN SET LOAD? ~ S240 → **Yes** → DECIDE OPERATION AMOUNT OF Z POSITION CONTROL ACTUATOR ~ S242

**No**

SET INITIAL Z POSITION ~ S245

OPERATE XYZ POSITION CONTROL ACTUATOR ~ S250

CAPTURE IMAGE OF NOZZLE END ~ S252

IS IT DIFFERENT FROM SET XYZ POSITION? ~ S254 → **Yes** → DECIDE OPERATION AMOUNT OF XYZ POSITION CONTROL ACTUATOR ~ S256

**No**

○ TO S300

*FIG.9B*

EP 4 219 676 A1

## S200

○ FROM S180

FORM AIR BUBBLE — S260

OPERATE Z POSITION CONTROL ACTUATOR — S262

MEASURE INNER PRESSURE OF AIR BUBBLE — S264

SET INNER PRESSURE CHANGE? — S266

No → DECIDE OPERATION AMOUNT OF Z POSITION CONTROL ACTUATOR — S268

Yes

REMOVE AIR BUBBLE — S270

SET INITIAL Z POSITION — S272

OPERATE XYZ POSITION CONTROL ACTUATOR — S274

CAPTURE IMAGE OF NOZZLE END — S276

IS IT DIFFERENT FROM SET XYZ POSITION? — S280

Yes → DECIDE OPERATION AMOUNT OF XYZ POSITION CONTROL ACTUATOR — S282

No

○ TO S300

*FIG.9C*

S300

FROM S200

OPERATE PUMP — S320

CAPTURE IMAGE OF NOZZLE END — S330

IS IT DIFFERENT FROM SET AIR BUBBLE SHAPE? — S340

Yes → DECIDE OPERATION AMOUNT OF PUMP — S342

No → TO S400

*FIG.10A*

EP 4 219 676 A1

## S300

FROM S200

OPERATE PUMP — S370

MEASURE INNER PRESSURE OF NOZZLE — S380

IS IT DIFFERENT FROM SET INNER PRESSURE? — S390

Yes → DECIDE OPERATION AMOUNT OF PUMP — S392

No → TO S400

*FIG.10B*

FIG.11A

EP 4 219 676 A1

FIG.11B

804a
804b
804c
804d

*FIG.11C*

FIRST TIME

(AIR)

SECOND TIME

810a        810b        810c

*FIG.11D*

EP 4 219 676 A1

*FIG.11E*

TRANSMISSION
Day 4:    IMAGE         Day 10:ALP STAIN (P1)    Day 30:ALP STAIN (P3)
          OBSERVATION

EMBODIMENT

830a                    831a                    832a

COMPARATIVE
EXAMPLE

830b                    831b                    832b
(n=4)                   (n=4)                   (n=3)

*FIG.11F*

FIG.11G

EP 4 219 676 A1

FIG.11H

FIG.11I

S500

FROM S400

OPERATE PUMP — S510

CAPTURE IMAGE OF NOZZLE END — S520

IS IT DIFFERENT FROM SET GAS-LIQUID INTERFACE POSITION? — S530

Yes → DECIDE OPERATION AMOUNT OF PUMP — S532

No ↓

DETACH CELL FROM INTERFACE? — S540

Yes → TAKE OUT NOZZLE FROM LIQUID — S542 → MOVE INTERFACE AT HIGH SPEED — S544

No → TO S640

FIG.12A

EP 4 219 676 A1

## S500

FROM S400

OPERATE PUMP — S560

↓

MEASURE INNER PRESSURE OF NOZZLE — S570

↓

S580 — IS IT DIFFERENT FROM SET INNER PRESSURE?

Yes → DECIDE OPERATION AMOUNT OF PUMP — S582

No ↓

S590 — DETACH CELL FROM INTERFACE?

Yes → TAKE OUT NOZZLE FROM LIQUID — S592 → MOVE INTERFACE AT HIGH SPEED — S594

No ↓

TO S640

*FIG.12B*

*FIG.13A*

EP 4 219 676 A1

FIG.13B

STABILIZATION OF AIR BUBBLE

DECREASE VOLUME OF GAS
910

MAKE FLOW CHANNEL EXTREMELY THIN
911

FILL FLOW CHANNEL WITH FILLER
912

TAKE LIQUID INTO FLOW CHANNEL TO PERFORM PARTITIONING
913

PROVIDE DIVIDING MEMBER IN FLOW CHANNEL TO PERFORM PARTITIONING
914

PROVIDE PORTION HAVING LARGE FLOW CHANNEL DIAMETER AND PORTION HAVING SMALL FLOW CHANNEL DIAMETER IN FLOW CHANNEL
915

INCREASE CHANGE IN LAPLACE PRESSURE
920

OPTIMIZE SHAPE OF END PART OF FLOW CHANNEL
921

SET CONTACT ANGLE BETWEEN FLOW CHANNEL MEMBER AND LIQUID TO 90 DEGREES OR LESS
922

PROVIDE STEPPED PORTION IN FLOW CHANNEL
923

DECREASE SURFACE TENSION OF GAS-LIQUID INTERFACE
930

ADD SOLUTE TO LIQUID
931

REMOVE SOLUTE FROM LIQUID
932

ADD OR REPLACE ANOTHER LIQUID
933

FIG.13C

EP 4 219 676 A1

*FIG.13D*

704a  704b  *FIG.13E*  704c  705

EP 4 219 676 A1

DIMENSIONLESS VOLUME

DIMENSIONLESS CURVATURE

298b

298c

298a

FIG.13F

EP 4 219 676 A1

*FIG.13G*

*FIG.13H*

EP 4 219 676 A1

EP 4 219 676 A1

*FIG.13I*

FIG.13J

FIG.13K

FIG.13L

EP 4 219 676 A1

FIG.14

## INTERNATIONAL SEARCH REPORT

| International application No. |
|---|
| **PCT/JP2021/035209** |

### A. CLASSIFICATION OF SUBJECT MATTER

*C12M 1/04*(2006.01)i; *C12N 1/00*(2006.01)i; *C12N 1/02*(2006.01)i
FI: C12M1/04; C12N1/02; C12N1/00 K

According to International Patent Classification (IPC) or to both national classification and IPC

### B. FIELDS SEARCHED

Minimum documentation searched (classification system followed by classification symbols)

C12M1/04; C12N1/00; C12N1/02

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | Microfilm of the specification and drawings annexed to the request of Japanese Utility Model Application No. 110417/1989 (Laid-open No. 50900/1991) (TSUMURA & CO.) 17 May 1991 (1991-05-17), claims, page 1, 4th line from the bottom to page 2, line 3, page 4, section of [action], page 6, second paragraph, fig. 1-3 | 1-2, 4-5, 7-15, 18-19 |
| A | | 3, 6, 16-17, 20-22 |
| A | WO 2015/098919 A1 (SEKISUI CHEMICAL CO., LTD.) 02 July 2015 (2015-07-02) | 1-22 |

☐ Further documents are listed in the continuation of Box C. ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **21 October 2021** | **02 November 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

### INTERNATIONAL SEARCH REPORT
**Information on patent family members**

International application No.

**PCT/JP2021/035209**

| Patent document<br>cited in search report | Publication date<br>(day/month/year) | Patent family member(s) | Publication date<br>(day/month/year) |
|---|---|---|---|
| JP        3-50900        U1 | 17 May 1991 | (Family: none) | |
| WO      2015/098919    A1 | 02 July 2015 | TW         201529854        A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016000007 A
- JP 7013083 A
- JP 3814869 B